# EUROPEAN PATENT SPECIFICATION

(11) **EP 2 904 032 B1**
(45) Date of publication and mention of the grant of the patent: **07.03.2018**
(21) Application number: 13776694.5
(22) Date of filing: 04.10.2013
(51) Int. Cl.: C08G 65/40, C08G 73/10, C08L 79/00

(54) **METHODS OF MANUFACTURE OF BIS(PHTHALIMIDE)S AND POLYETHERIMIDES, AND BIS(PHTHALIMIDE)S, AND POLYETHERIMIDES FORMED THEREFROM**
VERFAHREN ZUR HERSTELLUNG VON BIS(PHTHALIMID)EN UND POLYETHERIMIDEN SOWIE IN DIESEM VERFAHREN HERGESTELLTE BIS(PHTHALIMID)E UND POLYETHERIMIDE
PROCÉDÉS DE FABRICATION DE BIS(PHTALIMIDE)S ET DE POLYÉTHERIMIDES, ET BIS(PHTALIMIDE)S ET POLYÉTHERIMIDES FORMÉS À PARTIR DE CEUX-CI

(30) Priority: 04.10.2012 US 201213644633
(43) Date of publication of application: 12.08.2015
(73) Proprietor: SABIC Global Technologies B.V., 4612 PX Bergen op Zoom (NL)
(72) Inventor: CHIONG, Hendrich, Mt. Vernon, Indiana 47620-9367 (US); DALIPARTHI, Surya Prakasa Rao, Karnataka Bangalore 560066 (IN); DESHPANDE, Hareesh Shamrao, Karnataka Bangalore 560066 (IN); GUGGENHEIM, Thomas Link, Mt. Vernon, Indiana 47620-9367 (US); KHOURI, Farid Fouad, Niskayuna, New York 12309 (US); KUMAR, Mahesh Srinivas, Karnataka Bangalore 560066 (IN); DE CASTRO, Miguel Angel Navarro, 30390 Cartagena (ES); ODLE, Roy Ray, Mt. Vernon, Indiana 47620-9637 (US); P., Sivakumar, Karnataka Bangalore 560066 (IN); S., Dhumal Sunil, Karnataka Bangalore 560066 (IN); SREERAMAGIRI, Siva Kumar, Karnataka Bangalore 560066 (IN); SMITH, Brennan Alexander, Decatur, Illinois 62526 (US)
(74) Representative: Office Freylinger
(86) International application number: PCT/US2013/063444
(87) International publication number: WO 2014/055856

(56) References cited:
- EP-A1- 0 634 437
- EP-A1- 2 644 640
- EP-A1- 2 644 641
- WO-A1-2013/063470
- WO-A2-01/25196
- US-A1- 2007 043 203

## Description

### BACKGROUND OF THE INVENTION

This disclosure relates to bis(phthalimide) and polyetherimide compositions, as well as their method of manufacture and articles formed from the polyetherimide compositions.

Polyetherimides ("PEIs") are amorphous, transparent, high performance polymers having a glass transition temperature ("Tg") of greater than 180°C. PEIs further have high strength, heat resistance, modulus, and broad chemical resistance, and therefore are widely used in applications as diverse as automotive, telecommunication, aerospace, electrical/electronics, transportation, and healthcare. One process for the manufacture of polyetherimides is by polymerization of alkali metal salts of dihydroxyaromatic compounds, such as bisphenol A disodium salt ("BPANa₂"), with a substituted bis(phthalimide) such as a bis(halophthalimide). For example, polyetherimides can be produced by polymerization of BPANa₂ with 1,3-bis[N-(4-chlorophthalimido)]benzene ("4-ClPAMI"), which has the following structure:

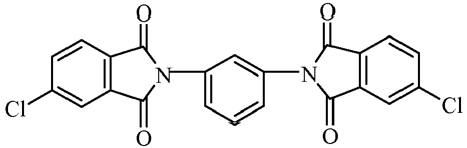

Other isomers of the ClPAMI can also be present. Substituted bis(phthalimides) such as bis(halophthalimide)s, in turn, can be produced by imidization of a substituted or halophthalic anhydride such as 3-chlorophthalic anhydride ("3-ClPA"), 4-chlorophthalic anhydride ("4-ClPA"), or mixtures thereof with an organic diamine such as m-phenylenediamine ("mPD") or p-phenylenediamine ("pPD").

An ongoing challenge associated with the imidization reaction is achieving high conversion of substituted phthalic anhydride and organic diamine to substituted bis(phthalimide) in a commercially useful reaction time. The rate of imidization can be increased by conducting the reaction at higher temperatures, but decomposition can occur or unwanted by-products can form. Alternatively, imidization can be conducted at a lower temperature in the presence of a catalyst. For example, U.S. Patent No. 6,235,866 discloses sodium phenylphosphinate ("SPP") as a useful catalyst for the imidization reaction. However, the use of SPP, like the use of higher temperatures, can result in unwanted by-products that can adversely affect the physical properties of the polyetherimide. Moreover, the presence of residual SPP in the polyetherimide can adversely affect the hydrolytic stability of the polyetherimide under certain conditions.

Thus there remains a need in the art for an improved process for the manufacture of polyetherimides that does not result in decomposition or side reactions that can adversely affect the properties of the polyetherimides, for example hydrolytic stability. It would be a further advantage if such methods allowed the production of polyetherimides without significantly adversely affecting other desirable properties of the polyetherimides. A still further advantage would be scalability of the process to industrial production levels. There also remains the need in the art for an improved method for manufacture of polyetherimides in which a catalyst can be used for both imidization and polymerization phases of manufacturing processes.

### SUMMARY OF THE INVENTION

Disclosed herein is a method of manufacture of a bis(phthalimide) composition, the method comprising catalyzing imidization of a substituted phthalic anhydride and an organic diamine with a catalyst, in the presence of a solvent, the catalyst being selected from quaternary ammonium salts, quaternary phosphonium salts, guanidinium salts, pyridinium salts, imidazolium salts, and combinations thereof; wherein the substituted phthalic anhydride has a formula 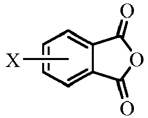 and
the organic diamine has a formula

H₂N-R-NH₂;

to provide the bis(phthalimide) composition comprising a residue of the catalyst, and a bis(phthalimide) of the formula 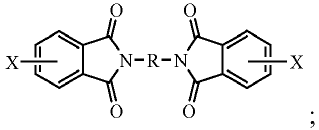 wherein, in the foregoing formulae, X is selected from fluoro, chloro, bromo, iodo, nitro, and combinations thereof, and R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 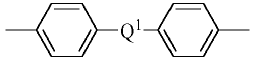 wherein Q¹ is a member selected from the group of a single bond, -O-, -S-, -C(O)-, -SO₂-, - SO-, -C_{y}H_{2y} wherein y is an integer from 1 to 5, and combinations thereof; wherein conversion to the bis(phthalimide) is 99% complete, based on the moles of the substituted phthalic anhydride, in less than 6 hours.

Also disclosed herein is a bis(phthalimide) composition, comprising a bis(phthalimide) having a formula 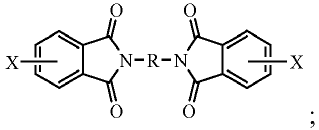 and a residue of a catalyst selected from quaternary ammonium salts, quaternary phosphonium salts, guanidinium salts, pyridinium salts, imidazolium salts, and combinations thereof, wherein the bis(phthalimide) is an imidization product of a substituted phthalic anhydride and an organic diamine that are catalyzed with the catalyst; the substituted phthalic anhydride having a formula 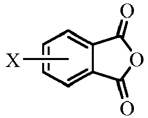 the organic diamine having a formula

H₂N-R-NH₂

wherein, in the foregoing formulae, X is selected from fluoro, chloro, bromo, iodo, nitro, and combinations thereof, and R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 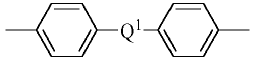 wherein Q¹ is a member selected from the group of a single bond, -O-, -S-, -C(O)-, -SO₂-, - SO-, -C_{y}H_{2y} wherein y is an integer from 1 to 5, and combinations thereof.

Also disclosed herein is a bis(chlorophthalimide) composition, comprising a bis(chlorophthalimide) of the formula 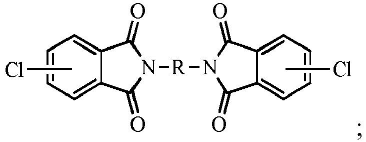 and a residue of a catalyst selected from quaternary ammonium salts, quaternary phosphonium salts, guanidinium salts, pyridinium salts, imidazolium salts, and combinations thereof, wherein the bis(chlorophthalimide) is an imidization product of a chlorophthalic anhydride and an organic diamine that are catalyzed with the catalyst; the chlorophthalic anhydride having a formula 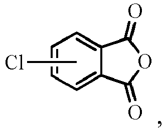 the organic diamine having a formula

H₂N-R-NH₂

wherein, in the foregoing formulae, R is selected from m-phenylene, p-phenylene, diarylsulfone, and a group of the formula 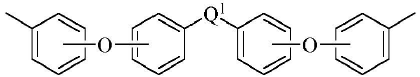 wherein Q¹ is a member selected from the group of a single bond, -O-, -S-, -C(O)-, -SO₂-, - SO-, -C_{y}H_{2y} wherein y is an integer from 1 to 5, and combinations thereof.

Also disclosed herein is a method for the manufacture of a polyetherimide composition, the method comprising catalyzing imidization of a substituted phthalic anhydride and an organic diamine with a catalyst in the presence of a solvent, the catalyst being selected from quaternary ammonium salts, quaternary phosphonium salts, guanidinium salts, pyridinium salts, imidazolium salts, and combinations thereof, wherein the substituted phthalic anhydride has a formula 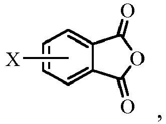 and
the organic diamine has a formula

H₂N-R-NH₂

to provide a bis(phthalimide) composition comprising a residue of the catalyst and a bis(phthalimide) of the formula 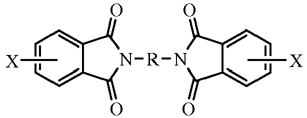 wherein conversion to the bis(phthalimide) is 99% complete, based on the moles of the substituted phthalic anhydride, in less than 6 hours, and catalyzing polymerization of the bis(phthalimide) and an alkali metal salt of a dihydroxy aromatic compound of the formula

MO-Z-OM

in the presence of the catalyst that catalyzes imidization of the substituted phthalic anhydride and the diamine to form the polyetherimide composition comprising a residue of the catalyst and a polyetherimide of the formula 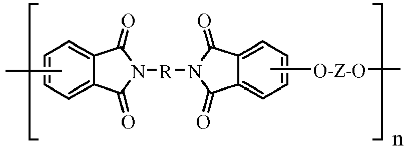 wherein in the foregoing formulae X is selected from fluoro, chloro, bromo, iodo, nitro, and combinations thereof; R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 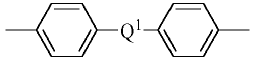 wherein Q¹ is selected from -O-, -S-, -C(O)-, -SO₂-, -SO-, -C_{y}H_{2y}- wherein y is an integer from 1 to 5, and combinations thereof; M is an alkali metal; Z is an aromatic C₆₋₂₄ monocyclic or polycyclic moiety optionally substituted with 1 to 6 C₁₋₈ alkyl groups, 1 to 8 halogen atoms, and combinations thereof; and n is an integer greater than 1.

Also disclosed herein is a polyetherimide composition comprising (i) a polyetherimide of the formula 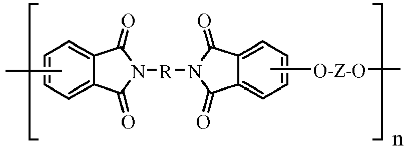 and (ii) a residue of a catalyst selected from quaternary ammonium salts, quaternary phosphonium salts, guanidinium salts, pyridinium salts, imidazolium salts, and combinations thereof; the polyetherimide being a catalyzed polymerization reaction product of (1) a bis(phthalimide) composition comprising an catalyzed imidization product of a substituted phthalic anhydride and an organic diamine and (2) an alkali metal salt of a dihydroxy aromatic compound, the imidization product being catalyzed by the catalyst; wherein the substituted phthalic anhydride has a formula 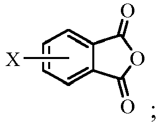 the organic diamine has a formula

H₂N-R-NH_{2 ;}

the bis(phthalimide) has a formula 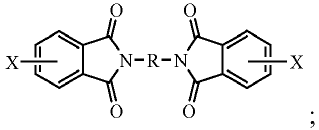 and the alkali metal salt of the dihydroxy aromatic compound has a formula

MO-Z-OM;

wherein in the foregoing formulae, X is selected from fluoro, chloro, bromo, iodo, nitro, and combinations thereof; R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 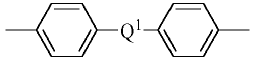 wherein Q¹ is selected from -O-, -S-, -C(O)-, -SO₂-, -SO-, -C_{y}H_{2y}- wherein y is an integer from 1 to 5, and combinations thereof; M is an alkali metal; Z is an aromatic C₆₋₂₄ monocyclic or polycyclic moiety optionally substituted with 1 to 6 C₁₋₈ alkyl groups, 1 to 8 halogen atoms, and combinations thereof; and n is an integer greater than 1.

Articles comprising the above polyetherimide compositions are also disclosed.

A method of forming the above articles comprises shaping, extruding, blow molding, or injection molding the above polyetherimide compositions to form the articles.

Still further disclosed is a composition comprising a catalyst selected from quaternary ammonium salts, quaternary phosphonium salts, guanidinium salts, pyridinium salts, imidazolium salts, and combinations thereof; a solvent; a substituted phthalic anhydride of the formula 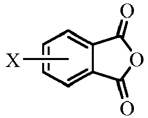 wherein X is selected from fluoro, chloro, bromo, iodo, nitro, and combinations thereof; and an organic diamine of the formula

H₂N-R-NH₂

wherein R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 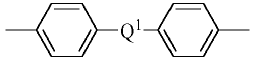 wherein Q¹ is selected from -O-, -S-, -C(O)-, -SO₂-, -SO-, and -C_{y}H_{2y}- wherein y is an integer from 1 to 5, and combinations thereof.

Also disclosed herein, is a method for the manufacture of a bis(phthalimide) composition, the method comprising catalyzing imidization of a substituted phthalic anhydride and an organic diamine with a catalyst, in the presence of a solvent, the catalyst being selected from quaternary ammonium salts, quaternary phosphonium salts, guanidinium salts, pyridinium salts, imidazolium salts, and combinations thereof;
wherein
the substituted phthalic anhydride has a formula 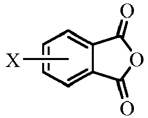 and
the organic diamine has a formula

H₂N-R-NH₂;

to provide the bis(phthalimide) composition comprising a residue of the catalyst, and a bis(phthalimide) of the formula 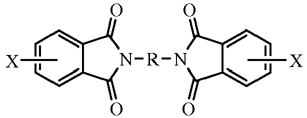 wherein, in the foregoing formulae,
X is selected from fluoro, chloro, bromo, iodo, nitro, and combinations thereof, and
R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 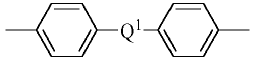 wherein Q¹ is a member selected from the group of a single bond, -O-, -S-, -C(O)-, -SO₂-, - SO-, -C_{y}H_{2y} wherein y is an integer from 1 to 5, and combinations thereof;
wherein conversion to the bis(phthalimide) is 99% complete, based on the moles of the substituted phthalic anhydride, in less than 6 hours, and
wherein the bis(phthalimide) comprises less than or equal to 0.5 mole percent residual substituted phthalic anhydride and less than or equal to 2 mole percent monoamine (20), based on the total moles of the substituted phthalic anhydride, the organic diamine, and a monofunctional reactant, if used.

In still another embodiment, disclosed herein is a composition comprising a residue of a catalyst from the manufacture of a bisphthalimide, wherein the catalyst is selected from quaternary ammonium salts, quaternary phosphonium salts, and combinations thereof; a solvent; a bisphthalimide having the formula 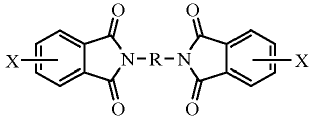 wherein X is selected from fluoro, chloro, bromo, iodo, nitro, and combinations thereof, and R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 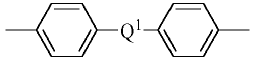 wherein Q¹ is selected from -O-, -S-, -C(O)-, -SO₂-, -SO-, and -C_{y}H_{2y}- wherein y is an integer from 1 to 5, and combinations thereof; an alkali metal salt of a dihydroxy aromatic compound of the formula

MO-Z-OM

wherein M is an alkali metal, and Z is an aromatic C₆₋₂₄ monocyclic or polycyclic moiety optionally substituted with 1 to 6 C₁₋₈ alkyl groups, 1 to 8 halogen atoms, and combinations thereof; and optionally a catalytically active amount of a polymerization catalyst selected from quaternary ammonium salts, quaternary phosphonium salts, guanidinium salts, pyridinium salts, imidazolium salts, and combinations thereof.

In another embodiment, disclosed herein is a polyetherimide composition comprising
(i) a polyetherimide of the formula 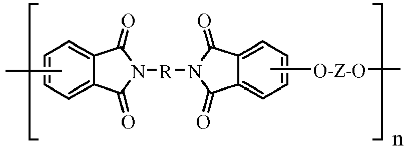 and
(ii) a residue of a catalyst selected from quaternary ammonium salts, quaternary phosphonium salts, guanidinium salts, pyridinium salts, imidazolium salts, and combinations thereof;
the polyetherimide being a catalyzed polymerization reaction product of (1) a bis(phthalimide) composition comprising a catalyzed imidization product of a substituted phthalic anhydride and an organic diamine and (2) an alkali metal salt of a dihydroxy aromatic compound, the imidization product being catalyzed by the catalyst, the bis(phthalimide) composition comprising less than or equal to 0.5 mole percent residual substituted phthalic anhydride and less than or equal to 2 mole percent monoamine (20), based on the total moles of the substituted phthalic anhydride, the organic diamine, and a monofunctional reactant, if used;
wherein
the substituted phthalic anhydride has a formula 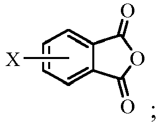 the organic diamine has a formula

H₂N-R-NH_{2 ;}

the bis(phthalimide) has a formula 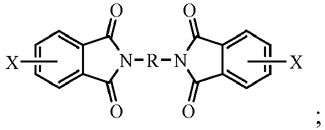 and
the alkali metal salt of the dihydroxy aromatic compound has a formula

MO-Z-OM;

wherein in the foregoing formulae,
X is selected from fluoro, chloro, bromo, iodo, nitro, and combinations thereof;
R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 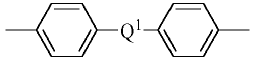 wherein Q¹ is selected from -O-, -S-, -C(O)-, -SO₂-, -SO-, -C_{y}H_{2y}- wherein y is an integer from 1 to 5, and combinations thereof;
M is an alkali metal;
Z is an aromatic C₆₋₂₄ monocyclic or polycyclic moiety optionally substituted with 1 to 6 C₁₋₈ alkyl groups, 1 to 8 halogen atoms, and combinations thereof; and
n is an integer greater than 1.

The invention is further illustrated by the Drawings, Detailed Description, Examples, and Claims.

### BRIEF DESCRIPTION OF THE DRAWINGS

FIG. 1 is a graph showing residual 4-ClPA as a function of reaction time for uncatalyzed and SPP catalyzed imidization reactions.
FIG. 2 is a graph showing the amount of residual 4-ClPA as a function of reaction time.
FIG. 3 is a graph showing the amount of residual 4-ClPA and MA as a function of reaction time.
FIG. 4 is a graph of Mw vs. polymerization time.
FIG. 5 is a graph showing the amount of residual 4-ClPA and MA as a function of reaction time.
FIG. 6 is a graph of Mw as a function of polymerization time.
FIG. 7 is a graph showing the amount of residual 4-ClPA and MA as a function of reaction time.
FIG. 8 is a graph of Mw as a function of polymerization time.
FIG. 9 is a graph showing Mw of polyetherimides as a function of times for uncatalyzed and HEGCl catalyzed polycondensation reactions between DDS and BPADA.
FIG. 10 is a graph showing Mw of polyetherimides as a function of times for uncatalyzed and HEGCl catalyzed polycondensation reactions between BPADA and mPD.

### DETAILED DESCRIPTION OF THE INVENTION

Our invention is based on the discovery that it is now possible to make polyetherimides having lower amount of by-products and hence improved properties, such as hydrolytic stability in an industrial setting. In particular, the inventors have discovered that reacting a substituted phthalic anhydride and an organic diamine in the presence of an imidization catalyst of this disclosure is effective to provide a bis(phthalimide) in high yields within a commercially useful reaction time. In addition, it is found that the catalyst can be used for both imidization and polymerization phases of the manufacturing processes, despite the imidization reaction and the polymerization reaction being fundamentally different from each other. The catalysts can be a quaternary ammonium salt, a quaternary phosphonium salt, a guanidinium salt, a pyridinium salt, an imidazolium salt, and combinations thereof. Use of the catalyst of this disclosure decreases or eliminates the decomposition and/or other side reactions that may lead to the formation of undesirable by-products adversely affecting the properties of the polyetherimide. In a still further advantage, the polyetherimides produced by the methods of this disclosure can exhibit improved hydrolytic stability. In particular, the polyetherimides exhibit improved melt flow rate retention and tensile strength retention upon aging under hydrolytic conditions. Moreover, the polyetherimides have good melt viscosity and an acceptable yellowness index.

Other than in the operating examples or where otherwise indicated, all numbers or expressions referring to quantities of ingredients, reaction conditions, and the like, used in the specification and claims are to be understood as modified in all instances by the term "about." Various numerical ranges are disclosed in this patent application. Because these ranges are continuous, they include every value between the minimum and maximum values. Unless expressly indicated otherwise, the various numerical ranges specified in this application are approximations. The endpoints of all ranges directed to the same property are inclusive of the endpoint and independently combinable.

All molecular weights in this application refer to weight average molecular weights unless indicated otherwise. All such mentioned molecular weights are expressed in Daltons.

As used herein, the terms "a" and "an" do not denote a limitation of quantity, but rather denote the presence of at least one of the referenced item. "Or" means "and/or." The term "combination thereof' is inclusive of one or more of the recited elements, optionally together with a like element not recited. Reference throughout the specification to "another embodiment," "an embodiment," "some embodiments," and so forth, means that a particular element (e.g., feature, structure, property, and/or characteristic) described in connection with the embodiment is included in at least one embodiment described herein, and can or cannot be present in other embodiments. In addition, it is to be understood that the described element(s) can be combined in any suitable manner in the various embodiments.

Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group. The term "alkyl" includes both C₁₋₃₀ branched and straight chain, unsaturated aliphatic hydrocarbon groups having the specified number of carbon atoms. Examples of alkyl include, but are not limited to, methyl, ethyl, n-propyl, i-propyl, n-butyl, s-butyl, t-butyl, n-pentyl, s-pentyl, n- and s-hexyl, n- and s-heptyl, and n- and s-octyl. The term "aryl" means an aromatic moiety containing the specified number of carbon atoms, such as phenyl, tropone, indanyl, or naphthyl. The term "hydrocarbyl moiety" means a group comprising carbon and hydrogen, optionally further comprising 1 to 6 heteroatoms, for example, oxygen, nitrogen, halogen, silicon, sulfur, or a combination thereof.

All ASTM tests are based on the 2003 edition of the Annual Book of ASTM Standards unless otherwise indicated.

The polyetherimides produced by the methods disclosed herein are of formula (1) 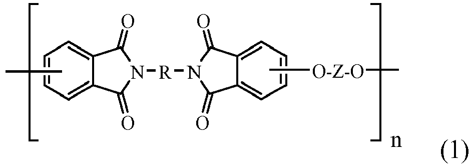 wherein n is greater than 1, for example 10 to 1,000 or more, or more specifically 10 to 500.

The group R in formula (1) is a C₆₋₂₇ aromatic hydrocarbon group or a halogenated derivative thereof, a straight or branched chain C₂₋₂₀ , specifically C₂₋₁₀ alkylene group or a halogenated derivative thereof, a C₃₋₂₀ cycloalkylene group or halogenated derivative thereof, -(C₆H₁₀)_{z}- wherein z is an integer from 1 to 4, an aromatic hydrocarbyl moiety having from 1 to 6 aromatic groups, and a group of formula (2) 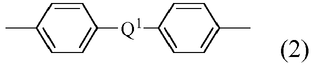 wherein Q¹ is selected from -O-, -S-, -C(O)-, -SO₂-, -SO-, -C_{y}H_{2y}- wherein y is an integer from 1 to 5, and combinations thereof.

In an embodiment R is -(C₆H₁₀)_{z}- wherein z is an integer from 1 to 4 or a divalent group of formulae (3) 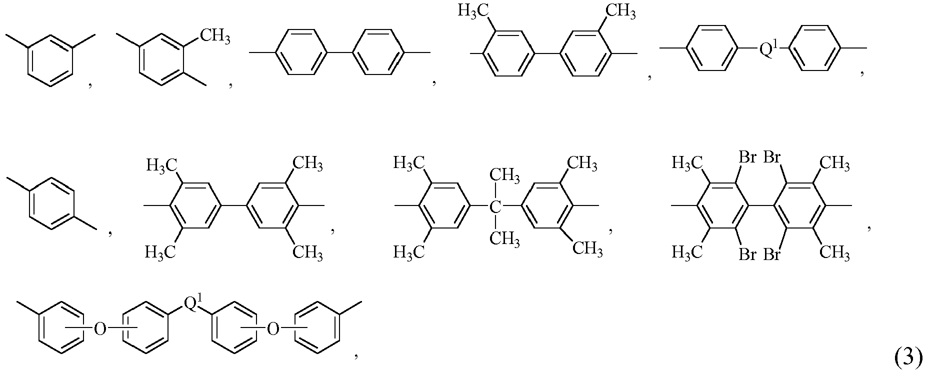 wherein Q¹ is -O-, -S-, -C(O)-, -SO₂-, -SO-, -C_{y}H_{2y}- wherein y is an integer from 1 to 5, and combinations thereof. In some embodiments, R is the diether aromatic moiety of formula (3) having four phenylene groups wherein Q¹ is a direct bond, -O-, -S-, -C(O)-, -SO₂-, -SO-, -C_{y}H_{2y}- wherein y is an integer from 1 to 5. In some embodiments R is m-phenylene, p-phenylene, or diarylsulfone. The diarylsulfone can be, for example, 4,4'-diphenylsulfone. Embodiments where R is a divalent arylene ether can also be specifically mentioned, for example an arylene ether of the formula 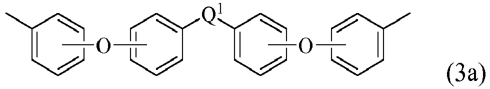 wherein Q¹ is selected from a direct bond, -O-, -S-, -C(O)-, -SO₂-, -SO-, -C_{y}H_{2y}- wherein y is an integer from 1 to 5, and combinations thereof. In an embodiment, Q¹ in formula (3a) is - O-.

The group Z in formula (1) is a substituted or unsubstituted divalent organic group, and can be an aromatic C₆₋₂₄ monocyclic or polycyclic moiety optionally substituted with 1 to 6 C₁₋₈ alkyl groups, 1 to 8 halogen atoms, or a combination thereof, provided that the valence of Z is not exceeded. Exemplary groups Z include groups of formula (4): 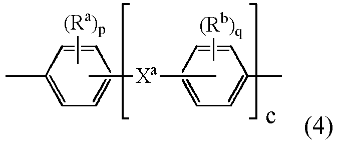 wherein R^{a} and R^{b} are each independently a halogen atom or a monovalent hydrocarbon group and can be the same or different; p and q are each independently integers of 0 to 4; c is zero or 1; and X^{a} is a bridging group connecting the two aromatic groups, where the bridging group and point of attachment of each C₆ arylene group are disposed ortho, meta, or para (specifically para) to each other on the C₆ arylene group. The bridging group X^{a} can be a single bond, -O-, -S-, -S(O)-, -S(O)₂-, -C(O)-, or a C₁₋₁₈ organic bridging group. The C₁₋₁₈ organic bridging group can be cyclic or acyclic, aromatic or non-aromatic, and can further comprise heteroatoms such as halogens, oxygen, nitrogen, sulfur, silicon, or phosphorous. The C₁₋₁₈ organic group can be disposed such that the C₆ arylene groups connected thereto are each connected to a common alkylidene carbon or to different carbons of the C₁₋₁₈ organic bridging group. A specific example of a group Z is a divalent group of formula (4a) 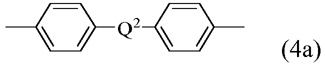 wherein Q² is -O-, -S-, -C(O)-, -SO₂-, -SO-, -C_{y}H_{2y}- and a halogenated derivative thereof wherein y is an integer from 1 to 5, including perfluoroalkylene groups. In a specific embodiment Q² is 2,2-isopropylidene.

In another specific embodiment, the polyetherimide comprises more than 1, specifically 10 to 1,000, or more specifically, 10 to 500 structural units, of formula (1) wherein R is a divalent group of formula (2) wherein Q¹ is -C_{y}H_{2y}- wherein y is an integer from 1 to 5, and Z is a group of formula (4a) wherein Q² is -O-, -S-, -C(O)-, -SO₂-, -SO-, and -C_{y}H_{2y}- or a halogenated derivative thereof wherein y is an integer from 1 to 5. In some embodiments, R is m-phenylene, p-phenylene, diphenylsulfone, and combinations thereof, and Z is 2,2-(4-phenylene)isopropylidene. In some embodiments, the polyetherimide is a polyetherimide sulfone. A specific polyetherimide sulfone comprises structural units of formula (1) wherein at least 50 mole percent of the R groups are of formula (4a) wherein Q² is -SO₂- and the remaining R groups are independently p-phenylene or m-phenylene or a combination comprising at least one of the foregoing; and Z is 2,2-(4-phenylene)isopropylidene.

The polyetherimides can optionally comprise additional structural imide units, for example imide units of formula (5) 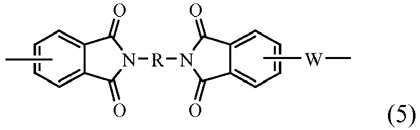 wherein R is as described in formula (1) and W is a linker of formulas (6).

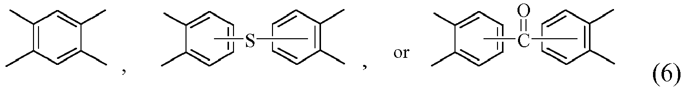

These additional structural imide units can be present in amounts ranging from 0 to 10 mole % of the total number of units, specifically 0 to 5 mole %, more specifically 0 to 2 mole %. In one embodiment no additional imide units are present in the polyetherimides.

The polyetherimides are prepared first by imidization of a substituted phthalic anhydride with an organic diamine to form a bis(phthalimide), followed by polymerization of the bis(phthalimide) at the substituted position. In this method, a substituted phthalic anhydride of formula (7) 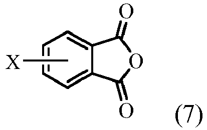 wherein X is a leaving group (such as a nitro group or a halogen), is condensed (imidized) with an organic diamine of formula (8)

H₂N-R-NH₂ (8)

wherein R is as described in formula (1), to form a bis(phthalimide) of formula (9) 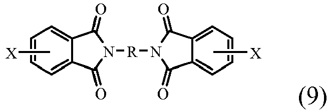 wherein X is a leaving group as in formula (7) and R is a linker as described in formula (1).

In an embodiment, X is a nitro group or a halogen, specifically fluoro, chloro, bromo, iodo, more specifically chloro. A mixture of different X groups can be used.

Illustrative examples of amine compounds of formula (8) include ethylenediamine, propylenediamine, trimethylenediamine, diethylenetriamine, triethylenetetramine, hexamethylenediamine, heptamethylenediamine, octamethylenediamine, nonamethylenediamine, decamethylenediamine, 1,12-dodecanediamine, 1,18-octadecanediamine, 3-methylheptamethylenediamine, 4,4-dimethylheptamethylenediamine, 4-methylnonamethylenediamine, 5-methylnonamethylenediamine, 2,5-dimethylhexamethylenediamine, 2,5-dimethylheptamethylenediamine, 2, 2-dimethylpropylenediamine, N-methyl-bis (3-aminopropyl) amine, 3-methoxyhexamethylenediamine, 1,2-bis(3-aminopropoxy) ethane, bis(3-aminopropyl) sulfide, 1,4-cyclohexanediamine, bis-(4-aminocyclohexyl) methane, m-phenylenediamine, p-phenylenediamine, 2,4-diaminotoluene, 2,6-diaminotoluene, m-xylylenediamine, p-xylylenediamine, 2-methyl-4,6-diethyl-1,3-phenylene-diamine, 5-methyl-4,6-diethyl-1,3-phenylene-diamine, benzidine, 3,3'-dimethylbenzidine, 3,3'-dimethoxybenzidine, 1,5-diaminonaphthalene, bis(4-aminophenyl) methane, bis(2-chloro-4-amino-3, 5-diethylphenyl) methane, bis(4-aminophenyl) propane, 2,4-bis(b-amino-t-butyl) toluene, bis(p-b-amino-t-butylphenyl) ether, bis(p-b-methyl-o-aminophenyl) benzene, bis(p-b-methyl-o-aminopentyl) benzene, 1, 3-diamino-4-isopropylbenzene, bis(4-aminophenyl) ether and 1,3-bis(3-aminopropyl) tetramethyldisiloxane. Mixtures of these amines can be used. Illustrative examples of amine compounds of formula (8) containing sulfone groups include diamino diphenyl sulfone (DDS) and bis(aminophenoxy phenyl) sulfones (BAPS). Combinations comprising any of the foregoing amines can be used.

Specifically, diamine (8) is a meta-phenylene diamine (8a), a para-phenylene diamine (8b), or a diamino diaryl sulfone (8c) 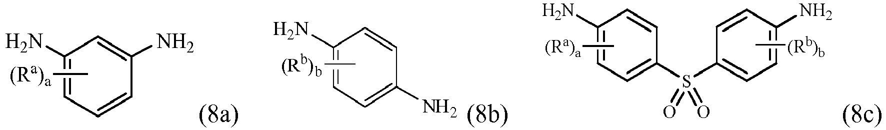 wherein R^{a} and R^{b} are each independently a halogen atom, nitro, cyano, C₂-C₂₀ aliphatic group, C₂-C₄₀ aromatic group, and a and b are each independently 0 to 4. Specific examples include meta-phenylenediamine (mDA), para-phenylenediamine (pDA), 2,4-diaminotoluene, 2,6-diaminotoluene, 2-methyl-4,6-diethyl-1,3-phenylenediamine, 5-methyl-4,6-diethyl-1,3-phenylenediamine, 1,3-diamino-4-isopropylbenzene, and 4,4'-diamino diphenyl sulfone. In some embodiments of bis(phthalimide) (9), X is chloro or fluoro, specifically chloro, and R is m-phenylene, p-phenylene, a diarylsulfone, or a combination thereof.

Condensation of a substituted phthalic anhydride of formula (7) and an organic diamine of formula (8) (imidization) is conducted in the presence of specific imidization catalysts. Imidization catalysts are selected from quaternary ammonium salts, quaternary phosphonium salts, guanidinium salts, pyridinium salts, imidazolium salts, and combinations thereof. The inventors have discovered that an imidization catalyst selected from these catalysts, instead of a catalyst such as SPP, is highly effective in catalyzing the reaction of a substituted phthalic anhydride with an organic diamine to form a bis(phthalimide). In particular, the imidization catalyst provides high levels of conversion in a commercially useful reaction time, and the process is scalable for industrial production. In addition, use of the imidization catalyst decreases or eliminates the decomposition and/or side reactions that can form undesirable by-products that in turn adversely affect the properties of the polyetherimide. In a still further advantage, the polyetherimides produced using this catalyst exhibit improved hydrolytic stability, in particular improved melt flow rate retention and tensile strength retention upon aging under hydrolytic conditions. Moreover, the polyetherimides have a good viscosity and an acceptable yellowness index.

The imidization catalyst is selected from quaternary ammonium salts, quaternary phosphonium salts, guanidinium salts, pyridinium salts, imidazolium salts, and combinations thereof. A combination of different salts can be used. The foregoing salts include an anionic component, which is not particularly limited. Examples of anions include chloride, bromide, iodide, sulfate, phosphate, acetate, mesylate, tosylate, and the like. A combination of different anions can be used. Salts are frequently referred to by the identity of the anion and as such the quaternary ammonium, quaternary phosphonium, guanidinium, pyridinium or imidazolium salt may be a halide salt, nitrate salt, nitrite salt, boron-containing salt, antimony-containing salt, phosphate salt, carbonate salt, carboxylate salt or a combination of two or more of the foregoing. The foregoing salts are of the formula G₄N⁺Y⁻ and G₄P⁺Y⁻, where each Y⁻ is independently an anionic component, which is not particularly limited, and each G is independently C₁₋₃₂ acyl, C₂₋₃₂ alkenyl or alkynyl, C₃₋₈ cycloalkyl, C₆₋₂₄ aryl, C₇₋₂₄ arylalkylene (e.g., benzyl), di(C₁₋₃₂ alkyl)amino, and C₃₋₉ heteroaryl containing 1 to 3 heteroatoms (N, P, O, S, or a combination thereof). Each of the foregoing groups can optionally be substituted with 1 to 4 substituents such as C₁₋₆ alkyl, C₁₋₆ alkoxy, nitro, cyano, halogen, di(C₁₋₃₂ alkyl)amino, C₁₋₆ alkylcarbonyloxy (e.g., H₃CC(O)O-), ₂₋₃₂ alkenyl or alkynyl, C₃₋₈ cycloalkyl, C₆₋₂₄ aryl, C₇₋₂₄ arylalkylene, or C₃₋₉ heteroaryl, provided that the valence of the group G is not exceeded.

Examples of quaternary ammonium salts include tetra(C₁₋₁₆ alkyl) ammonium salts, tetra(C₆₋₂₄)aryl ammonium salts, and tetra(C₇₋₂₄ arylalkylene) ammonium salts.

Examples of specific tetra(C₁₋₁₆ alkyl) ammonium salts include tetraethylammonium bromide, tetrapropylammonium bromide, tetrabutylammonium iodide, tetrabutylammonium bromide, tetrabutylammonium chloride, tetrabutylammonium fluoride, tetrabutylammonium acetate, tetrahexylammonium chloride, tetraheptylammonium chloride, benzyltriethylammonium bromide, hexadecyltrimethylammonium bromide, ALIQUAT 336 (methyltrioctylammonium chloride), ADOGEN 464 (methyltri(C₈-C₁₀ alkyl)ammonium chloride), and 1,6-bis(tributylammonium)hexane dibromide. Examples of tetra(C₆₋₂₄)aryl ammonium salts include tetraphenylammonium bromide.

Dialkyl heterocycloaliphatic salts (16) can be used: 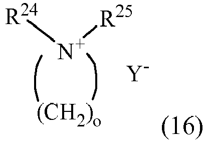 wherein R²⁴ and R²⁵ are each independently an alkyl groups having 1 to 4 carbons and n equals 4 to 6, o is an integer from 1 to 12, and Y⁻ is defined as above.

Bis-alkyl quaternary ammonium salts (17) can be used:

(R²⁶)ₖ-N⁺-(R²⁷)ₘ-N⁺-(R²⁸)ₖ [Y⁻]₂ (17)

wherein R²⁶ and R²⁸ are each independently an alkyl having 1 to 12 carbons, each R²⁷ is a hydrocarbyl group having 1 to 12 carbons provided that all R²⁷ groups, taken together, have 4 to 12 carbons, k is an integer from 1 to 3, and m is 4-k provided that at least three of the R²⁶, R²⁷ and R²⁸ are aliphatic or alicyclic, and Y⁻ is defined as above.

Quaternary phosphonium salts include tetra(C₁₋₁₆)alkyl, tetra(C₆₋₂₄)aryl, mixed (C₇₋₂₄arylalkylene)(C₁₋₁₆alkyl), and mixed (C₆₋₂₄aryl)(C₁₋₁₆alkyl) phosphonium salts, and phosphazenium salts. Examples of quaternary phosphonium salts include tetrabutylphosphonium bromide, tetrabutylphosphonium chloride, methylbenzyltriphenylphosphonium bromide, tetraphenylphosphonium bromide, and trihexyl-tetradecylphosphonium chloride.

Quaternary pyridinium salts include C₁₋₈alkyl pyridinium salts, N-(C₁₋₁₀alkyl)-4-di(C₁₋₁₀)alkylaminopyridinium salts, bis((C₁₋₁₂)alkyl pyridinium) salts.

Examples of N-(C₁₋₁₀)alkyl-4-di(C₁₋₁₀)alkylpyridinium salts include N-butyl-4-dimethylaminopyridinium chloride, N-2-ethylhexyl-4-dimethylaminopyridinium chloride, N-2-ethylexyl-4-methylpiperidinylpyridinium chloride, N-2-ethylhexyl-4-dibutylaminopyridinium chloride, N-2-ethylhexyl-4-dihexylaminopyridinium chloride, and N-neopentyl-4-dihexylaminopyridinium bromide. Examples of bis((C₁₋₁₂)alkyl pyridinium) salts include tetraethylene glycol bis-(4-dimethylaminopyridinium) bismethanesulfonate, 1,8-bis(4-dimethylaminopyridinium)octane dibromide, 1,6-bis(4-dihexylaminopyridinium)hexane dibromide, 1,8-bis(4-dihexylaminopyridinium)octane dibromide, and 1,10-bis(4-dihexylaminopyridinium)decane dibromide. Examples of (C₁₋₈)alkylimidazolium salts include 1-butyl-2,3-dimethylimidazolium chloride and 1-butyl-2,3-dimethylimidazo lium tetrafluoroborate.

Dialkylaminopyridinium salts (14) can be used: 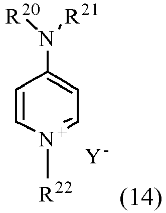 wherein R²⁰ and R²¹ are hydrocarbyl groups having 1 to 13 carbons. The hydrocarbyl groups may be substituted or unsubstituted and branched or not branched. R²⁰ and R²¹ together can form a cyclic hydrocarbyl group. R²² is a linear or branched alkyl group having 4 to 12 carbons. Y⁻ is as defined above.

Dialkylaminopyridinium salts (15) can also be used: 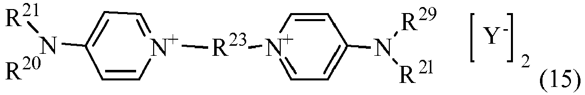 wherein R²⁰, R²¹, and Y⁻ are defined as above. R²³ is a linear hydrocarbyl having 4 to 25 carbon atoms.

Imidazolinium salts include (C₁₋₈)alkylimidazolium salts, and benzimidazolium salts.

Guanidinium salts (12) can be used 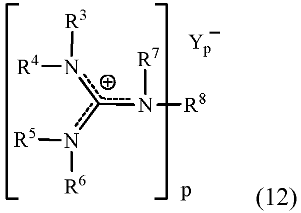 wherein each of R³, R⁴, R⁵, R⁶ and R⁷ is a C₁₋₁₂ primary alkyl radical and R⁸ is a C₁₋₁₂ primary alkyl or C₂₋₁₂ primary alkylene radical, or at least one of the R³-R⁴, R⁵-R⁶ and R⁷-R⁸ combinations with the connecting nitrogen atom forms a heterocyclic radical; Y⁻ is an anion; and n is 1 or 2. The alkyl radicals suitable as R³-R⁶ include primary alkyl radicals, generally containing about 1-12 carbon atoms. R⁷ is usually an alkyl radical of the same structure as R³-R⁶ or a C₂₋₁₂ alkylene radical in which the terminal carbons are primary; most preferably, it is C₂₋₆ alkyl or C₄₋₈ straight chain alkylene. Alternatively, any combination of R³-R⁸ and the corresponding nitrogen atom(s) may form a heterocyclic radical such as piperidino, pyrrolo, or morpholino. Y⁻ can be any anion, for example the conjugate base of a strong acid. Specific examples of Y⁻ are chloride, bromide, and methanesulfonate. The value of p is 1 or 2 depending on whether R⁷ is alkyl or alkylene. Specific guanidinium salts include hexa(C₁₋₆)alkylguanidinium and α,ω-bis(penta(C₁₋₆)alkylguanidinium)(C₁₋₆)alkane, and salts such as hexaethylguanidinium chloride, hexaethylguanidinium bromide, hexa-n-butylguanidinium bromide, and tris(piperidino)guanidinium bromide, 1,6-bis(N,N',N',N",N"-penta-n-butylguanidinium)hexane dibromide and 1,6-bis(N-n-butyl-N',N',N"N"-tetraethylguanidinium)hexane dibromide.

Guanidinium salts include bis-guanidinium alkane salts of structure (13): 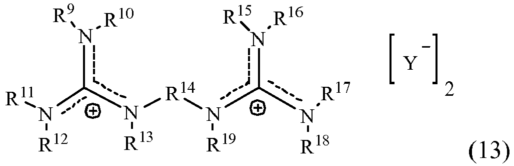 wherein R⁹-R¹³ and R¹⁵-R¹⁹ are each independently selected from the group comprising alkyl, cyclo alkyl, aryl, and aryl alkyl and have 1 to 20 carbons. R¹⁴ is an alkylene group having 2 to 12 carbons, or, more specifically, 4 to 8 carbons. In some embodiments R⁹-R¹³ and R¹⁵-R¹⁹ are each independently alkyl groups having 1 to 12, or, more specifically, 2 to 6 carbons. In some embodiments R⁶ is non-branched. Y⁻ can be any suitable anion referred to in the preceding paragraph and in some embodiments is the anion of a strong acid such as chloride or bromide.

A catalytically active amount of the imidization catalyst can be determined by one of skill in the art without undue experimentation, and can be, for example, more than 0 to 5 mole percent, specifically 0.01 to 2 mole percent, and more specifically 0.1 to 1.5 mole percent, and still more specifically 0.2 to 1.0 mole percent based on the moles of organic diamine (8). The catalyst for the bis(phthalimide) composition can consist essentially, or consist of the foregoing quaternary ammonium salts, quaternary phosphonium salts, guanidinium salts, pyridinium salts, imidazolium salts, and combinations thereof. In some embodiments, the foregoing bis(phthalimide) compositions have less than 100 parts per million (ppm) by weight of the composition of a sodium aryl phosphinate salt, or no detectable amount of a sodium aryl phosphinate salt. As used herein, no detectable amount means that the amount is not detectable by HPLC that has a detection limit of 25 ppm.

Specifically, the catalyst for the bis(phthalimide) composition can consist essentially, or consist of a guanidinium salt, such as a hexaethylguanidinium salt. In some embodiments, these bis(phthalimide) compositions have less than 100 ppm by weight of the bis(phthalimide) of a sodium aryl phosphinate salt, or no detectable amount of a sodium aryl phosphinate salt. Guanidinium salts have enhanced stability over other quaternary ammonium salts. Without being bound by theory, the delocalized nature of the positive charge (which is spread over the three nitrogen atoms and the carbon atom connecting them) is believed to stabilize the catalyst against decomposition at the reaction temperatures used, and thereby increase the effective amount for catalysis present throughout the reaction in comparison to other quaternary ammonium salts. Also, a major decomposition pathway in quaternary ammonium salts is dehydroamination to form an amine and an olefin. When the quaternary ammonium salt is a guanidinium salt, the leaving group is a guanidine, a stronger base, and therefore a weaker leaving group, than the amine that is formed in the decomposition of other quaternary ammonium salts. Thus decomposition of guanidinium salts is less energetically favored than decomposition of other quaternary ammonium salts.

The imidization catalyst can be added any time during the imidization reaction between the substituted phthalic anhydride and the organic diamine. For example, the imidization catalyst can be added at the beginning of the reaction, at the end of the reaction, or anytime during the reaction. The imidization catalyst can also be added continuously or in portions during the course of the reaction. An amount of imidization catalyst effective to catalyze the imidization of the substituted phthalic anhydride and the organic diamine can be added at the beginning of the reaction, for example about 0.2 mole %, based on the moles of organic diamine, and an additional amount can be added at the end of the reaction, to serve as the polymerization catalyst for the manufacture of the polyetherimide.

The imidization reaction is generally conducted in the presence of a relatively non-polar solvent, specifically with a boiling point above about 100°C, and more specifically above about 150°C, for example o-dichlorobenzene, dichlorotoluene, 1,2,4-trichlorobenzene, diphenyl sulfone, a monoalkoxybenzene such as anisole, veratrole, diphenylether, or phenetole. Ortho-dichlorobenzene and anisole can be particularly mentioned.

The bis(phthalimide)s (9) are generally prepared at least 110°C, specifically 150° to 275°C, more specifically 175 to 225°C. At temperatures below 110°C, reaction rates may be too slow for economical operation. Atmospheric or super-atmospheric pressures can be used, for example up to 5 atmospheres, to facilitate the use of high temperatures without causing solvent to be lost by evaporation.

The reaction of the substituted phthalic anhydride (7) with the organic diamine (8) to form bis(phthalimide) (9) is generally conducted for about 0.5 to about 30 hours, specifically about 1 to about 20 hours, more specifically about 1 to about 10 hours, still more specifically about 2 to about 8 hours, and yet more specifically about 3 to about 7 hours. Advantageously, conversion to the bis(phthalimide) is 99% complete, based on the moles of the substituted phthalic anhydride, in less than 6 hours.

The solvent, organic diamine (8) and substituted phthalic anhydride (7) can be combined in amounts such that the total solids content during the reaction to form bis(phthalimide) (9) does not exceed about 25 weight percent (wt%), or about 17 wt%. "Total solids content" expresses the proportion of the reactants as a percentage of the total weight including liquids present in the reaction at any given time.

It can be desirable to have low water content in the imidization reaction mixture. Thus, in some embodiments, the combined substituted phthalic anhydride, organic diamine, solvent and quaternary ammonium, phosphonium, guanidinium, pyridinium or imidazolium salt (reaction mixture) can comprise less than or equal to 200 parts per million parts of the combined components weight (ppm) of water, more specifically, less than or equal to 100 ppm of water, still more specifically, less than or equal to 50 ppm of water, or, yet more specifically, less than or equal to 25 ppm of water. In some embodiments, the combined substituted phthalic anhydride, organic diamine, solvent and quaternary ammonium, phosphonium, guanidinium, pyridinium, or imidazolium salt comprise less than or equal to 100 ppm water.

A molar ratio of substituted phthalic anhydride (7) to diamine (8) of 1.98:1 to 2.04:1, specifically about 2:1 can be used. While other ratios can be employed, a slight excess of anhydride or diamine may be desirable. A proper stoichiometric balance between substituted phthalic anhydride (7) and diamine (8) is maintained to prevent undesirable by-products that can limit the molecular weight of the polyetherimide polymer prepared from the bis(phthalimide), and/or result in polymers with amine end groups. Accordingly, in an embodiment, the catalytic imidization process includes adding diamine (8) to a combination of substituted phthalic anhydride (7) and solvent to form a reaction mixture having a targeted initial molar ratio of substituted phthalic anhydride to diamine; heating the reaction mixture to a temperature of at least 100°C (optionally in the presence of an imidization catalyst); analyzing the molar ratio of the heated reaction mixture to determine the actual initial molar ratio of substituted phthalic anhydride (7) to diamine (8); and, if necessary, adding substituted phthalic anhydride (7) or diamine (8) to the analyzed reaction mixture to adjust the molar ratio of substituted phthalic anhydride (7) to diamine (8) to 1.98:1 to 2.04:1.

In some embodiments, an endcapping agent is formed during imidization, or the imidization is conducted in the presence of an endcapping agent. The endcapping agent can be formed before or during imidization by addition of a monofunctional reactant that reacts with one of the amine groups of diamine (8), thereby "capping" the amine end group. Such mono-capped diamines endcap the polymer during polymerization, and thus can be used to control the molecular weight of the polymer or the end groups of the polymer. Alternatively, or in addition, the monofunctional reactant can be a monofunctional reactant that reacts with the anhydride group of substituted phthalic anhydride (7) to form phthalimides, thereby "capping" the substituted phthalic anhydride end groups. Accordingly, the mono functional reactant has a functional group that reacts with an amine of diamine (8) or with an anhydride group of substituted phthalic anhydride (7), for example, a phthalic anhydride, acyl alkyl halide, acyl aryl halide, aldehyde, ketone, ester, isocyanate, chloroformate, sulfonyl chloride, a C₁₋₁₂ primary amine, and the like. A combination of different monofunctional reactants can be present.

In an embodiment the monofunctional reactant is a phthalic anhydride without a halogen, nitrogen, or other leaving group substitution. For example, when a combination of substituted phthalic anhydride (7) and unsubstituted phthalic anhydride are reacted with organic diamine (8), the product comprises bis(phthalimide) (9) and monofunctional bis(phthalimide) (18) 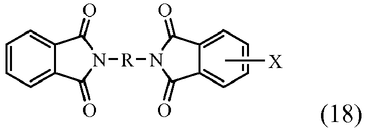 wherein R and X are as defined in formula (9).

Alternatively, a monofunctional reactant can include a primary organic amine can be used to cap substituted phthalic anhydride (7). For example, when the monofunctional reactant is aniline, a combination of diamine (8), aniline, and substituted phthalic anhydride (7) are reacted with to form bis(phthalimide) (9) and monofunctional phthalimide (19) 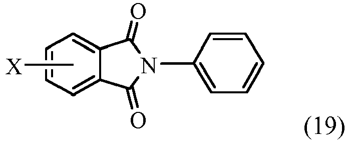 wherein X is as defined in formula (7).

The amount of monofunctional reactant added will depend on the desired amount of endcapping agent. For example, the amount of monofunctional reactant present in the imidization reaction can be more than 0 to 10 mole percent, specifically 1 to 10 mole percent, and more specifically 2 to 10 mole percent, based on total moles of diamine (8) and substituted phthalic anhydride (7). The monofunctional reactant can be added at any time, e.g., to the diamine (8), the substituted phthalic anhydride (7), the solvent, or a combination thereof, before or after imidization has started, in the presence or absence of the imidization catalyst.

Alternatively, or in addition to the above monofunctional reactants, monofunctional phthalimides or monofunctional bis(phthalimides) can be added as endcapping agents. Thus, in some embodiments, the method further comprises the addition of an endcapping agent such as a monofunctional bis(phthalimide) (18) or monofunctional phthalimide (19). Thus, imidization can be conducted by stepwise, simultaneously or essentially simultaneously combining the reactants, i.e., substituted phthalic anhydride (7), organic amine (8), solvent, imidization catalyst, and the monofunctional reactant and/or and endcapping agent such as a monofunctional phthalimide or monofunctional bis(phthalimide). It has been found by the inventors hereof that simultaneously or essentially simultaneously combining the reactants results in lower residuals of starting materials, by-products, and/or intermediates, in the bis(phthalimide) and/or polyetherimide compositions. For example, the imidization process can include: combining the substituted phthalic anhydride and the organic diamine in the solvent, adding a monofunctional reactant and/or a monofunctional phthalimide to the combination of the substituted phthalic anhydride, the organic diamine, and the solvent; and adding the catalyst to the combination of the substituted phthalic anhydride, the organic diamine, the solvent, and the monofunctional reactant and/or the monofunctional phthalimide.

Intermediate products are formed in the imidization reaction of the substituted phthalic anhydride and organic diamine. One of the intermediates is a monoamine having formula (20) 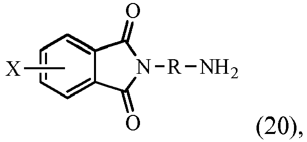 which is formed by reaction of substituted phthalic anhydride (7) with one equivalent of organic diamine (8). When the substituted phthalic anhydride is ClPA (X is Cl and R is meta-phenylene), the monoamine has formula (20a) 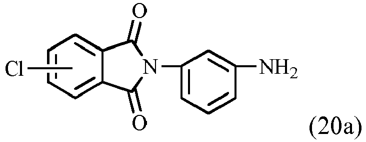 (1-amino-3-N-(chlorophthalimido)benzene). The imidization reaction scheme wherein the substituted phthalic anhydride is 4-ClPA and the organic diamine is meta-phenylenediamine is provided in Scheme 1 below:

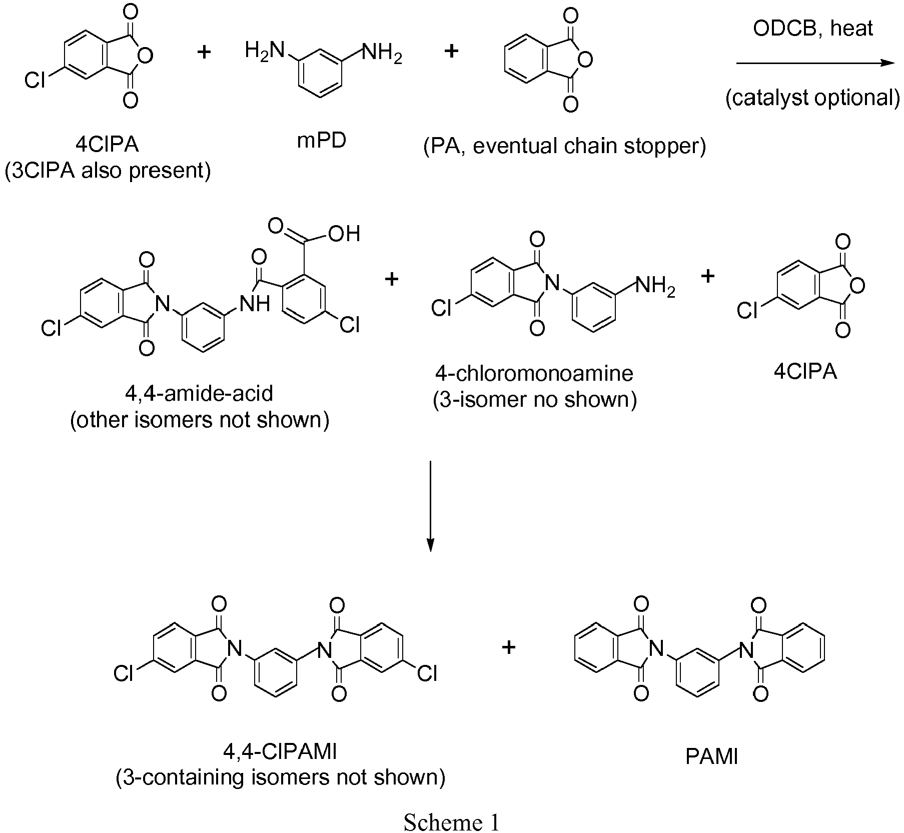

Bis(phthalimide) (9) is formed by reaction of substituted phthalic anhydride (7) with two equivalents of organic diamine (8). Thus, when monoamine (15) is present, unreacted substituted phthalic anhydride will also be present. It is undesirable to have high levels of residual monoamine and substituted phthalic anhydride in the bis(phthalimide) composition. Residual substituted phthalic anhydride can serve as an end capping agent by reacting with the alkali metal salt of the dihydroxy aromatic compound (10) in the polymerization reaction. This results in difficulty in obtaining the desired molecular weight of polyetherimide polymer. It can also result in a greater amount of residual dihydroxy aromatic compound (11) being present in the polymer. Besides the adverse effect on the molecular weight of the polyetherimide composition, high residual substituted phthalic anhydride can volatilize and foul the vacuum equipment used to remove solvent, resulting in operational difficulty. High residual substituted phthalic anhydride and monoamine can result in undesirable amine end groups in the polyetherimide. A high amine end group content in the polyetherimide can result in a thermally unstable and high color polyetherimide as compared to a polyetherimide having a lower amine end group content.

The inventors have discovered that when the imidization catalyst is selected from quaternary ammonium salts, quaternary phosphonium salts, guanidinium salts, pyridinium salts, imidazolium salts, and combinations thereof, the imidization reaction advantageously affords low levels of residual substituted phthalic anhydride and monoamine (20). Thus, in some embodiments, the bis(phthalimide) comprises less than or equal to 0.5 mole percent, 0.4 mole percent, 0.3 mole percent, 0.2 mole percent, 0.15 mole percent, 0.1 mole percent, or 0.05 mole percent of the substituted phthalic anhydride and less than or equal to 2 mole percent, 1.5 mole percent, 1 mole percent, 0.5 mole percent, 0.4 mole percent, 0.3 mole percent, 0.2 mole percent, 0.1 mole percent, or 0.05 mole percent monoamine (20), based on the total monomer content. By total monomer content is meant the total moles of the substituted phthalic anhydride, the organic diamine, and any monofunctional reactant, for example phthalic anhydride, and monofunctional phthalimide used. In some embodiments, if the catalyst reacts in the presence of the solvent at about five hours after the substituted phthalic anhydride and the diamine being reacting, the bis(phthalimide) comprises less than or equal to 0.15 mole percent of the substituted phthalic anhydride and less than or equal to 1.0 mole percent monoamine, based on the total monomer content.

The bis(phthalimide) composition comprises a residue of the imidization catalyst. A residue of the imidization catalyst as defined herein is that portion of the imidization catalyst that remains in the bis(phthalimide) composition after the reacting. The residue of the imidization catalyst can be the imidization catalyst itself, a decomposition or reaction product of the imidization catalyst, or combinations thereof. When the imidization catalyst is a guanidinium salt, the guanidinium salt residue is readily removed from the bis(phthalimide) composition. Thus, in some embodiments, a residue of the guanidinium salt is present in the bis(phthalimide) composition in an amount less than 1000 ppm, based on parts by weight of the bis(phthalimide) composition. In other embodiments, a residue of the guanidinium salt is present in the bis(phthalimide) composition in an amount ranging from 0.05 to 1 mole % based on the moles of the organic diamine.

As described above, sodium aryl phosphinate salts such as sodium phenylphosphinate (SPP) are known in the art as an imidization catalyst for the preparation of bis(phthalimide) (9). The inventive method for manufacture of the bis(phthalimide) does not use a sodium aryl phosphinate salt. Thus the bis(phthalimide) composition can have less than 100 ppm by weight of the bis(phthalimide) composition of a sodium aryl phosphinate salt, specifically no detectable amount of a sodium aryl phosphinate salt.

As has been found by the inventors hereof, the above imidization catalysts provide high conversion of substituted phthalic anhydride (7), organic diamine (8), and the intermediate monoamine (20) to bis(phthalimide) (9) in commercially useful reaction times, such that relatively low levels, or no, residual substituted phthalic anhydride (7), organic diamine (8), and monoamine (20) are present in bis(phthalimide) (9) composition. Thus, the bis(phthalimide) composition can have less than 1000 ppm total of the combination of substituted phthalic anhydride (7), organic diamine (8), and monoamine (20), based on parts by weight of the bis(phthalimide) composition. The low level of residuals means that the bis(phthalimide) composition comprising bis(phthalimide (9), solvent, substituted phthalic anhydride (7), organic diamine (8), and monoamine (20) can be used for the subsequent polymerization step, described below, without purification to remove or reduce the level of residuals. Alternatively, the bis(phthalimide) composition can be subject to further purification as is known in the art before polymerization.

Thus, after imidization, the leaving group X of bis(phthalimide) (9) 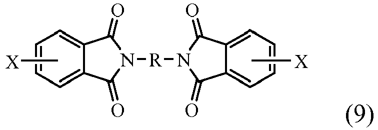 is displaced by reaction with an alkali metal salt of a dihydroxy aromatic compound of formula (10)

MO-Z-OM (10)

wherein M is an alkali metal and Z is as described in formula (1), to provide the polyetherimide of formula (1) 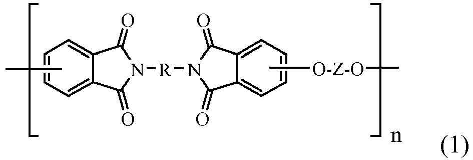 wherein n, R, and Z are as defined above.

Alkali metal M can be any alkali metal, for example lithium, sodium, potassium, and cesium. Thus alkali metal salt (10) is selected from lithium salts, sodium salts, potassium salts, cesium salts, and a combination thereof. Specific alkali metals are potassium or sodium. In some embodiments, M is sodium. The alkali metal salt can be obtained by reaction of the metal with aromatic C₆₋₂₄ monocyclic or polycyclic dihydroxy aromatic compound optionally substituted with 1 to 6 C₁₋₈ alkyl groups, 1 to 8 halogen atoms, or a combination thereof, for example a dihydroxy aromatic compound of formula (11): 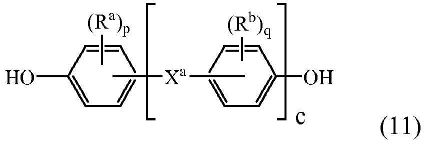 wherein R^{a}, R^{b}, and X^{a} are as described in formula (4). In some embodiments the dihydroxy aromatic compound is selected from bisphenol A, hydroquinone, bisphenol, resorcinol, and combinations thereof. For example, the dihydroxy aromatic compound can be 2,2-bis(4-hydroxyphenyl) propane ("bisphenol A" or "BPA").

Polymerization of bis(phthalimide) (9) with alkali metal salt (10) can be conducted in the presence of a polymerization catalyst. The polymerization catalyst can be the same as the imidization catalyst described above. Examples of polymerization catalysts are the quaternary ammonium salts, quaternary phosphonium salts, guanidinium salts, pyridinium salts, imidazolium salts described above, in particular guanidinium salts. Examples of guanidinium salts are hexaalkylguanidinium and α,ω-bis(pentaalkylguanidinium)alkane salts, and an example of a hexaalkylguanidinium salt is hexaethylguanidinium chloride.

In an embodiment, the polymerization catalyst is the same as the imidization catalyst. A catalytically active amount of the polymerization catalyst is more than 0 to 5 mole percent, specifically 0.01 to 2 mole percent, more specifically 0.1 to 1.5 mole percent, still more specifically, 0.1 to 1 mole percent, and yet more specifically, 0.2 to 0.5 mole percent, based on the moles of alkali metal salt of the dihydroxy aromatic compound. Additional amounts of catalyst can be added to the polymerization to supplement any imidization catalyst already present. When both an imidization catalyst and additional polymerization catalyst are used, the total amount of imidization catalyst and polymerization catalyst is more than 0 to 8 mole %, more specifically 0.02 to 4 mole %, and still more specifically 0.3 to 3 mole %, based on the moles of alkali metal salt of the dihydroxy aromatic compound. In some embodiments, no or substantially no polymerization catalyst is added to the bis(phthalimide) composition after manufacture thereof to form the polyetherimide composition. In these embodiments, residual imidization catalyst from the imidization step can serve as the polymerization catalyst.

Polymerization is generally conducted in the presence of a relatively non-polar solvent, preferably with a boiling point above about 100°C, specifically above about 150°C, for example o-dichlorobenzene, dichlorotoluene, 1,2,4-trichlorobenzene, diphenyl sulfone, a monoalkoxybenzene such as anisole, veratrole, diphenylether, or phenetole. Ortho-dichlorobenzene and anisole can be particularly mentioned. Alternatively, a polar aprotic solvent can be used, illustrative examples of which include dimethylformamide (DMF), dimethylacetamide (DMAc), dimethylsulfoxide (DMSO), and N-methylpyrrolidinone (NMP). A total solids content of the bis(phthalimide) (9) in the polymerization can be 15 to 25 wt%, based on the total weight of the polymerization mixture. "Total solids content" refers to the proportion of the reactants as a percentage of the total weight of the polymerization mixture, including solvents.

Polymerization can be conducted at least 110°C, specifically 150° to 275°C, more specifically 175 to 225°C. At temperatures below 110°C, reaction rates may be too slow for economical operation. Atmospheric or super-atmospheric pressures can be used, for example up to 5 atmospheres, to facilitate the use of high temperatures without causing solvent to be lost by evaporation.

The polymerization can be conducted for about 0.5 to about 30 hours, specifically about 1 to about 20 hours, more specifically about 1 to about 10 hours, still more specifically about 2 to about 8 hours, and yet more specifically about 3 to about 7 hours. The yellowness index of polyetherimide (1) can depend on the polymerization time such that the longer the polymerization time, the higher the yellowness index. Thus it is generally desirable to minimize the polymerization time.

In some embodiments, the alkali metal salt (10) is added to the organic solvent and the water is removed from the mixture, for example as its azeotrope. The bis(phthalimide) (9) is then added and water removed from the mixture, for example as its azeotrope, followed by addition of a catalyst in a pre-dried solution in organic solvent. Water removal from the system can be accomplished in either batch, semi-continuous or continuous processes using means known in the art such as a distillation column in conjunction with one or more reactors. In an embodiment, a mixture of water and non-polar organic liquid distilling from a reactor is sent to a distillation column where water is taken off overhead and solvent is recycled back into the reactor at a rate to maintain or increase the desired solids concentration. Other methods for water removal include passing the condensed distillate through a drying bed for chemical or physical adsorption of water.

The molar ratio of the bis(phthalimide) (9) to the alkali metal salt (10) can be 0.9:1 to 1.1:1, specifically 0.95:1 to 1:0.95, and more specifically 0.98:1 to 1.02:1.

The bis(phthalimide) composition and the polyetherimide composition can be manufactured in separate vessels. Advantageously, when the imidization catalyst and the polymerization catalyst are the same, there is no need to remove the imidization catalyst from the bis(phthalimide) prior to polymerization. Thus, the bis(phthalimide) composition and the polyetherimide composition can be manufactured in the same vessel. Manufacture in the same vessel is also made possible by the reduced levels of residual substituted phthalic anhydride (7), organic diamine (8), and monoamine (20), and the absence of sodium aryl phosphinate salt, in the bis(phthalimide) (9) composition, as discussed below. For example, when a sodium aryl phosphinate salt is used as a catalyst, the amounts of residual substituted phthalic anhydride (7), organic diamine (8), monoamine (20) and sodium aryl phosphinate can be such that the bis(phthalimide) composition must be purified to reduce the levels of these residuals before it can be polymerized with an alkali metal salt of a dihydroxy aromatic compound to form the polyetherimide. The purification can require transfer of the bis(phthalimide) composition to a second vessel. In contrast, when the inventive imidization catalyst is used, the residuals content can be low enough so that the bis(phthalimide) can be polymerized in the same vessel that it was manufactured in without any purification steps.

As described above, in some embodiments an endcapping agent is formed during imidization or added to the imidization. Alternatively, the polymerization further comprises the addition of an endcapping agent. The endcapping agent can be the monofunctional bis(phthalimide) (18), monofunctional phthalimide (19). Monofunctional endcapping agents (18) and (19) react with the alkali metal phenoxide end groups present in the polymerization step. Other endcapping agents reactive with the alkali metal dihydroxy aromatic compound (10) can be used. The amount of the endcapping agent can vary. In an embodiment, for instance, the amount can be 1 to 2 mole equivalents, per mole equivalent of excess alkali metal dihydroxy aromatic compound (10) present in the system. The endcapping agent can be added stepwise, simultaneously or essentially simultaneously with the bis(phthalimide) (9) and the alkali metal salt (10).

Conducting the imidization and polymerization as described above results in polyetherimides with a number of advantageous properties.

Thus in some embodiments, the polyetherimide has less than 1000 ppm total of the combination of the substituted phthalic anhydride, the organic diamine, the alkali metal salt of the dihydroxy aromatic compound, a monofunctional reactant, and a monofunctional phthalimide, based on parts by weight of the polyetherimide.

The polyetherimide composition comprises a residue of the imidization catalyst and optionally a residue of the polymerization catalyst. A residue of the imidization catalyst or polymerization catalyst as defined herein is that portion of the imidization catalyst or polymerization catalyst that remains in the polyetherimide composition after the reacting and any subsequent isolation steps. The residue of the imidization catalyst or polymerization catalyst can be the imidization catalyst or polymerization catalyst itself, a decomposition or reaction product of the imidization catalyst or polymerization catalyst, or combinations thereof. When the imidization catalyst and/or the polymerization catalyst are a guanidinium salt, the guanidinium salt residue is readily removed from the polyetherimide composition. In some embodiments, no polymerization catalyst is added, the imidization catalyst serving to catalyze both the imidization step and the polymerization step. In these embodiments, a residue of the guanidinium salt is present in an amount less than 1000 ppm, based on parts by weight of the polyetherimide composition. In the embodiments an imidization catalyst and polymerization catalyst are both added, and both catalysts are a guanidinium salt, a residue of the guanidinium salt is present in an amount less than 2000 ppm, based on parts by weight of the polyetherimide.

In some embodiments, the polyetherimide has less than 100 ppm by weight, based on parts by weight of the polyetherimide, of a sodium aryl phosphinate salt. In some embodiments, the polyetherimide has no detectable amount of a sodium aryl phosphinate salt.

The polyetherimide has physical properties that make it useful as a molding composition. For example, the polyetherimide can have an R* value that is less than or equal to 2.4, wherein R* is the ratio of viscosities measured under nitrogen at 1 rad/sec and 100 rad/sec at the temperature that gives a viscosity of 20,000 poise at 100 rad/sec.

The polyetherimide can have a yellowness index that is less than 120, measured on a 0.5 g solution of the polyetherimide in 10 mL of methylene chloride, and in accordance with ASTM E313.

The polyetherimide can have other physical properties that make it useful as a molding composition. For example, the polyetherimide can have a weight average molecular weight (Mw) of 5,000 to 100,000 grams per mole (g/mole) as measured by gel permeation chromatography (GPC). In some embodiments the Mw can be 10,000 to 80,000 g/mole. The molecular weights as used herein refer to the absolute weight averaged molecular weight (Mw).

During extrusion of the polyetherimide, the Mw of the polyetherimide in the feed to the extruder is generally less than the Mw of the extruded material, that is, extrusion results in an increase (build) in Mw. The molecular weight build across the extruder is problematic in that Mw of the polyetherimide after extrusion is not predictable. Thus, the desired Mw of the polyetherimide is not always obtained. It has been found that the Mw build across the extruder is less when the imidization catalyst is a quaternary ammonium salt, a quaternary phosphonium salt, a guanidinium salt, a pyridinium salt, or an imidazolium salt as compared to SPP. The Mw build when the imidization catalyst is a quaternary ammonium salt a quaternary phosphonium salt, a guanidinium salt, a pyridinium salt, or an imidazolium salt, can be at least 10% less, specifically at least 20% less, and more specifically, at least 30% less, as compared to the Mw build when the imidization catalyst is SPP. For example, the polyetherimide formed by polymerization of the bis(phthalimide) and the disodium salt of bisphenol A retains at least 80% of its weight average molecular weight, as measured by gel permeation chromatography after exposure to a temperature of 134°C for 4 days under steam and under pressure. Thus the use of quaternary ammonium salt a quaternary phosphonium salt, a guanidinium salt, a pyridinium salt, or an imidazolium salt as the imidization catalyst affords polyetherimide resin with a more predictable Mw, which eliminates the need to manage polyetherimide resin produced with variable Mw.

The polyetherimide can have an intrinsic viscosity greater than or equal to 0.2 deciliters per gram (dL/g) as measured in m-cresol at 25°C. Within this range the intrinsic viscosity can be 0.35 to 1.0 dL/g, as measured in m-cresol at 25°C.

The polyetherimide can have a glass transition temperature of greater than 180°C, specifically of 200°C to 500°C, as measured using differential scanning calorimetry (DSC) per ASTM test D3418. In some embodiments the polyimide, an in particular a polyetherimide has a glass transition temperature of 240 to 350°C.

The polyetherimide can have a melt index of 0.1 to 10 grams per minute (g/min), as measured by ASTM DI 238 at 340 to 370°C, using a 6.7 kilogram (kg) weight.

The polyetherimide can be melt-mixed with other polymers, fillers, and/or additives ordinarily incorporated into polymer compositions of this type to form compositions having the desired properties, for example a molding composition, with the proviso that the polymers, fillers, and/or additives are selected so as to not significantly adversely affect the desired properties of the polyetherimide composition.

The polyetherimides can be formulated to provide a wide variety of polyetherimide compositions for the manufacture of articles. The polyetherimide compositions can optionally comprise a filler. In some instances it is desired to have polyetherimide compositions wherein a filler is substantially absent. "Substantially absent" means that the composition has less than 3 wt% of a filler, and in other embodiments less than 1 wt% filler by weight of the composition. In other instances, it is advantageous to have polyetherimide compositions wherein a filler is absent.

The filler can be a reinforcing filler, for example a flat, plate-like, and/or fibrous filler. Typically, the flat, plate-like filler has a length and width at least ten times greater than its thickness, where the thickness is from 1 to 1000 micrometers. Exemplary reinforcing fillers of this type include glass flakes, mica, flaked silicon carbide, aluminum diboride, aluminum flakes, and steel flakes; wollastonite including surface-treated wollastonite; calcium carbonate including chalk, limestone, marble and synthetic, precipitated calcium carbonates, generally in the form of a ground particulates; talc, including fibrous, modular, needle shaped, and lamellar talc; kaolin, including hard, soft, calcined kaolin, and kaolin comprising various coatings known in the art to facilitate compatibility with the polymeric matrix resin; mica; and feldspar.

Exemplary reinforcing fillers also include fibrous fillers such as short inorganic fibers, natural mineral fibrous fillers, single crystal fibers, glass fibers, ceramic fibers and organic reinforcing fibrous fillers. Short inorganic fibers include borosilicate glass, carbon fibers, and those derived from blends comprising at least one of aluminum silicates, aluminum oxides, magnesium oxides, and calcium sulfate hemihydrate. Single crystal fibers or "whiskers" include silicon carbide, alumina, boron carbide, iron, nickel, and copper single crystal fibers. Glass fibers, including glass fibers such as E, ECR, S, and NE glasses and quartz, and the like can also be used.

Such reinforcing fillers can be provided in the form of monofilament or multifilament fibers and can be used either alone or in combination with other types of fiber, for example, co-weaving or core/sheath, side-by-side, orange-type or matrix and fibril constructions, or by other methods known to one skilled in the art of fiber manufacture. Typical cowoven structures include glass fiber-carbon fiber, carbon fiber-aromatic polyimide (aramid) fiber, and aromatic polyimide fiber-glass fiber. Fibrous fillers can be supplied in the form of, for example, rovings, woven fibrous reinforcements, such as 0-90 degree fabrics, non-woven fibrous reinforcements such as continuous strand mat, chopped strand mat, tissues, papers and felts and 3-dimensionally woven reinforcements, performs and braids.

The reinforcing fibers can have a diameter of 5 to 25 micrometers, specifically diameters of 9 to 15 micrometers. In preparing molding compositions it is convenient to use reinforcing fibers such as fiberglass in the form of chopped strands of from 3 millimeters to 15 millimeters long. In articles molded from these compositions, on the other hand, shorter lengths will typically be encountered because during compounding considerable fragmentation can occur. Combinations of rigid fibrous fillers with flat, plate-like fillers can be used, for example to reduce warp of a molded article.

In some applications it can be desirable to treat the surface of the filler with a chemical coupling agent to improve adhesion to a thermoplastic resin in the composition. Examples of useful coupling agents are alkoxy silanes and alkoxy zirconates. Amino, epoxy, amide, or thio functional alkoxy silanes are especially useful. Fiber coatings with high thermal stability are preferred to prevent decomposition of the coating, which could result in foaming or gas generation during processing at the high melt temperatures required to form the compositions into molded parts.

The amount of reinforcing filler used in the polyetherimide composition can vary widely, and is that amount effective to provide the desired physical properties and flame resistance. In some instances the reinforcing filler is present in an amount from more than 10 to 60 wt%, more specifically 15 to 40 wt%, and even more specifically 20 to 35 wt% each based on the total weight of the composition.

The polyetherimide composition can optionally further comprise one or more other types of particulate fillers. Exemplary particulate fillers include silica powder, such as fused silica and crystalline silica; boron-nitride powder and boron-silicate powders; alumina, and magnesium oxide (or magnesia); silicate spheres; flue dust; cenospheres; aluminosilicate (armospheres); natural silica sand; quartz; quartzite; perlite; tripoli; diatomaceous earth; synthetic silica; and combinations thereof. All of the above fillers can be surface treated with silanes to improve adhesion and dispersion with the polymeric matrix resin. When present, the amount of additional particulate filler in the polyetherimide composition can vary widely, and is that amount effective to provide the desired physical properties and flame resistance. In some instances the particulate filler is present in an amount from 1 to 80 wt%, specifically 5 to 30 wt%, more specifically 5 to 20 wt%, each based on the total weight of the composition. Alternatively, in some embodiments, our compositions do not contain appreciable amounts of fillers and in some embodiments, there are no detectable amounts of fillers, i.e., fillers are substantially absent or absent from the compositions. Accordingly, in some instances, the filler is present in an amount from 0 wt% to an amount that is less than or equal to an amount selected from 80 wt%, 75 wt%, 70 wt%, 65 wt%, 60 wt%, 55 wt%, 50 wt%, 45 wt%, 40 wt%, 35 wt%, 30 wt%, 25 wt%, 20, 15 wt%, 10 wt%, 5 wt%, and 1 wt%, each based on the total weight of the composition.

The polyetherimide compositions can include various additives ordinarily incorporated into polymer compositions of this type, with the proviso that the additives are selected so as to not significantly adversely affect the desired properties of the composition. Exemplary additives include catalysts, impact modifiers, fillers, antioxidants, thermal stabilizers, light stabilizers, ultraviolet light (UV) absorbing additives, quenchers, plasticizers, lubricants, mold release agents, antistatic agents, visual effect additives such as dyes, pigments, and light effect additives, flame retardants, anti-drip agents, and radiation stabilizers. In some embodiments, the polyetherimide composition comprises a solvent, and the composition is in the form of a varnish. Combinations of additives can be used, for example a combination of a heat stabilizer, a mold release agent, and optionally an ultraviolet light stabilizer. In general, the additives are used in the amounts generally known to be effective. The foregoing additives (except any fillers) are generally present in an amount from 0.005 to 20 wt%, specifically 0.01 to 10 wt%, based on the total weight of the composition. Alternatively, in some embodiments, our compositions do not contain appreciable amounts of additives, and in some embodiments, there are no detectable amounts of additives, i.e., additives are substantially absent or absent from the compositions. Accordingly, the foregoing additives (except any fillers) can be present in an amount from 0 to less than or equal to an amount selected from 20 wt%, 19 wt% 18 wt%, 17 wt%, 16 wt%, 15 wt%, 14 wt%, 13 wt%, 12 wt%, 11 wt%, 10 wt%, 9 wt%, 8 wt%, 7 wt%, 6 wt%, 5 wt%, 4 wt%, 3 wt%, 2 wt%, 1 wt%, and 0.0001 wt%, based on the total weight of the composition. In another embodiment, no appreciable amount of any additive other than a heat stabilizer, a mold release agent, and optionally an ultraviolet light stabilizer is present in the compositions. In still another embodiment, no detectable amount of any additive other than a heat stabilizer, a mold release agent, and optionally an ultraviolet light stabilizer is present in the compositions.

Suitable antioxidants can be compounds such as phosphites, phosphonites and hindered phenols or mixtures thereof. Phosphorus-containing stabilizers including triaryl phosphites and aryl phosphonates are useful additives. Difunctional phosphorus containing compounds can also be unseeded. Preferred stabilizers can have a molecular weight greater than or equal to 300. Some exemplary compounds are tris-di-tert-butylphenyl phosphite available from Ciba Chemical Co. as IRGAPHOS 168 and bis (2,4-dicumylphenyl) pentaerythritol diphosphite available commercially from Dover Chemical Co. as DOVERPHOS S-9228.

Examples of phosphites and phosphonites include: triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, bis(2,6-di-tert-butyl-4-methylphenyl)-pentaerythritol diphosphite, diisodecyloxy pentaerythritol diphosphite, bis(2,4-di-tert-butyl-6-methylphenyl)pentaerythritol diphosphite, bis(2,4,6-tris(tert-butylphenyl)pentaerythritol diphosphite, tristearyl sorbitol tri-phosphite, tetrakis(2,4-di-tert-butyl-phenyl) 4,4'-biphenylene diphosphonite, bis(2,4-di-tert-butyl-6-methylphenyl) methyl phosphite, bis(2,4-di-tert-butyl-6-methylphenyl) ethyl phosphite, 2,2',2"-nitrilo[triethyl tris(3,3',5,5'-tetra-tert-butyl-1,1 '-biphenyl-2,2'-diyl)phosphite], 2-ethylhexyl(3,3',5,5'-tetra-tert-butyl-1,1'-biphenyl-2,2'-diyl)phosphite and 5-butyl-5-ethyl-2-(2,4,6-tri-tert-butylphenoxy)-1,3,2-dioxaphosphirane.

Combinations comprising more than one organophosphorous compound are contemplated. When used in combination the organo phosphorous compounds can be of the same type or different types. For example, a combination can comprise two phosphites or a combination can comprise a phosphite and a phosphonite. In some embodiments, phosphorus-containing stabilizers with a molecular weight greater than or equal to 300 are useful. Phosphorus-containing stabilizers, for example an aryl phosphite are usually present in the composition in an amount from 0.005 to 3 wt%, specifically 0.01 to 1.0 wt%, based on total weight of the composition.

Hindered phenols can also be used as antioxidants, for example alkylated monophenols, and alkylated bisphenols or poly phenols. Exemplary alkylated monophenols include 2,6-di-tert-butyl-4-methylphenol; 2-tert-butyl-4,6-dimethylphenol; 2,6-di-tert-butyl-4-ethylphenol; 2,6-di-tert-butyl-4-n-butylphenol; 2,6-di-tert-butyl-4-isobutylphenol; 2,6-dicyclopentyl-4-methylphenol; 2-(alpha-methylcyclohexyl)-4,6-dimethylphenol; 2,6-dioctadecyl-4-methylphenol; 2,4,6-tricyclohexylphenol; 2,6-di-tert-butyl-4-methoxymethylphenol; nonyl phenols which are linear or branched in the side chains, for example, 2,6-di-nonyl-4-methylphenol; 2,4-dimethyl-6-(1'-methylundec-1'-yl)phenol; 2,4-dimethyl-6-(1'-methylheptadec-1'-yl)phenol; 2,4-dimethyl-6-(1'-methyltridec-1'-yl)phenol and mixtures thereof. Exemplary alkylidene bisphenols include 2,2'-methylenebis(6-tert-butyl-4-methylphenol), 2,2'-methylenebis(6-tert-butyl-4-ethylphenol), 2,2'-methylenebis[4-methyl-6-(alpha-methylcyclohexyl)-phenol], 2,2'-methylenebis(4-methyl-6-cyclohexylphenol), 2,2'-methylenebis(6-nonyl-4-methylphenol), 2,2'-methylenebis(4,6-di-tert-butylphenol), 2,2'-ethylidenebis(4,6-di-tert-butylphenol), 2,2'-ethylidenebis(6-tert-butyl-4-isobutylphenol), 2,2'-methylenebis[6-(alpha-methylbenzyl)-4-nonylphenol], 2,2'-methylenebis[6-(alpha, alpha-dimethylbenzyl)-4-nonylphenol], 4,4'-methylenebis-(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methyl-phenyl)-3-n-dodecylmercaptobutane , ethylene glycol bis[3,3-bis(3'-tert-butyl-4'-hydroxyphenyl)butyrate], bis(3-tert-butyl-4-hydroxy-5-methylphenyl)dicyclopentadiene, bis[2-(3'-tert-butyl-2'-hydroxy-5'-methylbenzyl)-6-tert-butyl-4-methylphenyl]terephthalate, 1,1-bis-(3,5-dimethyl-2-hydroxyphenyl)butane, 2,2-bis-(3,5-di-tert-butyl-4-hydroxyphenyl)propane, 2,2-bis-(5-tert-butyl-4-hydroxy2-methylphenyl)-4-n-dodecylmercaptobutane, 1,1,5,5-tetra-(5-tert-butyl-4-hydroxy-2-methylphenyl)pentane and mixtures thereof.

The hindered phenol compound can have a molecular weight of greater than or equal to 300 g/mole. The high molecular weight can help retain the hindered phenol moiety in the polymer melt at high processing temperatures, for example greater than or equal to 300°C. Hindered phenol stabilizers, are usually present in the composition in an amount from 0.005 to 2 wt%, specifically 0.01 to 1.0 wt%, based on total weight of the composition.

Examples of mold release agents include both aliphatic and aromatic carboxylic acids and their alkyl esters, for example, stearic acid, behenic acid, pentaerythritol tetrastearate, glycerin tristearate, and ethylene glycol distearate. Polyolefins such as high-density polyethylene, linear low-density polyethylene, low-density polyethylene and similar polyolefin homopolymers and copolymers can also be used a mold release agents. Mold release agents are typically present in the composition at 0.05 to 10 wt%, based on total weight of the composition, specifically 0.1 to 5 wt%. Preferred mold release agents will have high molecular weight, typically greater than 300, to prevent loss of the release agent from the molten polymer mixture during melt processing.

In particular, an optional polyolefin can be added to modify the chemical resistance characteristics and mold release characteristics of the polyetherimide composition. Homopolymers such as polyethylene, polypropylene, polybutene can be used either separately or in combination. Polyethylene can be added as high-density polyethylene (HDPE), low-density polyethylene (LDPE) or branched polyethylene. Polyolefins can also be used in copolymeric form with compounds containing carbonic acid radicals such as maleic acid or citric acid or their anhydrides, acid compounds containing acrylic acid radicals such as acrylic acid ester, and the like, as well as combinations comprising at least one of the foregoing. When present, the polyolefin, in particular HDPET, is used in an amount from more than 0 to 10 wt%, specifically 0.1 to 8 wt%, more specifically from 0.5 to 5 wt%, all based on the total weight of the polyetherimide composition.

In some embodiments, the polyetherimide composition can further include at least one additional polymer. Examples of such additional polymers include and are not limited to PPSU (polyphenylene sulfone), polyetherimides, PSU (polysulfone), PPE (polyphenylene ether), PFA (perfluoroalkoxy alkane), MFA (co-polymer of TFE tetrafluoroethylene and PFVE perfluorinated vinyl ether), FEP (fluorinated ethylene propylene polymers), PPS (poly(phenylene sulfide), PTFE (polytetrafluoroethylene), PA (polyamide), PBI (polybenzimidizole), PAI (poly(amide-imide)), poly(ether sulfone), poly(aryl sulfone), polyphenylene, polybenzoxazoles, polybenzthiazoles, as well as blends and co-polymers thereof. When present, the polymer is used in an amount from more than 0 to 20 wt%, specifically 0.1 to 15 wt%, and more specifically from 0.5 to 10 wt%, all based on the total weight of the polyetherimide composition. In some embodiments, no polymer other than the polyetherimide as described herein is present in the polyetherimide composition.

Colorants such as pigment and/or dye additives can also optionally be present. Useful pigments can include, for example, inorganic pigments such as metal oxides and mixed metal oxides such as zinc oxide, titanium dioxide, iron oxides, or the like; sulfides such as zinc sulfides, or the like; aluminates; sodium sulfo-silicates sulfates, chromates, or the like; carbon blacks; zinc ferrites; ultramarine blue; organic pigments such as azos, di-azos, quinacridones, perylenes, naphthalene tetracarboxylic acids, flavanthrones, isoindolinones, tetrachloroisoindolinones, anthraquinones, enthrones, dioxazines, phthalocyanines, and azo lakes; Pigment Red 101, Pigment Red 122, Pigment Red 149, Pigment Red 177, Pigment Red 179, Pigment Red 202, Pigment Violet 29, Pigment Blue 15, Pigment Blue 60, Pigment Green 7, Pigment Yellow 119, Pigment Yellow 147, Pigment Yellow 150, and Pigment Brown 24; or combinations comprising at least one of the foregoing pigments. Pigments are generally used in amount from 0 to 10 wt%, specifically 0 to 5 wt%, based on the total weight of the composition. In some instances, where improved impact is desired pigments such as titanium dioxide will have a mean particle size of less than 5 microns.

The polyetherimide composition can also optionally include a fluoropolymer in an effective amount to provide anti-drip or other beneficial properties to the resin composition. In one instance the fluoropolymer is present in an amount 0.01 to 5.0 wt% of the composition. Examples of suitable fluoropolymers and methods for making such fluoropolymers are set forth, for example, in U.S. Pat. Nos. 3,671,487, 3,723,373, and 3,383,092. Suitable fluoropolymers include homopolymers and copolymers that comprise structural units derived from one or more fluorinated alpha-olefin monomers, for example, CF₂=CF₂, CHF=CF₂, CH₂=CF₂ and CH₂=CHF and fluoro propylenes such as, for example, CF₃CF=CF₂, CF₃CF=CHF, CF₃CH=CF₂, CF₃CH=CH₂, CF₃CF=CHF, CHF₂CH=CHF and CF₃CF=CH₂.

Copolymers comprising structural units derived from two or more fluorinated alpha-olefin monomers can also be used, for example poly(tetrafluoroethylene-hexafluoroethylene), as well as copolymers comprising structural units derived from one or more fluorinated monomers and one or more non-fluorinated monoethylenically unsaturated monomers that are copolymerizable with the fluorinated monomers such as poly(tetrafluoroethylene-ethylene-propylene) copolymers. Suitable non-fluorinated monoethylenically unsaturated monomers include for example, alpha-olefin monomers such as ethylene, propylene, butene, acrylate monomers such as, methyl methacrylate, butyl acrylate, and the like, with poly(tetrafluoroethylene) homopolymer (PTFE) preferred.

The fluoropolymer can be pre-blended in some manner with a polymer such as an aromatic polycarbonate or polyimide resin. For example, an aqueous dispersion of fluoropolymer and a polycarbonate resin can be steam precipitated to form a fluoropolymer concentrate for use as a drip inhibitor additive in thermoplastic resin compositions, as disclosed, for example, in U.S. Pat. No. 5,521,230. Alternatively, the fluoropolymer can be encapsulated.

In some instances it is desired to have polyetherimide compositions that are essentially free of bromine and chlorine. "Essentially free" of bromine and chlorine means that the composition has less than 3 wt% of bromine and chlorine, and in other embodiments less than 1 wt% bromine and chlorine by weight of the composition. In other embodiments, the composition is halogen free. "Halogen free" is defined as having a halogen content (total amount of fluorine, bromine, chlorine and iodine) of less than or equal to 1000 parts by weight of halogen per million parts by weight of the total polyetherimide composition (ppm). The amount of halogen can be determined by ordinary chemical analysis such as atomic absorption.

The polyetherimide composition can be prepared by blending the ingredients under conditions for the formation of an intimate blend. Such conditions often include melt mixing in single or twin screw-type extruders, mixing bowl, or similar mixing devices that can apply a shear to the components. Twin-screw extruders are often preferred due to their more intensive mixing capability and self-wiping capability, over single screw extruders. It is often advantageous to apply a vacuum to the blend through at least one vent port in the extruder to remove volatile impurities in the composition. Often it is advantageous to dry the polyetherimide composition prior to melt mixing. The melt mixing is often done at 290 to 340°C to avoid excessive polymer degradation while still allowing sufficient melting to get an intimate polymer mixture free of any unbelted components. The polymer blend can also be melt filtered using a 40 to 100 micrometer candle or screen filter to remove undesirable black specks or other heterogeneous contaminants.

In an exemplary process, the polyetherimide, any other polymers, and any additives are placed into an extrusion compounder to produce a continuous strand that is cooled and then chopped into pellets. In another procedure, the components are mixed by dry blending, and then fluxed on a mill and comminuted, or extruded and chopped. The composition and any optional components can also be mixed and directly molded, e.g., by injection or transfer molding techniques. Preferably, all of the components are freed from as much water as possible. In addition, compounding is carried out to ensure that the residence time in the machine is short; the temperature is carefully controlled; the friction heat is utilized; and an intimate blend between the components is obtained.

The polyetherimide composition can be formed into an article by any number of methods including shaping, extruding (including profile extrusion), thermoforming, and molding, including injection molding, compression molding, gas assist molding, structural foam molding, and blow molding. In some embodiments, a method of forming an article comprises shaping, extruding, blow molding, or injection molding the composition to form the article. The polyetherimide compositions can also formed into articles using thermoplastic processes such as film extrusion, sheet extrusion, melt casting, blown film extrusion, and calendaring. Co-extrusion and lamination processes can be used to form composite multi-layer films or sheets. The article is selected from a sheet, film, multilayer sheet, multilayer film, molded part, extruded profile, coated part, pellets, powder, foam, fiber, fibrids, flaked fibers, and combinations thereof.

The polyetherimide composition can be molded into an article with any equipment conventionally used for molding thermoplastic compositions, such as a Newbury or van Dorn type injection-molding machine with conventional cylinder temperatures of 250°C to 320°C, and conventional mold temperatures of 55°C to 120°C.

The polyetherimide composition exhibits improved hydrolytic stability. (The term "hydrostable" as used herein, means "hydrolytically stable.") In some embodiments, when the polyetherimide composition is exposed to a temperature of 110°C for 10 days under steam and under pressure, the composition has a melt flow rate increase that is less than 100% greater than a melt flow rate of the composition at 0 days steam and pressure exposure. Melt flow can be measured at 295°C and a load of 6.7 kg according to ASTM D1238-10.

In some embodiments, the polyetherimide composition retains at least 60% of its initial tensile strength, after exposure to 110°C steam for 20 days. The initial tensile strength of the composition at yield can be from 9 to 15 MPa. Tensile strength can be measured according to ASTM D638-10. In some embodiments, the improved hydrolytic stability is obtained when the polyetherimide composition comprises less than 100 ppm by weight, based on the weight of the polyetherimide composition of a sodium aryl phosphinate salt.

The polyetherimide composition can exhibit a number of other advantageous physical properties. In some embodiments the polyetherimide composition has a melt viscosity of 50 to 20,000 Pascal-seconds, specifically 100 to 15,000 Pascal-seconds, or more specifically, 200 to 10,000 Pascal-seconds at 380°C as measured by ASTM method D3835 using a capillary rheometer with a shear rate of 100 to 10,000 1/sec.

In another embodiment, the polyetherimide composition can have a heat deflection temperature (HDT) of greater than or equal to 120°C, specifically 170°C to 400°C, measured according to ASTM D648 on a 3.2 mm bar at 0.46 MPa (66 psi).

The polyetherimide composition can have a melt flow rate (MFR) at 360°C of greater than 10 grams per 10 minutes, when measured in accordance with ASTM D1238 at 295°C using a load of 6.7 kg.

Because the polyetherimide compositions have improved hydrolytic stability, they are useful in many applications that require toughness and clarity after exposure to hot water or steam. Examples of applications include: food service, medical, lighting, lenses, sight glasses, windows, enclosures, safety shields, and the like. The high melt flow of the polyetherimide composition allows it to be molded into intricate parts with complex shapes and/or thin sections and requiring long polymer melt flow lengths. Examples of articles include, but are not limited to, cookware, medical devices, trays, plates, handles, helmets, animal cages, electrical connectors, enclosures for electrical equipment, engine parts, automotive engine parts, lighting sockets and reflectors, electric motor parts, power distribution equipment, communication equipment, computers and the like, including devices that have molded in snap fit connectors. Other articles include, for example, fibers, sheets, films, multilayer sheets, multilayer films, molded parts, extruded profiles, coated parts and foams: windows, luggage racks, wall panels, chair parts, lighting panels, diffusers, shades, partitions, lenses, skylights, lighting devices, reflectors, ductwork, cable trays, conduits, pipes, cable ties, wire coatings, electrical connectors, air handling devices, ventilators, louvers, insulation, bins, storage containers, doors, hinges, handles, sinks, mirror housing, mirrors, toilet seats, hangers, coat hooks, shelving, ladders, hand rails, steps, carts, trays, cookware, food service equipment, communications equipment and instrument panels. The polyetherimide compositions can also be made into film and sheet as well as components of laminate systems.

In summary, a method for the manufacture of a bis(phthalimide) composition comprises catalyzing imidization of a substituted phthalic anhydride and an organic diamine with a catalyst, in the presence of a solvent, the catalyst being selected from quaternary ammonium salts, quaternary phosphonium salts, guanidinium salts, pyridinium salts, imidazolium salts, and combinations thereof; wherein the substituted phthalic anhydride has a formula 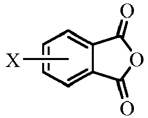 and
the organic diamine has a formula H₂N-R-NH₂; to provide the bis(phthalimide) composition comprising a residue of the catalyst, and a bis(phthalimide) of the formula 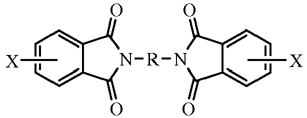 wherein, in the foregoing formulae, X is selected from fluoro, chloro, bromo, iodo, nitro, and combinations thereof, specifically X is chloro, fluoro, bromo, or nitro, more specifically, X is chloro; and R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 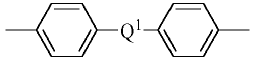 wherein Q¹ is a member selected from the group of a single bond, -O-, -S-, -C(O)-, -SO₂-, - SO-, -C_{y}H_{2y} wherein y is an integer from 1 to 5, and combinations thereof, specifically, R is a divalent radical of the formula -(C₆H₁₀)_{z}- wherein z is an integer from 1 to 4, 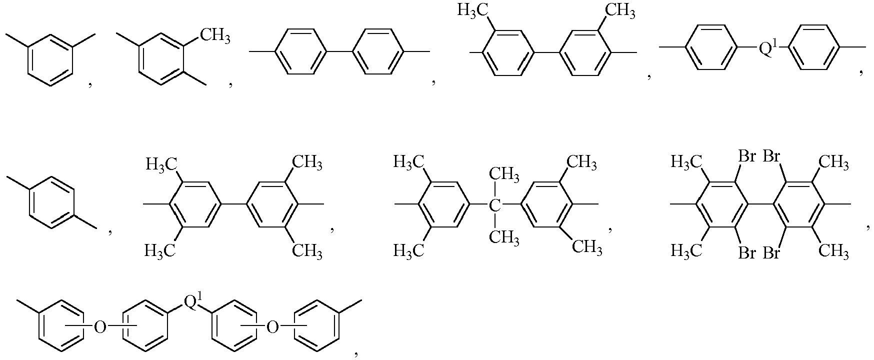 and combinations thereof, wherein Q¹ is selected from a single bond, -O-, -S-, -C(O)-, -SO₂-, -SO-, -C_{y}H_{2y}- wherein y is an integer from 1 to 5, and combinations thereof, more specifically, R is m-phenylene, p-phenylene, diarylsulfone, a group of the formula 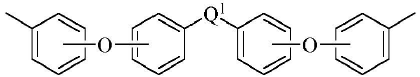 wherein Q¹ -is a member selected from the group of a single bond, -O-, -S-, -C(O)-, -SO₂-, - SO-, -C_{y}H_{2y} wherein y is an integer from 1 to 5, and combinations thereof; wherein conversion to the bis(phthalimide) is 99% complete, based on the moles of the substituted phthalic anhydride, in less than 6 hours.

In the foregoing method for the manufacture of a bis(phthalimide) composition, at least one of the following conditions apply: a polyetherimide formed by catalyzing polymerization of the bis(phthalimide) and a dialkali metal salt of a dihydroxy aromatic compound such as bisphenol A, hydroquinone, bisphenol, resorcinol, and combinations thereof, with the catalyst that catalyzes imidization of the substituted phthalic anhydride and the diamine has: (i) an R* of less than or equal to 2.4, wherein R* is the ratio of viscosities measured under nitrogen at 1 rad/sec and 100 rad/sec at the temperature that gives a viscosity of 20,000 poise at 100 rad/sec; and/or (ii) a yellowness index that is less than 120, measured on a 0.5 gram solution of the polyetherimide in 10 mL of methylene chloride, and in accordance with ASTM E313; a polyetherimide formed by polymerization of the bis(phthalimide) and the disodium salt of bisphenol A retains at least 80% of its weight average molecular weight, as measured by gel permeation chromatography after exposure to a temperature of 134°C for 4 days under steam and under pressure; the stoichiometric ratio of the substituted phthalic anhydride to the organic diamine is 1.98:1 to 2.04:1; the method further comprises determining the stoichiometric ratio of the substituted phthalic anhydride to the organic diamine during the imidization of the substituted phthalic anhydride and the organic diamine; and optionally adjusting the stoichiometric ratio by adding additional substituted phthalic anhydride or organic diamine; the method comprises combining the substituted phthalic anhydride, the organic diamine, the solvent, and the catalyst before heating the combination; the method further comprises combining the substituted phthalic anhydride, the organic diamine, and the solvent, heating the combined substituted phthalic anhydride, the organic diamine, and the solvent, and then adding the catalyst to the heated combination; the method further comprises adding a monofunctional reactant to the substituted phthalic anhydride, the organic diamine, the solvent, and combinations thereof before adding the catalyst, or adding a monofunctional phthalimide to the substituted phthalic anhydride, the organic diamine, the solvent, and combinations thereof before adding the catalyst; the method comprises combining the substituted phthalic anhydride and the organic diamine in the solvent; adding a monofunctional reactant or a monofunctional phthalimide to the combination of the substituted phthalic anhydride, the organic diamine, and the solvent; and adding the catalyst to the combination of the substituted phthalic anhydride, the organic diamine, the solvent, and the monofunctional reactant or the monofunctional phthalimide; the method comprises determining the stoichiometric ratio of the substituted phthalic anhydride to the organic diamine during the imidization of the substituted phthalic anhydride and the organic diamine; and optionally adjusting the stoichiometric ratio by adding additional substituted phthalic anhydride or organic diamine; the catalyst, for example a guanidinium salt, specifically hexaethylguanidinium chloride, is present in an amount of more than 0 to 5 mole %, specifically 0.1 to 1 mole%, based on the moles of the organic diamine; if the catalyst reacts in the presence of the solvent at about 5 hours after the substituted phthalic anhydride and the diamine being reacting, the bis(phthalimide) composition comprises less than 0.15 mole percent of residual substituted phthalic anhydride and less than 1.0 mole percent monoamine of the formula 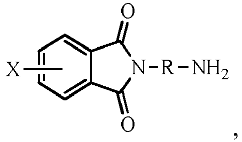 based on the total moles of the substituted phthalic anhydride, the organic diamine, and any monofunctional reactant and monofunctional phthalimide if present, wherein X and R are defined herein; or, the bis(phthalimide) composition comprises less than 100 ppm by weight or no detectable amount of a sodium aryl phosphinate salt, based on the weight of the bis(phthalimide) composition.

In an embodiment, a bis(phthalimide) composition comprises a bis(phthalimide) having a formula 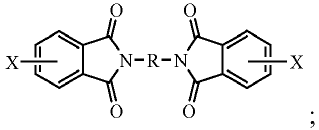 and a residue of a catalyst selected from quaternary ammonium salts, quaternary phosphonium salts, guanidinium salts, pyridinium salts, imidazolium salts, and combinations thereof, wherein the bis(phthalimide) is an imidization product of a substituted phthalic anhydride and an organic diamine that are catalyzed with the catalyst; the substituted phthalic anhydride having a formula 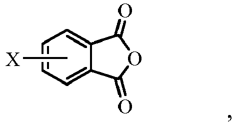 the organic diamine having a formula H₂N-R-NH₂, wherein, in the foregoing formulae, X is selected from fluoro, chloro, bromo, iodo, nitro, and combinations thereof, specifically, X is chloro, fluoro, bromo, or nitro, more specifically, X is chloro, and R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 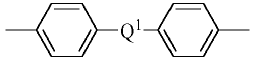 wherein Q¹ is a member selected from the group of a single bond, -O-, -S-, -C(O)-, -SO₂-, - SO-, -C_{y}H_{2y} wherein y is an integer from 1 to 5, and combinations thereof, specifically, R is a divalent radical of the formula -(C₆H₁₀)_{z}- wherein z is an integer from 1 to 4, 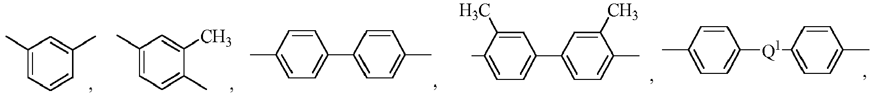 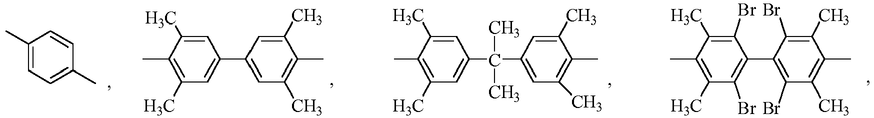 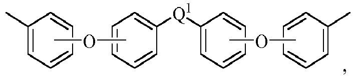 and combinations thereof, wherein Q¹ is selected from a single bond, -O-, -S-, -C(O)-, -SO₂-, -SO-, -C_{y}H_{2y}- wherein y is an integer from 1 to 5, and combinations thereof, more specifically, R is m-phenylene, p-phenylene, diarylsulfone, a group of the formula 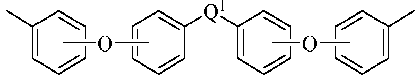 wherein Q¹ is a member selected from the group of a single bond, -O-, -S-, -C(O)-, -SO₂-, - SO-, -C_{y}H_{2y} wherein y is an integer from 1 to 5, and combinations thereof.

In the foregoing bis(phthalimide) composition, at least one of the following conditions apply: a polyetherimide formed by polymerization of the bis(phthalimide) and a dialkali metal salt of a dihydroxy aromatic compound such as bisphenol A, hydroquinone, bisphenol, resorcinol, and combinations thereof: (i) has an R* of less than or equal to 2.4, wherein R* is the ratio of viscosities measured under nitrogen at 1 rad/sec and 100 rad/sec at the temperature that gives a viscosity of 20,000 poise at 100 rad/sec; and/or (ii) has a yellowness index that is less than 120, measured on a 0.5 gram solution of the polyetherimide in 10 mL of methylene chloride, and in accordance with ASTM E313; a polyetherimide formed by polymerization of the bis(phthalimide) and the disodium salt of bisphenol A retains at least 80% of its weight average molecular weight, as measured by gel permeation chromatography after exposure to a temperature of 134°C for 4 days under steam and under pressure; the catalyst is a guanidinium salt such as hexaethylguanidinium chloride; the catalyst is a guanidinium salt, and a residue of the guanidinium salt is present in the composition in an amount ranging from 0.05 to 1 mole% based on the moles of the organic diamine; if the catalyst reacts in the presence of the solvent at about 5 hours after the substituted phthalic anhydride and the diamine being reacting, the composition comprises less than 0.15 mole percent of the substituted phthalic anhydride and less than 1.0 mole percent monoamine of formula 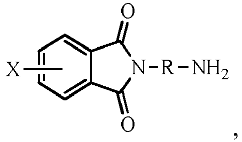 based on the total moles of the substituted phthalic anhydride, the organic diamine, and any monofunctional reactant and monofunctional phthalimide if present, wherein X and R are defined herein; or, the composition comprises less than 100 ppm by weight or no detectable amount of a sodium aryl phosphinate salt, based on the weight of the bis(phthalimide) composition.

In another embodiment, a method for the manufacture of a polyetherimide composition, comprises catalyzing imidization of a substituted phthalic anhydride and an organic diamine with a catalyst in the presence of a solvent, the catalyst being selected from quaternary ammonium salts, quaternary phosphonium salts, guanidinium salts, pyridinium salts, imidazolium salts, and combinations thereof, wherein the substituted phthalic anhydride has a formula 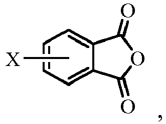 and
the organic diamine has a formula H₂N-R-NH₂ to provide a bis(phthalimide) composition comprising a residue of the catalyst and a bis(phthalimide) of the formula 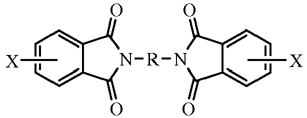 wherein conversion to the bis(phthalimide) is 99% complete, based on the moles of the substituted phthalic anhydride, in less than 6 hours, and catalyzing polymerization of the bis(phthalimide) and an alkali metal salt of a dihydroxy aromatic compound of the formula

MO-Z-OM

in the presence of the catalyst that catalyzes imidization of the substituted phthalic anhydride and the diamine to form the polyetherimide composition comprising a residue of the catalyst and a polyetherimide of the formula 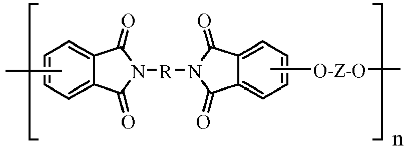 wherein in the foregoing formulae X is selected from fluoro, chloro, bromo, iodo, nitro, and combinations thereof, specifically, X is chloro, fluoro, bromo, or nitro, more specifically, X is chloro; R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 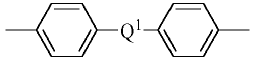 wherein Q¹ is selected from -O-, -S-, -C(O)-, -SO₂-, -SO-, -C_{y}H_{2y}- wherein y is an integer from 1 to 5, and combinations thereof, specifically, R is a divalent radical of the formula - (C₆H₁₀)_{z}- wherein z is an integer from 1 to 4, 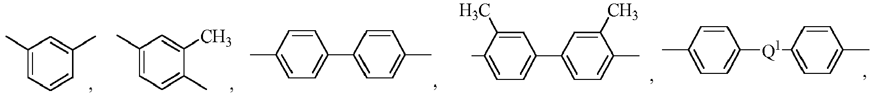 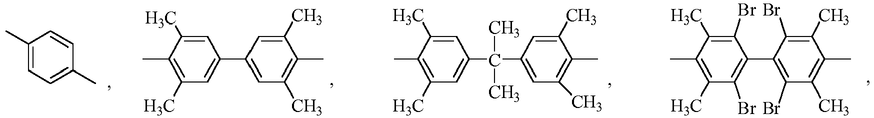 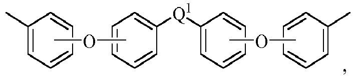 and combinations thereof, wherein Q¹ is selected from a single bond, -O-, -S-, -C(O)-, -SO₂-, -SO-, -C_{y}H_{2y}- wherein y is an integer from 1 to 5, and combinations thereof, more specifically, R is m-phenylene, p-phenylene, diarylsulfone, or a group of the formula 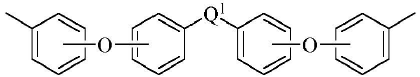 wherein Q¹ is -is a member selected from the group of a single bond, -O-, -S-, -C(O)-, -SO₂-, -SO-, -C_{y}H_{2y} wherein y is an integer from 1 to 5, and combinations thereof; M is an alkali metal; Z is an aromatic C₆₋₂₄ monocyclic or polycyclic moiety optionally substituted with 1 to 6 C₁₋₈ alkyl groups, 1 to 8 halogen atoms, and combinations thereof, specifically, Z is a divalent group of formula 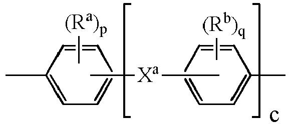 wherein R^{a} and R^{b} are each a halogen atom or a monovalent hydrocarbon group and can be the same or different; X^{a} is a single bond, -O-, -S-, -S(O)-, -S(O)₂-, -C(O)-, or a C₁₋₁₈ organic group; p and q are each independently integers of 0 to 4; and c is 0 or 1, more specifically, Z is 2,2-(4-phenylene)isopropylidene; and n is an integer greater than 1.

In specific embodiments of the foregoing method for the manufacture of a polyetherimide composition, one or more of the following conditions can apply: a polyetherimide formed by polymerization of the bis(phthalimide) and the disodium salt of bisphenol A retains at least 80% of its weight average molecular weight, as measured by gel permeation chromatography after exposure to a temperature of 134°C for 4 days under steam and under pressure; the method further comprises determining the stoichiometric ratio of the substituted phthalic anhydride to the organic diamine during the imidization reaction; and optionally adjusting the stoichiometric ratio by adding additional substituted phthalic anhydride or organic diamine; the method comprises combining the substituted phthalic anhydride, the organic diamine, the solvent, and the catalyst before heating the combination; the method further comprises combining the substituted phthalic anhydride, the organic diamine, and the solvent, heating the combined substituted phthalic anhydride, the organic diamine, and the solvent, and then adding the catalyst to the heated combination; the method further comprises adding a monofunctional reactant to a combination of the substituted phthalic anhydride and the organic diamine before adding the catalyst, or adding a monofunctional phthalimide to a combination of the substituted phthalic anhydride and the organic diamine before adding the catalyst; the method comprises combining the substituted phthalic anhydride and the organic diamine in the solvent; adding a monofunctional reactant or a monofunctional phthalimide to the combination of the substituted phthalic anhydride, the organic diamine, and the solvent; and adding the catalyst to the combination of the substituted phthalic anhydride, the organic diamine, the solvent, and the monofunctional reactant or the monofunctional phthalimide; the stoichiometric ratio of the substituted phthalic anhydride to the organic diamine is 1.98:1 to 2.04:1, and the stoichiometric ratio of the bis(phthalimide) to the alkali metal salt of the dihydroxy aromatic compound is 0.9:1 to 1.1:1; the catalyst is present in an amount ranging from 0.05 to 1 mole% based on the moles of the organic diamine during the imidization, and a second catalyst is added before the polymerization, wherein the second catalyst is selected from quaternary ammonium salts, quaternary phosphonium salts, guanidinium salts, pyridinium salts, imidazolium salts, and combinations thereof, and wherein the second catalyst is the same or different from the catalyst for the imidization; the catalyst is a guanidinium salt such as hexaethylguanidinium chloride; the bis(phthalimide) composition and the polyetherimide composition are manufactured in the same vessel; if the catalyst reacts in the presence of the solvent at about 5 hours after the substituted phthalic anhydride and the diamine being reacting, the bis(phthalimide) composition comprises less than 0.15 mole percent of the substituted phthalic anhydride and less than 1.0 mole percent monoamine of formula 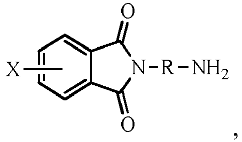 based on the total moles of the substituted phthalic anhydride, the organic diamine, and any monofunctional reactant and monofunctional phthalimide if present, wherein X and R are as defined herein; the catalyst are a guanidinium salt, and the polyetherimide composition comprises less than 1000 ppm of the residue of the catalyst, based on the weight of the polyetherimide; or, the polyetherimide comprises less than 100 ppm by weight or no detectable amount of a sodium aryl phosphinate salt, based on the weight of the polyetherimide.

In another embodiment, a polyetherimide composition comprises (i) a polyetherimide of the formula 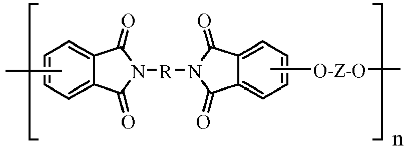 and (ii) a residue of a catalyst selected from quaternary ammonium salts, quaternary phosphonium salts, guanidinium salts, pyridinium salts, imidazolium salts, and combinations thereof; the polyetherimide being a catalyzed polymerization reaction product of (1) a bis(phthalimide) composition comprising a catalyzed imidization product of a substituted phthalic anhydride and an organic diamine and (2) an alkali metal salt of a dihydroxy aromatic compound, the imidization product being catalyzed by the catalyst; wherein the substituted phthalic anhydride has a formula 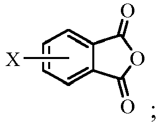 the organic diamine has a formula H₂N-R-NH₂; the bis(phthalimide) has a formula 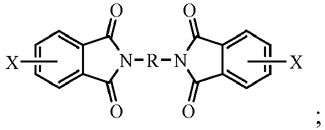 and the alkali metal salt of the dihydroxy aromatic compound has a formula

MO-Z-OM;

wherein in the foregoing formulae, X is selected from fluoro, chloro, bromo, iodo, nitro, and combinations thereof, specifically, X is chloro, fluoro, bromo, or nitro, more specifically, X is chloro; R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 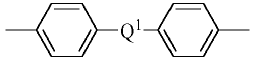 wherein Q¹ is selected from -O-, -S-, -C(O)-, -SO₂-, -SO-, -C_{y}H_{2y}- wherein y is an integer from 1 to 5, and combinations thereof, specifically, R is a divalent radical of the formula - (C₆H₁₀)_{z}- wherein z is an integer from 1 to 4, 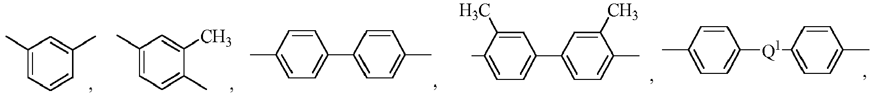 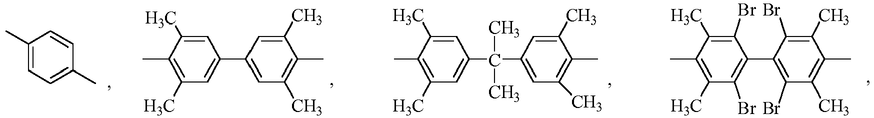 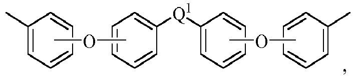 and combinations thereof, wherein Q¹ is selected from a single bond, -O-, -S-, -C(O)-, -SO₂-, -SO-, -C_{y}H_{2y}- wherein y is an integer from 1 to 5, and combinations thereof, more specifically, R is m-phenylene, p-phenylene, diarylsulfone, a group of the formula 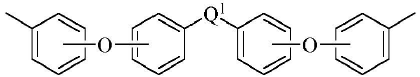 wherein Q¹ is a member selected from the group of a single bond, -O-, -S-, -C(O)-, -SO₂-, - SO-, -C_{y}H_{2y} wherein y is an integer from 1 to 5, and combinations thereof; M is an alkali metal; Z is an aromatic C₆₋₂₄ monocyclic or polycyclic moiety optionally substituted with 1 to 6 C₁₋₈ alkyl groups, 1 to 8 halogen atoms, and combinations thereof, specifically, Z is a divalent group of the formula 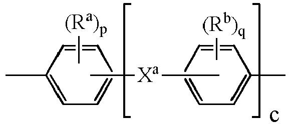 wherein R^{a} and R^{b} are each a halogen atom or a monovalent hydrocarbon group and can be the same or different, X^{a} is a single bond, -O-, -S-, -S(O)-, -S(O)₂-, -C(O)-, or a C₁₋₁₈ organic group, p and q are each independently integers of 0 to 4, and c is 0 or 1, more specifically, Z is 2,2-(4-phenylene)isopropylidene; and n is an integer greater than 1,

In specific embodiments of the foregoing polyetherimide composition, one or more of the following conditions can apply: the polymerization is catalyzed by the same catalyst that catalyzes the imidization; the catalyst is a guanidinium salt such as hexaethylguanidinium chloride; the catalyst is a phosphonium salt; the catalyst is an imidazolium salt; the polyetherimide has an R* value that is less than or equal to 2.4, wherein R* is the ratio of viscosities measured under nitrogen at 1 rad/sec and 100 rad/sec at the temperature that gives a viscosity of 20,000 poise at 100 rad/sec, and a yellowness index that is less than 120, measured on a 0.5 g solution of the polyetherimide in 10 mL of methylene chloride, and in accordance with ASTM E313; the dihydroxy aromatic compound is bisphenol A, and the polyetherimide retains at least 80% of its weight average molecular weight, as measured by gel permeation chromatography after exposure to a temperature of 134°C for 4 days under steam and under pressure; if the catalyst reacts at about 5 hours after the substituted phthalic anhydride and the diamine being reacting, the bis(phthalimide) composition comprises less than 0.15 mole percent of the substituted phthalic anhydride and less than 1.0 mole percent monoamine of formula 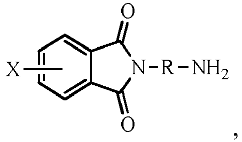 based on the total moles of the substituted phthalic anhydride, the organic diamine, and any monofunctional reactant and monofunctional phthalimide if present, wherein X and R are as defined herein; the catalyst is a guanidinium salt, and a residue of the guanidinium salt is present in an amount less than 2000 ppm or less than 1000 ppm, based on parts of the polyetherimide composition; the composition comprises less than 100 ppm by weight or no detectable amount of a sodium aryl phosphinate salt, based on the weight of the polyetherimide composition; the composition further comprises a filler; a filler is substantially absent; a filler is absent; the composition further comprises an additive selected from catalysts, impact modifiers, fillers, reinforcing agents, anti-oxidants, thermal stabilizers, light stabilizers, ultraviolet light (UV) absorbers, quenchers, plasticizers, lubricants, mold release agents, antistatic agents, colorants, blowing agents, flame retardants, anti-drip agents, radiation stabilizers, and combinations thereof; the composition further comprises an additive selected from an antioxidant, a UV absorber, a mold release agent, and combinations thereof; or, the composition further comprises a solvent, and the composition is in the form of a vanish.

An article comprising the polyetherimide composition of any one of the above embodiments is also disclosed. The article is selected from a sheet, film, multilayer sheet, multilayer film, molded part, extruded profile, coated part, pellets, powder, foam, fiber, fibrids, flaked fibers, and combinations thereof; or the article is a composite comprising the polyetherimide composition according to the foregoing embodiments.

A method of forming an article comprises shaping, extruding, blow molding, or injection molding the polyetherimide composition disclosed herein to form the article.

Without further elaboration, it is believed that one skilled in the art can, using the description herein, utilize the present invention. The following examples are included to provide additional guidance to those skilled in the art of practicing the claims. Accordingly, these examples are not intended to limit the invention in any manner.

### EXAMPLES

### Materials

The materials in Table 1 were used or made in the following Examples and Comparative Examples.

**Table 1**

| Acronym | Description | Source |
|---|---|---|
| PA | Phthalic anhydride | |
| 3-ClPA | 3-Chlorophthalic anhydride | SABIC |
| 4-ClPA | 4-Chlorophthalic anhydride | SABIC |
| ClPA | Mixture of 3-chlorophthalic anhydride and 4-chlorophthalic anhydride | SABIC |
| C1PAMI | 1,3 -bis[N-(4-chlorophthalimido)]benzene | Examples |
| Mono-ClPAMI (MA) | Mixture of 1-amino-3-N-(4-chlorophthalimido)benzene, 1-amino-3-N-(3-chlorophthalimido)benzene | Examples |
| mPD | meta-Phenylene diamine | DuPont |
| DDA | 4,4'-diaminodiphenyl sulfone | Atul |
| BPA | 2,2-Bis(4-hydroxyphenyl)propane, (Bisphenol A) | Hexion |
| BPANa₂ | Bisphenol, disodium salt | SABIC |
| BPADA | Bisphenol A dianhydride | SABIC |
| PCP | para-Cumyl phenol | SABIC |
| PEI | Polyetherimide | Examples |
| o-DCB | ortho-Dichlorobenzene | Fischer |
| HEGCl | Hexaethylguanidinium chloride | Atul Ltd. |
| SPP | Sodium phenylphosphinate | Akzo |
| TPPBr | Tetraphenylphosphonium bromide | Sigma-Aldrich |
| C6B | 1,6-Bis(tributylammonium)-hexane dibromide | Sigma-Aldrich |
| PyrEHCl | 4-(N,N-dimethyl)-2-ethylhexylpyridinium chloride | Sigma-Aldrich |
| TBAB | Tetrabutylammonium bromide | Sigma-Aldrich |
| 18-C-6 | 1,4,7,10,13,16-Hexaoxacyclooctadecane | Sigma-Aldrich |
| TTPCl | Trihexyltetradecylphosphonium chloride | Sigma-Aldrich |
| BDMICl | 1-butyl-2,3-dimethylimidazolium chloride | Sigma-Aldrich |
| H₃PO₄ | Phosphoric acid | Fischer |
| IRGAPHOS 168 | Tris(2,4-di-tert-butylphenyl)phosphite | CIBA Specialty Chemicals |

### PROPERTY TESTING

Weight average molecular weight (Mw) of the polymer product was determined by gel permeation chromatography (GPC) using polystyrene standards.

The weight average molecular weight (Mw), polydispersity indices PDI and PDI* were determined by gel permeation chromatography (GPC) using a Mixed Bed C, PLgel 5 µm, 300 x 7.5 mm, P/N 1110-6500 column, methylene chloride as eluent at 1 mL/min, UV detection at 254 nm, and a GPC calculation software program. PDI is Mw/Mn, wherein Mn is the number average molecular weight. PDI* is Mz/Mw, wherein Mz is the z-average molecular weight.

Residual ClPAMI, mono-ClPAMI, and PAMI contents were determined by high pressure liquid chromatography (HPLC). The detection limit was 25 ppm.

Residual chlorine was measured by total ion chromatography combustion and/or hexylamine digestion.

Polymer end groups were identified and quantitated by derivatization with a phosphorylation reagent, followed by phosphorous-31 nuclear magnetic resonance spectroscopy (³¹P-NMR).

Water (moisture) content of reaction mixtures was determined by Karl-Fisher titration.

The shear thinning behavior of the polyetherimides was assessed by measurement of R* by parallel plate rheometry using a Rheometric Scientific ARES instrument equipped with 25 mm parallel plates. R* is a measure of shear thinning behavior of the polymer. It is calculated as the ratio of viscosities measured at 1 rad/sec and 100 rad/sec at a fixed temperature (the R* temperature) under nitrogen. The R* temperature is defined as the temperature that gives a viscosity of 20,000 poise at 100 rad/sec, under nitrogen. This temperature is typically between 310 and 345 °C, depending on the molecular weight of the material. The method is based upon, but is not in full compliance with, ASTM D4440-01. All samples are vacuum dried at 150 °C for at least 4 hours before measurement.

Yellowness Index (YI) was measured according to ASTM E313. ASTM D-1925 is also an acceptable method. Generally, the YI is a number calculated from spectrophotometric data that describes the color of a test sample as being clear or white (low YI) versus being more yellow (high YI). Sample handling and preparation can affect the test results. The yellowness index of the polyetherimide polymer pellets was determined by dissolving 0.5 grams of polyetherimide pellets in 10 mL of methylene chloride, and measuring the YI of the resulting solution on an Xrite 7000 Color Eye device (Xrite, Incorporated) in accordance with ASTM E313.

Melt flow rate was measured at 295°C and a load of 6.7 kg according to ASTM D1238-10.

Initial tensile strength at yield was measured according to ASTM D638-10.

Where indicated, "dry o-DCB" having moisture content of less than 10 ppm was used in the reactions. The dry o-DCB was kept in a glove box over 4-Angstrom molecular sieves.

### BPANa₂

BPANa₂ was obtained by mixing sodium hydroxide with bisphenol A in a molar ratio of 2:1 at 70°C in demineralized water under nitrogen. A solution was formed first. Then the solution was added slowly to boiling o-DCB in a reactor equipped with a Dean Stark condenser until all the salt was suspended in o-DCB. Water was eliminated to a moisture content of less than 20 ppm. The o-DCB was then removed using a rotary evaporator followed by Kugelrohr vacuum distillation at 250°C under nitrogen to yield a white solid being BPANa₂. The white solid obtained was kept in a nitrogen glove box to prevent rehydration and oxidation.

### COMPARATIVE EXAMPLES 1, 4 AND 10, AND EXAMPLES 2, 3, 5-9 AND 11-13

Phase transfer catalysts were screened to determine their efficacy as imidization catalysts. The specific catalysts screened were hexaethylguanidinium chloride (HEGCl), sodium phenylphosphinate (SPP), tetraphenylphosphonium bromide (TPPBr). 1,6-bis(tributylammonium)-hexane dibromide (C6B), 4-(N,N-dimethyl)-2-ethylhexylpyridinium chloride (PyrEHCl), tetrabutylammonium bromide (TBAB), 1,4,7,10,13,16-hexaoxacyclooctadecane (18-C-6), trihexyltetradecylphosphonium chloride (TTPCl), and 1-butyl-2,3-dimethylimidazolium chloride (BDMICl). To this end, 4-ClPA was reacted with mPD in the presence of phthalic anhydride in refluxing ortho-dichlorobenzene, to afford ClPAMI, a monomer used in the production of polyetherimides. The synthesis of ClPAMI can use various ratios of 3- and 4-ClPA, with 1 to 3 mol % phthalic anhydride (with respect to the total moles of substituted phthalic anhydride and phthalic anhydride employed). The phthalic anhydride becomes the eventual chain terminator in the polyetherimide resulting from the reaction of ClPAMI with disodium salt of bisphenol in o-DCB in the presence of an imidization catalyst.

### GENERAL PROCEDURE TO EVALUATE SCREEN CATALYST EFFECTIVENESS AS AN IMIDIZATION CATALYST

The procedure to screen the catalysts used standard laboratory glassware. A 3-necked, 1-liter, round-bottomed flask with 24/40 joints, was fitted with a center mounted mechanical stirrer with a Teflon paddle stir blade (the bottom of the stir blade was 1 cm from the bottom of the reaction vessel, a Dean and Stark Receiver placed in one of the side necks topped with a reflux condenser, and a solid addition funnel (LabGlass catalog #LG-8281t-104) topped with a nitrogen inlet. The outlet of the condenser was plumbed to a bubbler filled with a small amount of silicone oil. The inlet nitrogen supply was equipped with a flow meter that read 0.0 to 5.0 standard cubic feet per hour (scfh). The flask was charged with 69.30 grams (379.6 mmol) of 4-chlorophthalic anhydride (4-ClPA) containing 4 wt% of 3-chlorophthalic anhydride (3-ClPA) and 434 grams of reagent grade ortho-dichlorobenzene. The mixture was heated with an external oil bath under a nitrogen blanket with mechanical stirring (150 rpm) to afford a clear solution. The oil temperature was set at 175°C. The oil level in the oil bath was placed at the same level as the material inside the flask. The solid addition funnel was charged with 20.60 grams (190.5 mmol) of DuPont solid flake metaphenylene diamine (mPD) under nitrogen, with a nitrogen flow rate of 1 standard cubic foot per hour. The mPD was added to the o-DCB/ClPA solution over a 45-minute period. Generally, about 5 mL of o-DCB and about 5 mL of water collected in the arm of the receiver over the course of the mPD addition, and was discarded. The product (ClPAMI) precipitated from the solution as the reaction progressed, to afford a thick yellow slurry. During the reaction, the stirring rate was gradually increased to 350 rpm as the reaction mixture thickened. The stirring rate was periodically adjusted to minimize splattering of the material on the upper climbs of the vessel. The solid addition funnel was heated with a heat gun to melt any residual mPD left in the funnel and was then rinsed with 20 grams of hot reagent grade o-DCB to complete the transfer of mPD to the vessel. The addition funnel was removed from the flask and the nitrogen supply was adapted in its place.

The oil temperature was then increased to 185°C and the nitrogen flow rate was increased to 1.5 scfh. o-DCB was allowed to distill from the vessel and was collected in the arm of the receiver. About 47 grams of o-DCB was allowed to distill from the vessel. The condensed o-DCB was removed from the arm of the receiver and not allowed to return to the vessel. Once 47 grams of o-DCB had been collected, the vessel was placed under a blanket of nitrogen rather than a sweep of nitrogen (i.e., the nitrogen was adjusted to a minimum flow).

Phthalic anhydride (0.566 gr, 3.82 mmol) in 3 mL of o-DCB was added to the flask 1.25 hours after the end of the mPD addition to the flask (2 hours total reaction time). The nitrogen flow was increased to 1.5 scfh with an oil temperature of 185°C. Once 3 mL of o-DCB had been collected in the arm of the receiver, the nitrogen flow was then decreased to maintain a nitrogen blanket on the vessel. At this point, the amount of o-DCB in the vessel was about 407 grams and the weight of the ClPAMI product present was about 83.3 grams (17.0% solids). The reaction was heated with 185°C oil for one hour (3 hours of total reaction time), and then a sample of the reaction was taken and analyzed for residual 4-ClPA and the residual chloromonoamine (structure 20a) by HPLC (high pressure liquid chromatography. An imidization catalyst (1 mole%, with respect to the amount of mPD employed in the reaction) was added to the vessel just after the reaction mixture was sampled (i.e., after 3 hours of reaction time). In the instance of HEGCL, the catalyst was delivered as a dry solution of HEGCl dissolved in dry o-DCB at 17 to 20 wt% solids. The moisture of the solution was generally 10 ppm water. The reaction mixture was then heated for 1 hour with 185°C oil, and a sample of the reaction was taken (after 4 hours of reaction time) and analyzed for residual ClPA and the residual chloromonoamine (structure 20a) by HPLC (high pressure liquid chouromatography). The reaction was continued and the reaction mixture was then sampled for ClPAMI, residual ClPA and 4-chloromonoimide over the desired amount of time. The results are shown in Tables 2 and 3. The overall stoichiometry of each CIPAMI reaction as a function of time is shown in Table 4.

**Table 2. Amount of Residual ClPA (4-ClPA plus 3-ClPA) in ClPAMI (mol% based on total monomer content) for Screened Catalysts as a Function of Time.**

| | C.Ex. 1 | Ex. 2 | Ex. 3 | C.Ex. 4 | Ex. 5 | Ex. 6 | Ex. 7 | Ex. 8 | Ex. 9 | C.Ex. 10 | Ex. 11 | Ex. 12 |
|---|---|---|---|---|---|---|---|---|---|---|---|---|
| Time* | No catalyst | 1 mol% HEGC1 | 0.2 mol% HEGC1 | 0.25 mol% SPP | 1 mol% TPPBr | 1 mol% C6B | 1 mol% PyrEHCl | 1 mol% TBAB | 0.2 mol% PyrEHCl | 1 mol% 18-C-6 | 1 mol% TTPC1 | 1 mol% BDMICl |
| 0 | NM | NM | NM | NM | NM | NM | NM | NM | NM | NM | NM | NM |
| 1 | NM | NM | NM | NM | NM | NM | NM | NM | NM | NM | NM | NM |
| 2 | NM | NM | NM | NM | NM | NM | NM | NM | NM | NM | NM | NM |
| 3 | 1.52 | ** 1.5 | ** NM | ** NM | ** 1.45 | ** 1.55 | ** 1.83 | ** 1.79 | ** 1.58 | ** 1.62 | ** 1.83 | ** 1.94 |
| 4 | 1.13 | 0.13 | 1.75 | 1.85 | 0.49 | 0.28 | 0.21 | 0.46 | 0.59 | 1.34 | 0.98 | 0.61 |
| 5 | 0.88 | 0.07 | 0.53 | 0.84 | 0.31 | 0.19 | 0.12 | 0.33 | 0.35 | 1.17 | 0.56 | 0.44 |
| 6 | 0.82 | <=.07 | 0.31 | 0.55 | 0.22 | NM | 0.09 | 0.26 | 0.19 | 1.03 | 0.46 | 0.40 |
| 7 | 0.77 | <=.07 | 0.2 | 0.38 | NM | NM | NM | NM | NM | 0.93 | NM | NM |

| | | | | | | | | | | | | |
|---|---|---|---|---|---|---|---|---|---|---|---|---|
| * Time given is the time the substituted phthalic anhydride and the diamine have reacted. For example, if the imidization catalyst is added when the substituted phthalic anhydride and the diamine initially react, the time is 0. If the imidization catalyst is added three hours after the substituted phthalic anhydride and the diamine have reacted, the time is 3 hours. ** Indicates time when the catalyst is added after the substituted phthalic anhydride and the diamine have reacted. NM - not measured. | | | | | | | | | | | | |

**Table 3. Amount of Residual chloromonoamine (4-chloromonoamine plus 3-chloromonoamine) (20a) in ClPAMI (mol% based on total monomer content) for Screened Catalysts as a Function of Time.**

| | C.Ex. 1 | Ex. 2 | Ex. 3 | C.Ex. 4 | Ex. 5 | Ex.6 | Ex. 7 | Ex. 8 | Ex. 9 | C.Ex. 10 | Ex. 11 | Ex. 12 |
|---|---|---|---|---|---|---|---|---|---|---|---|---|
| Time* | No catalyst | 1 mol% HEGC1 | 0.2 mol% HEGC1 | 0.25 mol% SPP | 1 mol% TPPBr | 1 mol% C6B | 1 mol% PyrEHCl | 1 mol% TBAB | 0.2 mol% PyrEHCl | 1 mol% 18-C-6 | 1 mol% TTPC1 | 1 mol% BDMICl |
| 0 | NM | NM | NM | NM | NM | NM | NM | NM | NM | NM | NM | NM |
| 1 | NM | NM | NM | NM | NM | NM | NM | NM | NM | NM | NM | NM |
| 2 | NM | NM | NM | NM | NM | NM | NM | NM | NM | NM | NM | NM |
| 3 | 1.605 | ** 1.643 | ** NM | ** NM | ** 1.602 | ** 1.661 | ** 1.857 | ** 1.835 | ** 1.725 | ** 1.782 | ** 1.28% | ** 1.53% |
| 4 | 1.32 | 0.25 | 1.675 | 1.837 | 0.697 | 0.447 | 0.278 | 0.572 | 0.763 | 1.644 | 0.46% | 0.36% |
| 5 | 1.103 | 0.131 | 0.59 | 0.943 | 0.478 | 0.253 | 0.145 | 0.326 | 0.494 | 1.361 | 0.16% | 0.18% |
| 6 | 0.98 | NM | 0.339 | 0.581 | 0.369 | NM | 0.08 | 0.334 | 0.271 | 1.271 | 0.08% | 0.12% |
| 7 | 0.908 | NM | 0.195 | 0.382 | NM | NM | NM | NM | NM | 1.285 | NM | NM |

| | | | | | | | | | | | | |
|---|---|---|---|---|---|---|---|---|---|---|---|---|
| * Time given is the time the substituted phthalic anhydride and the diamine have reacted. For example, if the imidization catalyst is added when the substituted phthalic anhydride and the diamine initially react, the time is 0. If the imidization catalyst is added three hours after the substituted phthalic anhydride and the diamine have reacted, the time is 3 hours. ** Indicates time when the catalyst is added after the substituted phthalic anhydride and the diamine have reacted. NM - not measured. | | | | | | | | | | | | |

**Table 4 Stoichiometry of ClPAMI (mol%) for Screened Catalysts as a Function of Time.**

| | C.Ex. 1 | Ex. 2 | Ex. 3 | C.Ex. 4 | Ex. 5 | Ex. 6 | Ex. 7 | Ex. 8 | Ex. 9 | C.Ex. 10 | Ex. 11 | Ex. 12 |
|---|---|---|---|---|---|---|---|---|---|---|---|---|
| Time * | No catalyst | 1 mol% HEGCl | 0.2 mol% HEGC1 | 0.25 mol% SPP | 1 mol% TPPBr | 1 mol% C6B | 1 mol% PyrEHCl | 1 mol% TBAB | 0.2 mol% PyrEHCl | 1 mol% 18-C-6 | 1 mol% TTPC1 | 1 mol% BDMICl |
| 0 | NM | NM | NM | NM | NM | NM | NM | NM | NM | NM | NM | NM |
| 1 | NM | NM | NM | NM | NM | NM | NM | NM | NM | NM | NM | NM |
| 2 | NM | NM | NM | NM | NM | NM | NM | NM | NM | NM | NM | NM |
| 3 | -0.09 | ** -0.14 | ** NM | ** NM | ** -0.15 | ** -0.11 | ** -0.03 | ** -0.04 | ** -0.15 | ** -0.16 | ** 0.56 | ** 0.41 |
| 4 | -0.19 | -0.12 | 0.08 | 0.01 | -0.21 | -0.17 | -0.07 | -0.11 | -0.17 | -0.30 | 0.53 | 0.25 |
| 5 | -0.22 | -0.06 | -0.06 | -0.10 | -0.17 | -0.06 | -0.03 | 0.00 | -0.14 | -0.19 | 0.39 | 0.26 |
| 6 | -0.16 | NM | NM | -0.03 | -0.15 | NM | 0.01 | -0.07 | -0.08 | -0.24 | 0.38 | 0.28 |
| 7 | -0.14 | NM | NM | 0.00 | NM | NM | NM | NM | NM | -0.36 | NM | NM |

| | | | | | | | | | | | | |
|---|---|---|---|---|---|---|---|---|---|---|---|---|
| * Time given is the time the substituted phthalic anhydride and the diamine have reacted. For example, if the imidization catalyst is added when the substituted phthalic anhydride and the diamine initially react, the time is 0. If the imidization catalyst is added three hours after the substituted phthalic anhydride and the diamine have reacted, the time is 3 hours. ** Indicates time when the catalyst is added after the substituted phthalic anhydride and the diamine have reacted. NM - not measured. | | | | | | | | | | | | |

As shown in the Tables 2-4, only 18-C-6 was ineffective as an imidization catalyst. Other than SPP and 18-C-6, the catalysts screened are capable of facilitating the reaction of ClPA with mPD to produce ClPAMI. Our results, as evidenced by Table 2, also show if the imidization catalyst reacts in the presence of the solvent at about 5 hours after the substituted phthalic anhydride and the diamine have reacted, the use of imidization catalyst selected from quaternary ammonium salts, quaternary phosphonium salts, guanidinium salts, pyridinium salts, imidazolium salts results in a reaction mixture having significantly lower substituted phthalic anhydride residuals as compared to situations where other catalysts (or no catalysts) are used.

The normal amount of SPP catalyst used to prepare ClPAMI is 0.25 mol%, when added with the monomers (ClPA, PA and mPD) at the beginning of the reaction. It was observed in a separate instance that when SPP was added at the beginning of the reaction between the dianhydride and the diamine (time =0), the amount of ClPA residuals at 3,4,5,6, and 7 hours after the dianhydride and the diamine reaction were 0.42, 0.34, 0.33. 0.8, 0.08, respectively. It can be seen from the 5-hour data that every catalyst evaluated was better or nearly as good as 0.25 mol% SPP added with the monomers (including PA) at the beginning of the reaction.

### COMPARATIVE EXAMPLES 14 AND 15

Another set of potential catalysts for imidization was evaluated. In this study, the molar ratio of amine groups to anhydride groups was 1 mol% excess amine. It is known that the rate of ClPAMI formation is accelerated when excess amine groups are present. (The opposite is also true, the rate of ClPAMI formation is accelerated when excess anhydride groups are present.) Running the screening study with 1 mol% excess amine groups was done to facilitate the speed of the study. For comparative purposes, the imidization reaction was run at 1 mol% excess amine groups compared to anhydride groups, in the presence and absence of sodium phenyl phosphinate (0.29 mol% with respect to the moles of mPD used), as described below, in comparative examples 14 and 15.

### Comparative Example 14

Synthesis of ClPAMI in the presence of SPP was carried out as follows. A 3-neck-round bottomed flask is charged with m-phenylene diamine (2.743 g, 25.365 mmol), 4-chlorophthalic anhydride (9.225 g, 50.531 mmol), sodium phenylphosphinate (SPP, 12 mg, 0.0731 mmol) and o-dichlorobenzene (65 g). The flask is assembled with a mechanical stirrer, a Dean-Stark trap and a nitrogen inlet then placed in pre-heated oil bath (170°C). The mixture is stirred and the oil temperature is increased to 180°C. Nitrogen flow is gradually increased to allow a steady collection of water/o-DCB mixture in the Dean-Stark trap. Nitrogen flow is decreased when about 10 mL of o-DCB has been collected in the trap. The mixture is allowed to stir until no further change in residual 4-ClPA and 4-MA (final residual content: 0.8 to 1.0 mol% 4-MA and less than 0.05 mol% 4-ClPA with respect to m-PD charged). This experiment was repeated with similar results.

### Comparative Example 15

Synthesis of ClPAMI in the absence of SPP was carried out as follows. A 3-neck-round bottomed flask is charged with m-phenylene diamine (2.743 g, 25.365 mmol), 4-chlorophthalic anhydride (9.225 g, 50.531 mmol) and o-dichlorobenzene (65 g). The flask is assembled with a mechanical stirrer, a Dean-Stark trap and a nitrogen inlet then placed in pre-heated oil bath (170°C). The mixture is stirred and the oil temperature is increased to 180°C. Nitrogen flow is gradually increased to allow a steady collection of water/o-DCB mixture in the Dean-Stark trap. Nitrogen flow is decreased when about 10 mL of o-DCB has been collected in the trap. The mixture is allowed to stir until no further change in residual 4-ClPA and 4-MA (final residual content: 0.8 to 1.0 mol% 4-MA and less than 0.05 mol% 4-ClPA with respect to m-PD charged). This experiment was repeated with similar results.

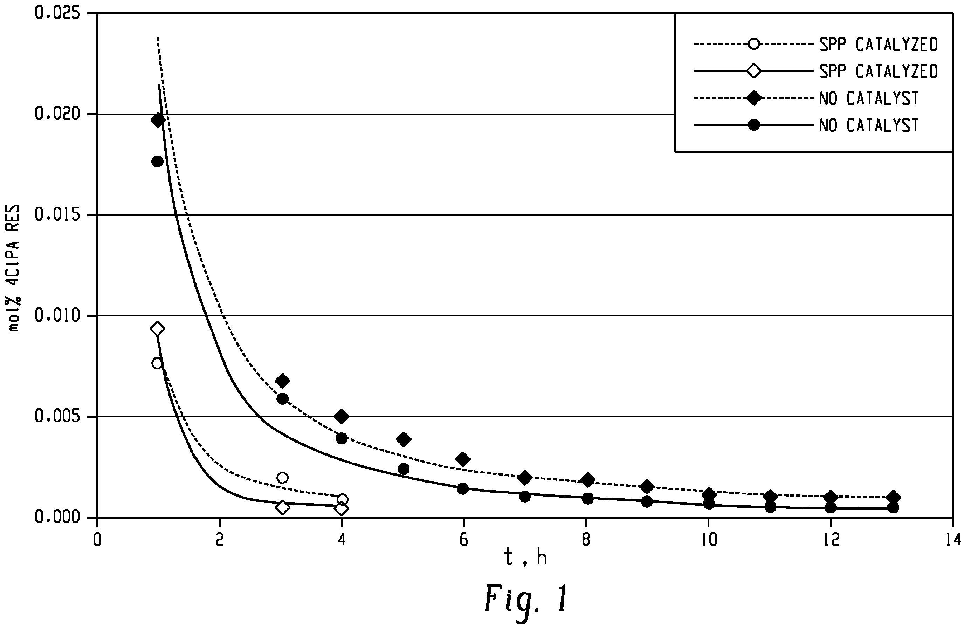

FIG. 1 shows the amount of residual ClPA in ClPAMI when made with 1 mol% excess amine groups, in the presence and absence of SPP imidization catalyst. As can be seen the presence of SPP results in a ClPAMI product with less than 0.15 mol% residual ClPA in the product, the desired specification for ClPAMI produced under these conditions. For a catalyst to be useful in a manufacturing process that requires a certain production rate, the amount of residual ClPA in the ClPAMI should be less than 0.15 mol% within 6 hours, when the ClPAMI is made under the reaction conditions for comparative Example 15.

### COMPARATIVE EXAMPLES 16-25 AND EXAMPLES 26-29

The compounds shown in Table 5 were evaluated for effectiveness as a catalyst for the imidization reaction and the polymerization reaction, and the results are shown in Table 5.

**Table 5**

| Ex. | Catalyst | Amount (mol%)^{a} | Time to Completion (hour) | Catalyst for Imidization? | Catalyst for Polymerization? |
|---|---|---|---|---|---|
| C.Ex.16 | None | none | 13 | No | No |
| C.Ex.17 | SPP | 0.12 | 5 | Yes | No |
| C.Ex.18 | Zinc acetate | 0.12 | >6 | No | No |
| C.Ex.19 | Aluminum acetylacetate | 0.12 | 6 | Yes | No |
| C.Ex.20 | Sodium tetraphenylborate | 0.12 | 4 | Yes | No |
| C.Ex. 21 | Cloisite sodium clay | 5.0wt%^{b} | 5 | Yes | No |
| C.Ex.22 | Sodium chloride | 0.12 | >6 | No | No |
| C.Ex.23 | Tolyl phthalizinone | 0.12 | >6 | No | No |
| CEx. 24 | Molecular sieves (14 A) | 5.0 wt%^{b} | 4 | yes | No |
| C.Ex.25 | Caffeine | 0.12 | >6 | no | No |
| Ex.26 | HEGCl | 1.00 | 3 | yes | Yes |
| Ex.27 | HEGCl | 0.20 | 4 | yes | Yes |
| Ex.28 | HEGCl | 0.10 | 6 | yes | Yes |
| C.Ex.29 | HEGCl | 0.05 | >6 | no | Yes |

| | | | | | |
|---|---|---|---|---|---|
| a. Based on moles of mPD, except where indicated. b. Based on PEI polymer weight. | | | | | |

SPP, aluminum acetylacetate, sodium tetraphenylborate, the clay, molecular sieves, and HEGC1 (0.1 to 1.0 mol%, or more, based on moles of mPD employed) were found to be useful imidization catalysts. Of the catalysts screened, only HEGC1 could also be used as the catalyst to effect the polymerization of BPA disodium salt and ClPAMI to produce polyetherimide.

### COMPARATIVE EXAMPLES 30 TO 39 AND EXAMPLES 40-46

These examples demonstrate that the use of HEGCl as the imidization catalyst ultimately provides a polyetherimide with a lower yellowness index, lower R* and lower Mz/Mw than when SPP is used as the catalyst.

### Comparative Examples 30 to 39 Imidization Process

Distilled o-DCB (19.74 parts) was charged to an appropriately sized oil-jacketed vessel, equipped with a mechanical stirrer, material addition lines, and charge pot, an overhead vapor line with condenser, and means to maintain a nitrogen atmosphere. The quantity of o-DCB used in a particular reaction was based on the desired percentage solids (% solids) of the imidization reaction. The o-DCB was heated to 120°C under nitrogen with stirring. Molten chlorophthalic anhydride (155.04 parts) was added to the vessel with stirring under nitrogen. Solid phthalic anhydride (0.0309 parts, 0.216 mole equivalents) was charged to the vessel through a charge pot, under nitrogen. Sodium phenylphosphinate (0.0038 parts, 0.024 mole equivalents) was then charged to the vessel through the charge pot. The pot was flushed with a small amount of distilled o-DCB. The temperature of the mixture was increased to about 160°C over a period of 45 minutes by applying hot oil on the jacket of the vessel, with stirring, under nitrogen.

Molten meta-phenylene diamine (1 part, 9.58 mole equivalents), stored under nitrogen at 100°C, was then added to the vessel over a 90 minute period, with agitation, under nitrogen. After completion of the mPD addition, the temperature of the reactor was increased to 180°C. During this period mPD reacted with ClPA to afford a CIPAMI/o-DCB slurry containing intermediate products of the reaction and water as by-product. Water vapor leaving the reactor along with o-DCB were condensed and collected in a collection vessel. The reaction was allowed to heat for 1.5 to 2.0 hours with agitation under nitrogen to complete the reaction. A sample was taken from the vessel to measure the stoichiometry of the reaction by HPLC analysis. The desired stoichiometry specification of the product was - 0.2 to 0.3 mole% of excess ClPA to monoamine (20a). If the reaction was not within specification, ClPA or mPD was added to the vessel at 180°C (hereafter this procedure is referred as stoich correction) to achieve the desired product specification. After 1 hour from the completion of stoic correction a sample was drawn again for measuring the stoichiometry. The procedure of sampling, analysis, and stoich (stoichiometry) correction was continued until the desired stoichiometry was achieved. Once the product was within specification, o-DCB was distilled from the vessel until the moisture in the o-DCB condensate was less than 20 ppm water. Generally, the ClPAMI was present in the o-DCB at about 20 wt% solids by the time the reaction was judged to be dry. If the reaction was not dry, then dry distilled o-DCB was added to the vessel and removed by distillation until the reaction was judged dry. The dry CIPAMI/o-DCB on-stoichiometry slurry at 18 to 21 wt% solids was ready for polymerization. Generally at the completion of a batch of ClPAMI manufacture, the amount of residual monoamine (20a) was less than 0.2 mol%, and the amount of residual ClPA was less than 0.4 mol%, with respect to the total moles of amine groups charged to the reactor in the form of mPD.

### Examples 40-46 Imidization process

ClPAMI was also made with the use of HEGCl as the imidization catalyst instead of SPP. Distilled o-DCB (19.74 parts) was charged to an appropriately sized oil jacketed vessel, equipped with a mechanical stirrer, material addition lines and charge pot, an overhead vapor line with condenser, and means to maintain a nitrogen atmosphere. The quantity of o-DCB used in a particular reaction was based on the desired percentage solids of the imidization reaction. The o-DCB was heated to 120°C under nitrogen with stirring. Molten chlorophthalic anhydride (155.04 parts) was added to the vessel with stirring under nitrogen. Solid phthalic anhydride (0.0309 parts, 0.216 mole equivalents) was charged to the vessel through a charge pot, under nitrogen. The pot was flushed with a small amount of distilled o-DCB. The temperature of the mixture was increased to about 160°C over a period of 45 minutes by applying hot oil on the jacket of the vessel, with stirring under nitrogen.

Molten metaphenylene diamine (1 part, 9.58 mole equivalents), stored under nitrogen at 100°C, was then added to the vessel over a 90 minute period, with agitation under nitrogen. After completion of the mPD addition, the temperature of the reactor was increased to 180°C. During this period mPD reacted with ClPA to afford a CIPAMI/o-DCB slurry containing intermediate products of this reaction and water as byproduct. Water vapors leaving the reactor along with o-DCB were condensed and collected in the collection vessel. The reaction was allowed to heat for 1.5 to 2.0 hours with agitation under nitrogen to complete the reaction. A sample was taken from the vessel to measure the stoichiometry of the reaction by HPLC analysis. The desired stoichiometry specification of the product was - 0.2 to 0.3 mole% of excess ClPA compared to the moles of monoamine (20a). If the reaction was not within specification, ClPA or mPD was added to the vessel at 180°C (hereafter this procedure is referred as stoichiometric correction) to achieve the desired product specification. After 1 hour from the completion of stoichiometric correction a sample was drawn again for measuring the stoichiometry. The procedure of sampling, analysis, and stoich correction was continued until the desired stoichiometry was achieved. Once the product was within specification, o-DCB was distilled from the vessel until the moisture in the o-DCB condensate was less than 100 ppm water. Generally at this point in the batch, the ClPAMI was present in the o-DCB at 18 to 20 wt% solids. HEGC1 (0.022 parts, 0.085 mole equivalents) was then added to the vessel as a 20 wt% solution in dry o-DCB. The reaction mixture was heated for 60 to 90 minutes with agitation under nitrogen. o-DCB was distilled from the vessel until the moisture in the o-DCB condensate was less than 30 ppm water. Heating the reaction mixture in the presence of the HEGC1 for 60 minutes resulted in the conversion of ClPA and monoamine (20a) to ClPAMI, and the conversion of amic-acids (see scheme 1) to ClPAMI. The amount of residual chloromonoamine (20a) was less than 0.1 mol%, and the amount of residual ClPA was less than 0.3 mol%, with respect to the total moles of amine groups charged to the reactor in the form of mPD. If the reaction was not dry, then dry distilled o-DCB was added to the vessel and removed by distillation until the reaction was judged dry. The dry CIPAMI/o-DCB on-stoichiometry slurry at 18 to 22 wt% solids was ready for polymerization.

### Polymerization and Quenching (General Procedure)

BPANa₂ slurry in o-DCB containing potassium phosphate (2.474 parts of BPANa₂ dry weight, 0.055 parts of potassium phosphate (K₃PO₄) (dry weight, 9.414 mole equivalents of BPANa₂, and 0.268 mole equivalents of potassium phosphate, all at about 21.40 wt% in o-DCB) was charged to the vessel containing the previously prepared CIPAMI/o-DCB slurry over a 45 minute period at 180°C, with agitation, under nitrogen. The molecular weight target for the reaction was 45,000 Daltons. The exothermic reaction ensued upon addition of the BPANa2 salt to the ClPAMI mixture. o-DCB was stripped from the reactor until the polymer wt% solids in the reactor was about 30 wt% solids.

After 2 hours from the completion of BPANa₂ salt addition, an aliquot sample was drawn for measuring the molecular weight (Mw) of the polymer. Similarly samples from the reactor were drawn after every hour and the Mw of the polymer was determined. This activity was carried out until the Mw of the polymer had leveled off (plateaued). The criterion used to identify when the polymer Mw was not significantly increasing was that three successive samples had a standard deviation of less than 500 Daltons. If the desired Mw was not achieved within 3000 Daltons, then a calculated amount of the BPANa₂/K₃PO₄ o-DCB slurry was charged to the reactor. After 1 hour from the completion of the salt correction, samples were drawn again and analyzed for Mw until a plateau of Mw was observed. If the desired Mw had not been achieved, a subsequent addition of a calculated amount of BPANa₂/K₃PO₄ o-DCB slurry was charged to the reactor. Once the desired Mw of the polymer was achieved, the polymer solution was cooled to about 165°C. At this stage dry o-DCB was added to dilute the polymer solution from about 30 wt% solids to about 20 wt% solids (actual % solids might vary from batch to batch). Phosphoric acid (85 wt% in water, 0.055 parts of H₃PO₄ dry weight, 0.58 mole equivalents) was then charged to the reactor to quench the reaction. The mixture was stirred for one hour at 150 to 165°C, under nitrogen. The mixture was then diluted with distilled dry o-DCB to provide a mixture that was about 10 wt% polymer containing sodium chloride and phosphates salts that had precipitated from the solution.

### Filtration

The polymer reaction mixture described above was pumped through a 13 to 20 micron sintered metal stainless steel filter at 100 to 165°C. The salt cake on the filter was rinsed with a clean dry o-DCB to provide a filtrate that was 6 to 9 wt% polymer solution in o-DCB that contained less than 10 ppm by weight of sodium, and the combined amount of hexaethylguanidinium salt and pentaethylguanidine present was in less than 4000 ppm. The filtrate was collected in a tank and cooled to 90 to 110°C.

### Polymer Purification

The o-DCB polymer solution was pumped to an appropriately sized tank and contacted with water in a continuous fashion, with mixing at 90 to 110°C, under pressure if necessary to prevent boiling of the two phase system. The pH of the water was controlled to 3 to 5 with the use of phosphoric acid. The volume ratio of polymer solution to water was 2 to 1. The time in the agitated vessel was 30 seconds to 15 minutes. The agitation speed was low enough such that the two phases did not emulsify, but the contact between the phases was adequate. The contents of the mixer were then conveyed to a liquid full decanter operated under pressure to prevent boiling of the liquids, where the polymer solution phase separated from aqueous phase. The aqueous phase contained extracted hexaethylguanidinium salt and pentaethylguanidine and dissolved o-DCB. The water phase was then conveyed to an activated carbon bed to remove the organic constituents before discharge to a wastewater treatment plant. The organic phase was conveyed to another mixer and contacted with pH 3 to 5 water in a volume ratio of 2 to 1 at 90 to 110°C under pressure, to remove any hexaethylguanidinium salt and pentaethylguanidine that was not removed in the first extraction. The time in the agitated vessel was 30 seconds to 15 minutes. The agitation speed was such that the two phases did not emulsify. The contents of the mixer were then conveyed to a liquid full decanter, where the polymer solution phase separated from aqueous phase. The water phase was then conveyed to an activated carbon bed to remove the organic constituents before discharging to a wastewater treatment plant. The organic phase was collected in a vessel under nitrogen. The sum of hexaethylguanidinium salt and pentaethylguanidine in the final polymer solution was less than 200 ppm.

### Polymer Isolation

The vessel that had collected the purified polymer solution was equipped with an agitator, an overheads line with condenser, a bottom nozzle, and line connected to a pump. The pump was connected to a heat exchanger. The discharge of the heat exchanger was connected to the top of the vessel. The heat exchanger was heated with hot oil. The material in the vessel was circulated through the pump and heat exchanger and back into the vessel, to bring the material in the vessel to reflux (180 to 190°C), under nitrogen and 0 to 1 psig (pounds per square inch gauge). o-DCB was removed overhead to provide a polymer solution that was 27 to 35 wt% solids in the vessel.

The 30 wt% concentrated polymer solution was conveyed in a continuous manner via a pump to a heat exchanger that heated the material to 280 to 290°C and then to the feed throat of a twin screw counter current extruder; there was a back pressure control valve between the extruder and the heat exchanger to prevent boiling inside of the heat exchanger. The extruder was equipped with a feed zone electrically heated to 300°C with an atmospheric vent, followed by a barrel zone heated to 350°C. This later zone was equipped with a vent operated at 80 mm pressure, followed by three vents operated at 30 mm pressure. Each vent was connected to a condenser to condense the o-DCB that was vaporized from feed zone and each of the barrel sections of the extruder. The condensed o-DCB was combined and collected in a vessel. The o-DCB was distilled before reuse. The extruder conveyed the molten polymer through a die head to produce strands of polymer, which were cooled in an external water bath and taken up in a chopper to produce polymer pellets. The pellets weighed between 5 and 50 mg. The pellets contained less than 500 ppm o-DCB. Mw, PDI, PDI***, Yellowness Index (YI) and R* were measured as described above. All samples were vacuum dried at 150°C for at least 4 hours before measurement.

Table 6 shows the analysis of polyetherimide pellets made with the use of SPP as the imidization catalyst (at .0038 parts, 0.024 mole equivalents).

**Table 6. Resin Properties Resulting From the Use of SPP in Imidization**

| C.Ex. No. | Imidization Catalyst | Mw | PDI | Mz/Mw | R* | YI^{a} |
|---|---|---|---|---|---|---|
| 30 | SPP | 51329 | 2.72 | 1.65 | 3.05 | 148 |
| 31 | SPP | 51144 | 2.73 | 1.65 | 3.02 | 140 |
| 32 | SPP | 49122 | 2.76 | 1.72 | 3.68 | 183 |
| 33 | SPP | 47352 | 2.64 | 1.65 | 3.07 | 162 |
| 34 | SPP | 50411 | 2.70 | 1.66 | 3.45 | 135 |
| 35 | SPP | 48971 | 2.72 | 1.71 | 3.40 | 162 |
| 36 | SPP | 47410 | 2.69 | 1.67 | 2.91 | 131 |
| 37 | SPP | 45868 | 2.65 | 1.66 | 2.97 | 121 |
| 38 | SPP | 44697 | 2.63 | 1.65 | 2.89 | 121 |
| 39 | SPP | 57075 | 2.96 | 1.77 | 3.75 | 134 |

| | | | | | | |
|---|---|---|---|---|---|---|
| Measured on 0.5 g of polyetherimide pellets dissolved in 10 mL of methylene chloride. | | | | | | |

Table 7 shows the analysis of polyetherimide pellets made with the use of HEGC1 as the imidization catalyst (0.022 parts, 0.085 mole equivalents).

**Table 7. Resin Properties Resulting From the Use of HEGCl in Imidization**

| Example No. | Imidization Catalyst | Mw | PDI | Mz/Mw | R* | YI^{a} |
|---|---|---|---|---|---|---|
| 40 | HEGCl | 46462 | 2.63 | 1.60 | 2.37 | 102 |
| 41 | HEGCl | 47543 | 2.66 | 1.61 | 2.37 | 104 |
| 42 | HEGCl | 46215 | 2.56 | 1.57 | 2.30 | 111 |
| 43 | HEGCl | 43417 | 2.54 | 1.57 | 2.25 | 110 |
| 44 | HEGCl | 43362 | 2.46 | 1.55 | 2.21 | 108 |
| 45 | HEGCl | 42347 | 2.50 | 1.57 | 2.35 | 102 |
| 46 | HEGCl | 45247 | 2.48 | 1.57 | 2.32 | 102 |

| | | | | | | |
|---|---|---|---|---|---|---|
| ^{a} Measured on 0.5 g of polyetherimide pellets dissolved in 10 mL of methylene chloride. | | | | | | |

As can be seen from Tables 6 and 7, the PDI* (Mz/Mw), R*, and YI were higher on average when SPP was used as the imidization catalyst compared to when HEGC1 was used as the imidization catalyst. The data presented in Tables 6 and 7 was collected from a production facility when each step of the process was working properly. Over a one month period of continuous production of polymer (at 2 batches of polymer per day), the average YI of the final polymer pellets was 130 when SPP was employed as the imidization catalyst. Over a 2 month period of continuous production of polymer (at 2 batches per day), the average YI of the final polymer pellets was 109 when HEGC1 was employed as the imidization catalyst.

HEGC1 is an effective imidization catalyst when employed at 0.2 to 1.0 mol% (with respect to the moles of mPD used) at the start of an imidization reaction, or when added to the ClPAMI just after it is brought on stoichiometry. Either mode of addition results in a ClPAMI with low residual ClPA and low residual chloromonoamine (20a). Moreover, minimization of the time that HEGC1 is heated with ClPAMI at 180°C results in a lower color polymer.

A higher PDI* (Mz/Mw) or a higher R* indicates a polymer that is more branched. A branched polymer behaves rheologically different than a less branched or unbranched polymer when molded. These differences can negatively affect molding cycle times. Branched polymers can become non-processable if they were to branch slightly more. For example, when a branched polymer is molded at a slightly higher than normal temperature, then more branching of the polymer can occur during molding, resulting in a cross-liked part with decreased tensile strength and impact resistance.

The Mw of the polymer in the feed to the extruder is typically less than the Mw of the extruded material. The molecular weight build across the extruder is problematic in that the desired Mw of polymer is not always obtained, nor is predictable. It has been found that the Mw build across the extruder is less when HEGC1 is used as the imidization catalyst added at the end of the ClPAMI reaction (1 mol%, with respect to the number of moles of mPD employed), as compared to when SPP is used as the imidization catalyst (0.25 mol% with respect to the number of moles of mPD employed). For example, polymer made on an industrial scale using SPP as the imidization catalyst, as described for comparative examples 30-39, gave an average of a Mw increase of 8195 Dalton (with a standard deviation of 2762 Daltons) upon extrusion. Polymer made on an industrial scale using HEGC1 as the imidization catalyst, as described for examples 40-46, gave an average of a Mw increase of 2784 Dalton (with a standard deviation of 1218 Daltons) upon extrusion. Thus the use of HEGC1 as the imidization catalyst affords polyetherimide polymer with a more predictable Mw, which eliminates the need to manage polyetherimide polymer produced with variable Mw.

### COMPARATIVE EXAMPLES 47-52 AND EXAMPLES 53-58

Polyetherimide pellets made using either SPP or HEGC1 as the imidization catalyst using the procedures of comparative examples 30-39 and examples 41-46, were aged at 134°C in the presence of water vapor in an autoclave over 6 days. Samples of the polymer were removed from the autoclave on day 2, 4, and 6. The samples were analyzed by GPC to determine the Mw of the polymer. The Mw decrease after 4 days was compared to the initial Mw, and the percent Mw retention was calculated.

The results are shown in Table 8.

**Table 8. Hydro-Stability Data of Polyetherimide Resins**

| Example | Synthetic Route to PEI^{a} | Mw (Time 0) | MW (Day 2) | Mw (Day 4) | Mw (Day 6) | Mw Retention (after 4 days) |
|---|---|---|---|---|---|---|
| C.Ex.47 | Dianhydride and Diamine | 44858 | 51538 | 43285 | 43215 | 96% |
| C.Ex.48 | Dianhydride and Diamine | 43852 | 44123 | 42396 | 41831 | 97% |
| C.Ex.49 | Dianhydride and Diamine | 52649 | 26350 | 49641 | 48253 | 94% |
| C.Ex.50 | Dianhydride and Diamine | 44063 | 43310 | 41941 | 40861 | 95% |
| C.Ex.51 | ClPAMI/SPP and BPANa2 | 47254 | 41400 | 38235 | 36355 | 81% |
| C.Ex.52 | ClPAMI/SPP and BPANa2 | 47136 | 44907 | 41880 | 40224 | 89% |
| 53 | ClPAMI/HEGCl and BPANa₂ | 41969 | 22357 | 38908 | 38382 | 93% |
| 54 | ClPAMI/HEGCl and BPANa₂ | 41732 | 39621 | 38528 | 37425 | 92% |
| 55 | ClPAMI/HEGCl and BPANa₂ | 41282 | 40467 | 39841 | 38438 | 97% |
| 56 | ClPAMI/HEGCl and BPANa₂ | 42741 | 40749 | 39350 | 38429 | 92% |
| 57 | ClPAMI/HEGCl and BPANa₂ | 43797 | 22405 | 39631 | 39414 | 90% |
| 58 | ClPAMI/HEGCl and BPANa₂ | 43757 | 42070 | 39773 | 38886 | 91% |

| | | | | | | |
|---|---|---|---|---|---|---|
| ^{a} Dianhydride and diamine = PEI formed by polycondensation of BPA dianhydride with meta-phenyldiamine (commercial process). ClPAMI/SPP and BPANa₂ = PEI made from ClPAMI (SPP as the imidization catalyst) and bisphenol A disodium salt. ClPAMI/HEGCl and BPANa₂ = PEI made from ClPAMI (HEGCl as the imidization catalyst) and bisphenol A disodium salt. | | | | | | |

It was found that polymer made from using HEGC1 as the imidization catalyst (Examples 53-58) aged better than polymer made from using SPP (Comparative Examples 51 and 52) as the imidization catalyst under the autoclave conditions. A commercial polyetherimide made from bisphenol A dianhydride and meta-phenylene diamine by the polycondensation method aged better than either of the polymers made from BPANa₂ and ClPAMI (Comparative Examples 47-50). The commercial samples were made from the condensation of BPA-DA and MPD using PA to adjust Mw. The reaction was conducted in ODCB at 25 to 60 % solids and 180 °C. After about 3 to 6 hours of reaction the polymerization solution was fed into a series of wiped film evaporators to finish the polymerization and remove all of the solvent. No catalyst was used.

### COMPARATIVE EXAMPLE 59 AND EXAMPLES 60-65

The following examples demonstrate the use of HEGC1 as an effective catalyst for the imidization between 3-chlorophthalic anhydride and diaminodiphenyl sulfone to produce DDS-ClPAMI in accordance with Equation 1. U.S. Patent No. 7,981,996 describes DDS-ClPAMI in detail.

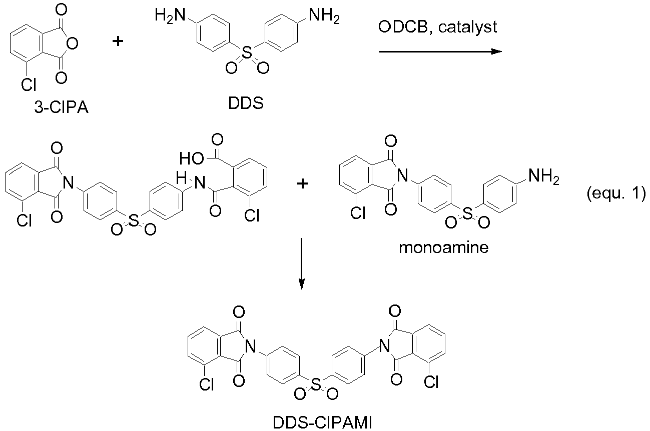

### Comparative Example 59

The following procedure is for the reaction of 3-ClPA with 4,4'-DDS with the use of SPP as the imidization catalyst. A 250 mL, 3-necked round-bottomed flask equipped with a mechanical stirrer, a nitrogen inlet and a Dean-Stark trap was charged with 4,4'-diaminodiphenylsulfone (DDS) (6.4 gr, 25.775 mmol), 3-chlorophthalic anhydride (3-ClPA) or a mixture of 3-ClPA with up to 5% of 4-ClPA (9.411 gr, 51.551 mmol), sodium phenylphosphinate (SPP), (21.1 mg, 0.128 mmol, 0.5 mol % relative to DDS) and 100 mL of o-DCB. The flask was immersed in an oil bath and heated to reflux with azeotropic removal of water. After 40 mL of o-DCB was distilled the reaction solution remained as a clear solution and after a while the mixture turned into thick white slurry. The reaction was allowed to proceed to a point where the intermediate monoamine-imide (MA, equation, monoamine) was at a relatively low level that it could be completely quantified by the HPLC method to give a correct bisimide analysis. A 3-ClPA or DDS correction was made to place the stoichiometry at 0.25-0.3 mol % excess ClPA based on ClPA charge. Internal reflux was maintained without additional removal of o-DCB until the reaction reached a specified residual monoamine level. As a representative example, the residual 3-ClPA and monoamine were 1.26 and 0.84 mol % respectively after 24 hours at reflux subsequent to final stoichiometry correction.

### Examples 60-65.

The following general procedure describes the use of HEGCl as an imidization catalyst to produce DDS-ClPAMI from 3-ClPA and DDS. As described above, 4,4'-DDS, 3-ClPA (or 3- and 4-ClPA mixture), 1 mol% of HEGCl (54.8 mg as a 17 wt% solution of HEGC1 in dry o-DCB), and the solvent were charged to the flask and heated to reflux. Approximately 40 mL of o-DCB was distilled from the flask to afford a clear solution. The reaction was kept at reflux for 1.5 hours. A sample was withdrawn for HPLC analysis and the appropriate correction was made (DDS or ClPA add) to place the stoichiometry at 0.25 to 0.3 mol% excess ClPA. The reaction mixture was allowed to proceed with internal reflux until the residual ClPA and monoamine (MA) levels were met (7-9 hours) (Example 60).

In Example 61, 0.2 moles (with respect to DDS) was added with the monomers, and the reaction was allowed to proceed as described above.

In Example 62, 0.2 moles of HEGC1 (with respect to DDS) was added after the reaction mixture began to reflux.

In Example 63, 0.2 moles of HEGC1 was added with the monomers, the reaction was brought to reflux, the stoichiometry adjusted, and then an additional 0.6 moles of HEGC1 was added to the reaction and the mixture was refluxed for 12 hours to provide a DDS-ClPAMI with low residual amounts of 3-ClPA (0.15 mol%), and the monoamine (0.02 mol%, equation 1).

Example 64 was run as described for Example 63, except that the reaction was run on a larger scale (42 grams of DDS) at higher wt% solids (18.5).

In Example 65, a 250 mL 3-necked round bottomed flask equipped with a mechanical stirrer, a Dean-Stark trap and a pressure equalizing addition funnel was charged under nitrogen with 4,4'-DDS (8.4 gr., 33.83 mmol) and o-DCB (85 mL). The flask and its contents were heated in an oil bath at 160°C at which temperature DDS was completely soluble. Molten 3-ClPA (12.352 gr., 67.66 mmol) was charged dropwise from the addition funnel during 15 minutes. All through the 3-ClPA addition, all contents in the flask remained in solution. The addition funnel was rinsed with 2 mL of o-DCB and the whole reaction mixture was heated to distill 19 mL of o-DCB leaving a reaction mixture at 18% solids. Another 19 mL of solvent were distilled off in 30 minutes and a solid product started to precipitate after an additional 60 minutes at reflux. At that point, HEGC1 (18 mg. as 17% solution in o-DCB) was added and the reaction was heated for an hour accompanied by distillation of 8 mL of solvent leaving a reaction mixture at 20% solids. HPLC analysis revealed the need for a 3-ClPA correction, which was made, and an additional amount of HEGC1 catalyst was added (54 mg as 17% o-DCB solution). The reaction was placed under internal reflux for 4 hours to complete the imidization, and HPLC analysis revealed 0.06 and 0.073 mol% residual 3-ClPA and monoamine (MA) respectively.

Reaction conditions and residual ClPA and MA amounts for Comparative Example 59 and Examples 60-65 are summarized in Table 9.

**Table 9 Use of SPP and HEGC1 as Imidization Catalyst for DDS and 3-ClPA**

| Ex. | DDS (g) | Cat. | Mole % Cat. / Addition Mode | Reaction Time** (hour) | (hour) after Cat Addn (time dose is in reaction) | % Solids | Residual ClPA (mol%) | Residual MA (mol%)*** | Comment |
|---|---|---|---|---|---|---|---|---|---|
| 59* | 6.4 | SPP | 0.5 / upfront | 24 | 24 | 16.5 | 1.26 | 0.84 | Monomers charged cold (room temperature). |
| 60 | 6.4 | HEGC1 | 1.0/ upfront | 4 | 4 | 18.5 | 0.41 | 0.17 | Monomers charged cold (room temperature) with catalyst. |
| 61 | 6.4 | HEGC1 | 0.2/ upfront | 18 | 18 | 16.5 | 0.24 | 0.03 | Monomers charged cold (room temperature) with catalyst. |
| 62 | 6.4 | HEGC1 | 0.2 / at 180°C | 7 | 5.5 | 16.5 | 0.26 | 0.078 | Monomers charged cold (room temperature), but catalyst charged hot. |
| 63 | 6.4 | HEGC1 | 0.2 upfront + 0.6 / at 180°C and after stoich. | 12 | (0.2 dose:12 0.8 dose: 10.5 hours: | 16.5 | 0.15 | 0.02 | Monomers and 0.2 % catalyst charged cold (room temperature), , then 0.6 % catalyst charged after on stoichiometry. |
| 64 | 42 | HEGC1 | 0.2 up front + 0.6 / at 180°C and after stoich. | 5 | (0.2)>5 0.8<5 | 18.5 | 0.1 | 0.019 | 1 mol% PA added (based on moles of ClPA employed) |
| 65 | 8.4 | HEGC1 | 0.2 upfront + 0.6 / at 180°C and after stoich. | 4 | (0.2):4 0.6<3 hours | 20 | 0.06 | 0.073 | Melt ClPA addition to DDS at 160°C with 0.2 % catalyst, then 0.6 % catalyst after on stoichiometry. |

| | | | | | | | | | |
|---|---|---|---|---|---|---|---|---|---|
| *Comparative **After Stoich. Correction ***Final Stoichiometry | | | | | | | | | |

It can be seen that the use of HEGCl as the imidization catalyst in examples 60-65 afforded a DDS-ClPAMI with significantly less residual ClPA and less residual monoamine than when SPP was used as the catalyst (Comparative Example 59). More particularly, in Comparative Example 59, the residual ClPA and residual monoamine amounts were 1.26 and 0.84, respectively, when 0.5 mol% of the SPP was added at the start of the reaction (at room temperature). In Example 60, the residual ClPA and residual monoamine amounts were 0.41 and 0.17, respectively, when 1 mol% of the HEGCL was added at the start of the reaction (at room temperature). In Example 61, the residual ClPA and residual monoamine amounts were 0.24 and 0.03, respectively, when 0.2 mol% of the HEGCL was added at the start of the reaction (at room temperature). In Example 62, the residual ClPA and residual monoamine amounts were 0.26 and 0.078, respectively, when 0.2 mol% of the HEGCL was added approximately 1.5 hours when the reaction mixture had reached a temperature of 180°C. In Example 63, the residual ClPA and residual monoamine amounts were 0.15 and 0.02, respectively, when 0.2 mol% of the HEGCL was added at the start of the reaction (at room temperature) and 0.6 mol% of HEGCl were added approximately 1.5 hours when the reaction mixture had reached a temperature of 180°C. In Example 64, the residual ClPA and residual monoamine amounts were 0.1 and 0.019, respectively, when 0.2 mol% of the HEGCL was added at the start of the reaction (at room temperature) and 0.6 mol% of HEGCl were added approximately 1.5 hours when the reaction mixture had reached a temperature of 180°C. In Example 65, the residual ClPA and residual monoamine amounts were 0.0.6 and 0.073, respectively, when 0.2 mol% of the HEGCL was added after 30 minutes of the reaction (at 180°C) and 0.6 mol% of HEGCl were added approximately 1.5 hours after the start of the reaction when the reaction mixture had reached a temperature of 180°C.

### EXAMPLE 67 AND COMPARATIVE EXAMPLE 68

These examples show that the use of HEGCl as a catalyst for the imidization reaction to form DDS-ClPAMI, and subsequent polymerization of the DDS-ClPAMI, affords a lower PDI* (Mz/Mw) than when SPP is used as the imidization catalyst. Lower PDI* is indicative of a polymer that is less branched, and less prone to shear thinning behavior when under a stress.

### Example 67

The purpose of this example was to make a polyetherimide by using the same HEGC1 during the imidization phase as well as the polymerization phase of the polyetherimide preparation process. A 1-liter 3-necked round-bottomed flask equipped with a mechanical stirrer, a nitrogen inlet and a Dean-Stark trap was charged at room temperature with 4,4'-diaminodiphenylsulfone (DDS) (42 gr, 169.15 mmol), 3-chlorophthalic anhydride (3-ClPA, 61.00 gr, 334.14 mmol), phthalic anhydride (PA, 616.2 mg, 4.163 mmol) and 500 mL of o-DCB. The flask was immersed in a hot oil bath and gradually heated to reflux with azeotropic removal of water. After the temperature reached 180°C, the reaction mixture was a clear solution and 80 mL of o-DCB have already distilled. About 90 mg HEGC1 (17% solution in o-DCB) was added and the reaction was allowed to proceed with additional distillation of o-DCB (35 mL) at which point the intermediate monoamine (MA) was at a relatively low level that it could be accurately quantified by the HPLC method to give a correct analysis. In this case a 3-ClPA correction was made to put the stoichiometry at 0.1 mol % excess ClPA based on ClPA charge. Additional HEGC1 catalyst (approx. 270 mg as a 17% o-DCB solution) was added and more o-DCB was distilled (55 mL) leaving the reaction at 18.5% solids (330 mL o-DCB in slurry). Internal reflux was maintained for 5 hours without additional removal of o-DCB. The residual monoamine (MA) and 3-ClPA were 0.019 and 0.102 mol% respectively.

To the bisimide slurry from the HEGC1 catalyzed imidization described above in 330 mL o-DCB was added additional o-DCB (100 mL). The mixture was heated to distill 150 mL of solvent and dry the slurry before adding BPA disodium salt to initiate the polymerization. In a separate oven dried 500 mL, round-bottomed flask equipped with an overhead mechanical stirrer were placed 180 gr of a dry BPA disodium salt slurry (at 24.3% solids in o-DCB) and then diluted with 120 mL of dry o-DCB. The slurry was gently stirred under nitrogen and heated to distill 35 mL of o-DCB leaving a dry salt slurry at 14.8% solids. The dry slurry was cooled to approximately 170°C and transferred under nitrogen pressure during 15 minutes through a high temperature Teflon tubing to the stirred bisimide slurry at 180°C. The BPA salt was rinsed with 20 mL of cold (room temperature) dry o-DCB and also transferred to the bisimide slurry. The combined bisimide-salt slurry was heated rapidly to start concentration and 267 mL of o-DCB were distilled off in 40 minutes to put the polymerization solution at about 30% solids based on the theoretical polymer weight. The polymerization was continued under total reflux and monitored by GPC for Mw measurement. The first plateau was achieved in 6 hours at a Mw of 36.95 KD after being at 30% solids. A salt slurry correction (4.0 gr., 24.3% solids) was made and Mw build plateaued again after 4 hours at 53.25 KD. Another salt correction with 0.6 gr of 24.3% slurry only increased Mw to 53.9 KD after 3 hours. The reaction was then quenched with 1.1 gr. of 85% aqueous phosphoric acid at 180 °C for 20 minutes. GPC on final quenched polymer showed an Mw = 51.977 KD, Mn = 22.597 KD, and Mz = 78.162 KD (PDI=2.3; PDI*=1.503).

### Comparative Example 68

A 250 mL, 3-necked round-bottomed flask equipped with a mechanical stirrer, a nitrogen inlet and a Dean-Stark trap was charged with 4,4'-diaminodiphenylsulfone (DDS) (6.4 gr, 25.775 mmol), 3-chlorophthalic anhydride (3-ClPA) or a mixture of 3-ClPA with up to 5% of 4-ClPA (9.411 gr, 51.551 mmol), sodium phenylphosphinate (SPP), (21.1 mg, 0.128 mmol, 0.5 mol % relative to DDS) and 100 mL of o-DCB. The flask was immersed in an oil bath and heated to reflux with azeotropic removal of water. After 40 mL of o-DCB was distilled the reaction solution remained as a clear solution and after a while the mixture turned into thick white slurry. The reaction was allowed to proceed to a point where the intermediate monoamine-imide (MA) was at a relatively low level that it could be completely quantified by the HPLC method to give a correct bisimide analysis. A 3-ClPA or DDS correction was made to put the stoichiometry at 0.25-0.3 mol % excess ClPA based on ClPA charge. At this point the contents of the flask were transferred to a Parr reactor, which was heated and maintained at 230°C and about 25 psi while stirring by using a partial condenser and a pressure relief valve. The reactor was depressurized and cooled after 12-15 hours. HPLC analysis indicated residual MA and ClPA to be in spec.

The polymerization of this SPP-based high temperature/pressure imidization followed a similar procedure as described in example 67, with one modification. After the imidization reactor was depressurized and cooled down, the required amount of HEGCl as a polymerization catalyst was added and the combined bisimide/HEGCl catalyst slurry was dried by distilling o-DCB to achieve 20 ppm water in the distillate. A BPA salt slurry was dried and added as in Example 67 followed by concentration and polymerization to build Mw. Salt corrections were made as needed to achieve the target Mw: Mw = 57.03 KD; Mn = 21.669 KD and Mz = 95.541 KD (PDI=2.68; PDI*=1.68).

The PDI* of the polyetherimide resulting from the use of HEGCl as the imidization catalyst was 1.503 (Example 67), while the PDI* of the polymer resulting from the use of SPP as the imidization catalyst was 1.68 (comparative Example 68). A higher PDI* (Mz/Mw) indicates a polymer that is more branched. A branched polymer behaves rheologically different than a less branched or unbranched polymer when molded. These differences can negatively affect molding cycle times. Branched polymers can become non-processable if they were to branch slightly more. For example, when a branched polymer is molded at a slightly higher than normal temperature, then more branching of the polymer can occur during molding, resulting in a cross-liked part with decreased tensile strength and impact resistance.

### EXAMPLES 69-80

These examples show that HEGC1 can be used as an imidization catalyst in the displacement process when added at the beginning of the imidization reaction or near the end of the reaction. ClPAMI was prepared as described for Examples 1-13, except that HEGC1 was added to the reaction vessel at the times indicated in Table 12. Table 12 shows the time at which the HEGC1 was added to the reaction mixture, the total reaction time for each reaction, the amount of time that the HEGC1 was in the reaction, the mol% of residual ClPA and the mol% of residual monoamine at the end of the reaction, and the overall stoichiometry of the ClPAMI at the end of reaction. A positive stoichiometry indicates excess ClPA present in the ClPAMI, and a negative stoichiometry indicates an excess of monoamine present in the ClPAMI.

**Table 12**

| Ex. No. | HEGC1 (mol%)* | Time HEGC1 added to the Reaction (hour) | Total Reaction Time (hour) | Time HEGC1 with ClPAMI (hour) | mol% Residual ClPA | mol% Residual Monoamine | ClPAMI Stoichiometry (mol%) |
|---|---|---|---|---|---|---|---|
| 69 | 0.05 | 3 | 15 | 12 | 0.27 | 0.05 | 0.22 |
| 70 | 0.1 | 3 | 10 | 7 | 0.17 | 0.04 | 0.13 |
| 71 | 0.1 | 2 | 6 | 4 | 0.24 | 0.03 | 0.21 |
| 72 | 0.1 | 2 | 10 | 8 | 0.17 | 0.01 | 0.16 |
| 73 | 0.2 | 5 | 8 | 3 | 0.08 | 0.15 | -0.07 |
| 74 | 0.2 | 8 | 11 | 3 | 0.20 | 0.02 | 0.18 |
| 75 | 0.2 | 1 | 4 | 3 | 0.23 | 0.02 | 0.21 |
| 76 | 1 | 1 | 3 | 2 | 0.02 | 0.02 | 0.00 |
| 77 | 1 | 0 | 5 | 5 | 0.00 | 0.04 | -0.04 |
| 78 | 1 | 0 | 6 | 6 | 0.03 | 0.02 | 0.01 |
| 79 | 1 | 8 | 10 | 2 | 0.19 | 0.02 | 0.17 |
| 80 | 1 | 8 | 10 | 2 | 0.20 | 0.04 | 0.17 |

| | | | | | | | |
|---|---|---|---|---|---|---|---|
| * Based on amount of m-PD | | | | | | | |

Example 69 shows that 0.05 mol% HEGCl, with respect to the amount of mPD employed in the reaction, when added 3 hours into the reaction, gave a ClPAMI with very low residual monoamine (0.05 mol%) after 15 hours of reaction time, with an acceptable final ClPAMI stoichiometry. This material is suitable for the displacement polymerization reaction with bisphenol A disodium salt.

Example 71 shows that 0.1 mol% HEGCl, with respect to the amount of mPD employed in the reaction, when added 2 hours into the reaction, gave a ClPAMI with very low residual monoamine (0.03 mol%) after 15 hours of reaction time, with an acceptable final ClPAMI stoichiometry. This material is suitable for the polymerization reaction with bisphenol A disodium salt.

Example 75 shows that 0.2 mol% HEGCl, with respect to the amount of mPD employed in the reaction, when added 1 hours into the reaction, gave a ClPAMI with very low residual monoamine (0.02 mol%) after 4 hours of reaction time, with an acceptable final ClPAMI stoichiometry. This material is suitable for the polymerization reaction with bisphenol A disodium salt.

Example 77 shows that 1 mol% HEGCl, with respect to the amount of mPD employed in the reaction, when added at the beginning of the reaction, gave a ClPAMI with very low residual monoamine (0.04 mol%) after 5 hours of reaction time, with an acceptable final ClPAMI stoichiometry. This material is suitable for the polymerization reaction with bisphenol A disodium salt.

Example 79 shows that 1 mol% HEGCl, with respect to the amount of mPD employed in the reaction, when added 8 hours into the reaction, gave a ClPAMI with very low residual monoamine (0.02 mol%) after 10 hours of reaction time, with an acceptable final ClPAMI stoichiometry. This material is suitable for the polymerization reaction with bisphenol A disodium salt.

All the examples shown in Table 12 demonstrate that HEGCl can be added at the beginning of the imidization reaction or near the end of the reaction, in an amount of 0.1 mol% HEGCl to 1.0 mol% HEGCl (with respect to the moles of mPD employed in the reaction), and afford a finished ClPAMI with low residual ClPA and monoamine, suitable for polymerization with bisphenol A disodium salt. Though not shown in Table 12, it was found that the more catalyst that was used, the faster the imidization reaction was completed. Completion of the imidization reaction is defined as affording a ClPAMI with residual monoamine of less than 0.10 mol% and the overall stoichiometry is less than 0.30 mol%.

### COMPARATIVE EXAMPLES 81 AND 82, AND EXAMPLE 83

### Comparative Example 81

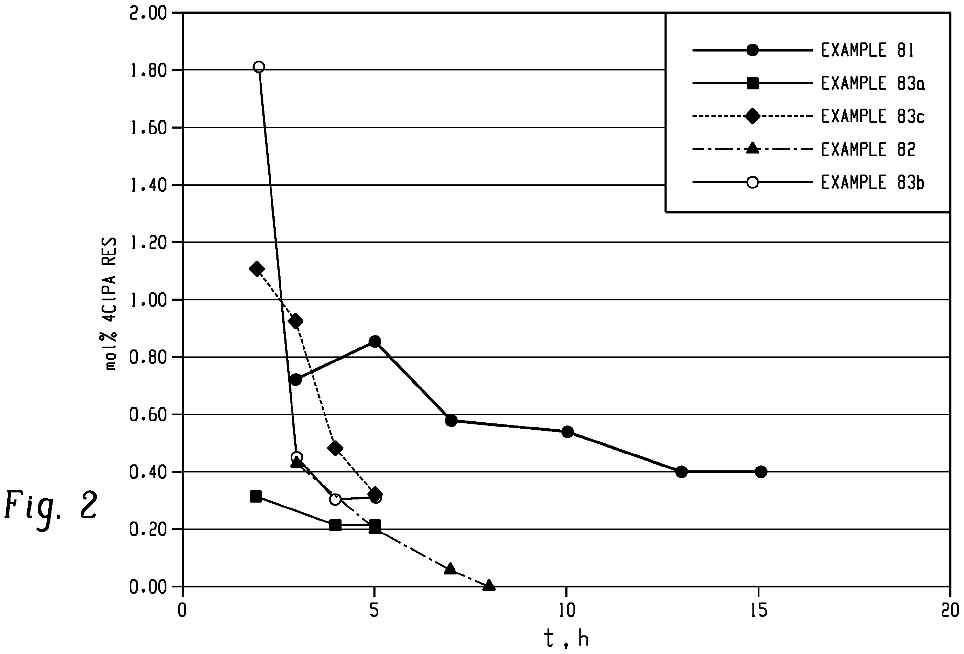

ClPAMI was prepared as slurry in o-DCB by condensing of 4-ClPA and m-phenylenediamine ("mPD") in o-DCB with azeotropic removal of water. A slight excess of 4-ClPA (about 0.2 mol%) was used. The reaction temperature was initially 100°C and ramped slowly to 150°C, 180°C, and then 200°C. The o-DCB was removed from the mixture until it reached 20 to 50 wt% solids. No imidization catalyst was present. After 14 to 15 hours, the amount of residual 4-ClPA was 0.4 mol%, and the amount of residual mPD was 0.11 mol%. The amount of 4-ClPA as a function of reaction time is provided in FIG. 2 ( curve Example 81). This curve represents a typical reaction profile when ClPAMI is made without an imidization catalyst. Due to second order kinetics of the reaction, at the low levels of unreacted ClPAMI and mPD present after 14-15 hours, the reaction substantially stops.

### Comparative Example 82

The same procedure as Comparative Example 81 was followed, except about 2 mol% excess mPD was used. The imidization was complete in about 8 hours, when all the 4-ClPA was consumed. The amount of 4-ClPA as a function of reaction time is provided in FIG. 2 (curve Example 82). Thus, use of excess mPD is an effective way to achieve a commercially viable reaction time. Since it has been observed that when mPD is used in excess, the use of the resulting imidized product results in crosslinked polymer (ordinarily an undesired results), the resulting amine end groups in the polyetherimide formed by polymerization of the 4-ClPAMI so made should be capped, which can be a disadvantage unless amine end groups are desired.

### Example 83a

The same procedure as Comparative Example 81 was followed, except that equimolar amounts of ClPAMI and mPD were used, and 0.18 mol% of ALIQUAT® HTA-1 was used as an imidization catalyst at the beginning of the reaction. ALIQUAT® HTA-1 is hexaethylguanidinium chloride ("HEGCl"). The imidization went to completion within 3 hours. The amount of 4-ClPA as a function of reaction time is provided in FIG. 2 as curve Example 83a.

### Example 83b

The same procedure as Comparative Example 81 was followed, except that equimolar amounts of ClPAMI and mPD were used, and 0.18 mol% of ALIQUAT® HTA-1 was used as an imidization catalyst and added 2 hours into the reaction. ALIQUAT® HTA-1 is hexaethylguanidinium chloride ("HEGCl"). The amount of 4-ClPA as a function of reaction time is provided in FIG. 2 as curve Example 83b.

### Example 83c

The same procedure as Comparative Example 81 was followed, except that equimolar amounts of ClPAMI and mPD were used, and 0.18 mol% of ALIQUAT® HTA-1 was used as an imidization catalyst was added 3 hours into the reaction. ALIQUAT® HTA-1 is hexaethylguanidinium chloride ("HEGCl). The amount of 4-ClPA as a function of reaction time is provided in FIG. 2 as curve Example 83c.

### EXAMPLES 84-86

Stoichiometric correction is a procedure in which the amounts of residual reactants during a reaction, and the stoichiometry of the reactants are adjusted by adding additional reactant, e.g., additional 4-ClPA or mPD, for the imidization reaction. The inventors hereof have found that the stoichiometric correction procedure can also be used when an imidization catalyst is present.

### Example 84

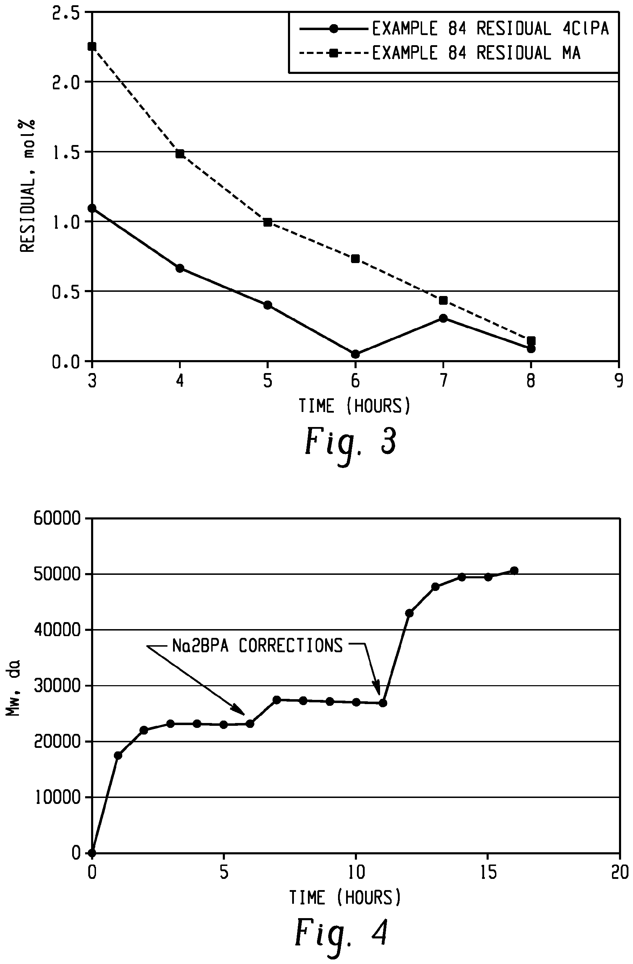

The same procedure as Comparative Example 81 was followed, except 0.20 mol% of HEGC1 was added 5 hours into the imidization reaction. The moisture content of the o-DCB distillate at this stage was less than 50 ppm. (However, the HEGC1 is also effective as a catalyst at higher moisture contents.) Determination of the molar ratio of residual mPD to 4-ClPA 6 hours into the reaction indicated a deficit of 4-ClPA. Fresh 4-ClPA was added. As indicated by FIG. 3, the imidization proceeded to very low levels of residual 4-ClPA after 8 hours from the start of the imidization and only 3 hours after addition of HEGCl. It is notable that stoichiometric correction was possible even in the presence of HEGCl. This is an important technical feature because this provides both a way to complete the imidization in a commercially viable time without using an excess of either reactant, while maintaining the ability to make adjustments during the imidization. Final residuals were: r4ClPA=0.08; rMA=0.14 at 99.71% conversion. Additional HEGC1 (0.8 mole%) was added to the ClPAMI, the reaction mixture was dried by azeotropic distillation, and a slurry of bisphenol A disodium salt ("BPANa₂") in o-DCB was added. A plot of Mw vs. polymerization time showed that the polymerization was complete, as defined by reaching a molecular weight of about 50,000 g/mole, after 16 hour (FIG. 4). This example shows that ClPAMI produced by the inventive process is suitable for the production of polyetherimide.

### Example 85

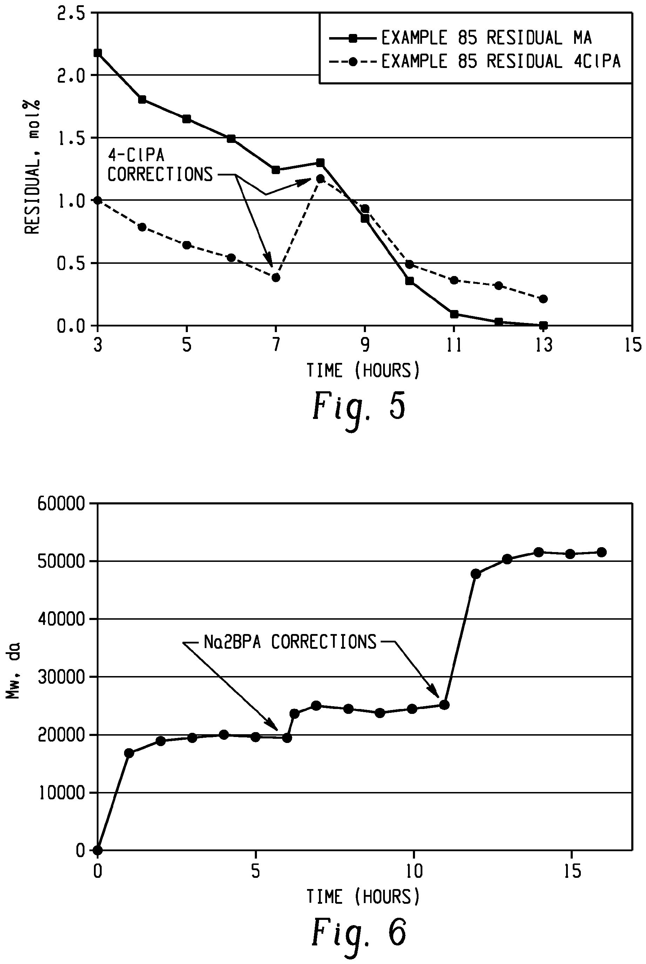

The procedure of Example 84 was followed for both ClPAMI and polymerization, except that the molar ratio of residual mPD to 4-ClPA was determined and 4ClPA made corrections at both 7^{th} and 8^{th} hour and 0.2 HEGCl was added at 9.5^{th} hour. Similar results were obtained. The imidization to produce ClPAMI was complete 13 hours from the start and 3.5 hours from the addition of HEGCl. Final residual of rClPA was 0.21 and the final residual of rMA was not determined. The reaction profiles of the imidization reaction and the polymerization reaction are provided in Figs. 5 and 6, respectively.

### Example 86

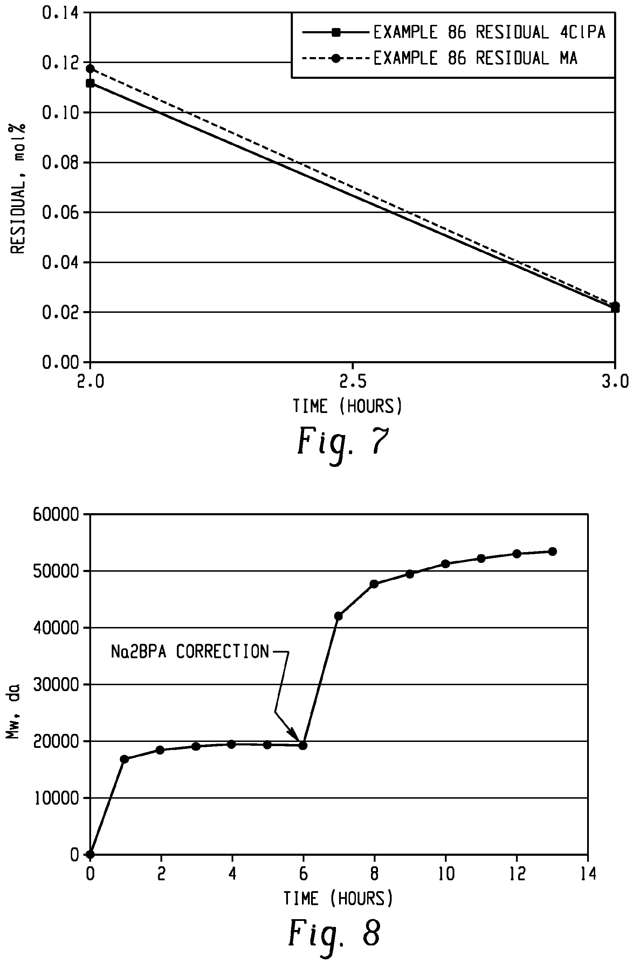

The same procedure as Example 84 was followed, except 1 mol% HEGC1 was used as the imidization catalyst instead of 0.18 mol%, and the HEGCl was added at the beginning of the imidization reaction, instead of 5 hours into the imidization reaction. The imidization reaction was substantially complete after only 3 hours and no stoichiometric adjustment was necessary (FIG. 7). This short imidization reaction time indicates the effectiveness of HEGCl as an imidization catalyst. After addition of BPA·Na₂ slurry in o-DCB, polymerization was complete in 13 hours. (FIG. 8) The Example demonstrates that when 1 mol% of HEGCl is added up front to catalyze the imidization, not only is the rate of ClPAMI formation accelerated, but polymerization takes place at an acceptable rate without additional polymerization catalyst.

### COMPARATIVE/ALTERNATIVE POLYCONDENSATION EXAMPLES 87-91

The purpose of Examples 87-89 was to observe whether the catalysts useful in our invention were also useful in polycondensation process for making polyetherimides. These examples demonstrate the use of HEGCl as an effective catalyst for the condensation polymerization of DDS and BPADA to form a polyetherimide as shown in Equation 2.

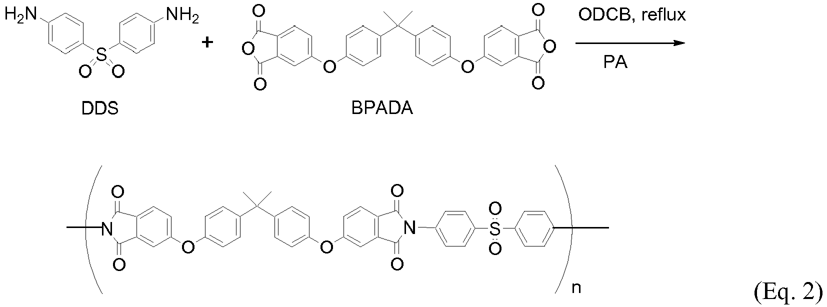

### Comparative Example 87

This examples shows polymerization according to Equation 2 in the absence of a catalyst. A 250 mL 3-necked round-bottomed flask equipped with a mechanical stirrer, a nitrogen inlet and a Dean-Stark trap was charged at room temperature with 4,4'-diaminodiphenylsulfone (DDS, 10.8 gr, 43.49 mmol), BPADA (22.512 g, 43.25 mmol) phthalic anhydride (PA, 250 mg, 1.69 mmol) and 100 mL of o-DCB. While stirring and keeping a positive nitrogen flow, the flask was then immersed in an oil bath and gradually heated to 190°C and then to 210°C during which 42 mL of o-DCB were distilled off within 3 hours. Heating was continued at reflux for 50 hours after which GPC measurement indicated a Mw of 39.7 KD based on PS standards calibration.

### Comparative Example 88

Following a similar procedure described in example 87, the reaction flask was charged at room temperature with the same amounts of 4,4'-DDS, 3-ClPA, PA and o-DCB. The flask was then immersed in an oil bath and gradually heated to 190°C and then to 210°C within 1.5 hours during which 42 mL of o-DCB were distilled off and GPC indicated an Mw of 4.83 KD. HEGC1 catalyst was added (about 58 mg as a 17% solution in o-DCB, 0.5 mol% based on DDS charge). Heating was continued at reflux for 10 hours. GPC measurement of samples drawn at 7 and 10 hours showed an Mw of 43.83 and 43.89 KD, respectively, indicating that the reaction was complete in 7 hours.

### Comparative Example 89.

Following a similar procedure as described in comparative example 87, the reaction flask was charged at room temperature with the same amounts of 4,4'-DDS, 3-ClPA, PA and o-DCB. The flask was then immersed in an oil bath and gradually heated to 190°C and then to 210°C within 1.5 hours during which 43 mL of o-DCB were distilled off. HEGC1 catalyst was added (about 23 mg as a 17% solution in o-DCB, 0.2 mol% based on DDS charge). Heating was continued at reflux for 10 hours. GPC measurement of samples drawn at 7, 8.5 and 10 hours showed Mw of 39.19, 40.79 and 40.04 KD respectively indicating that the reaction was complete in 8.5 hours.

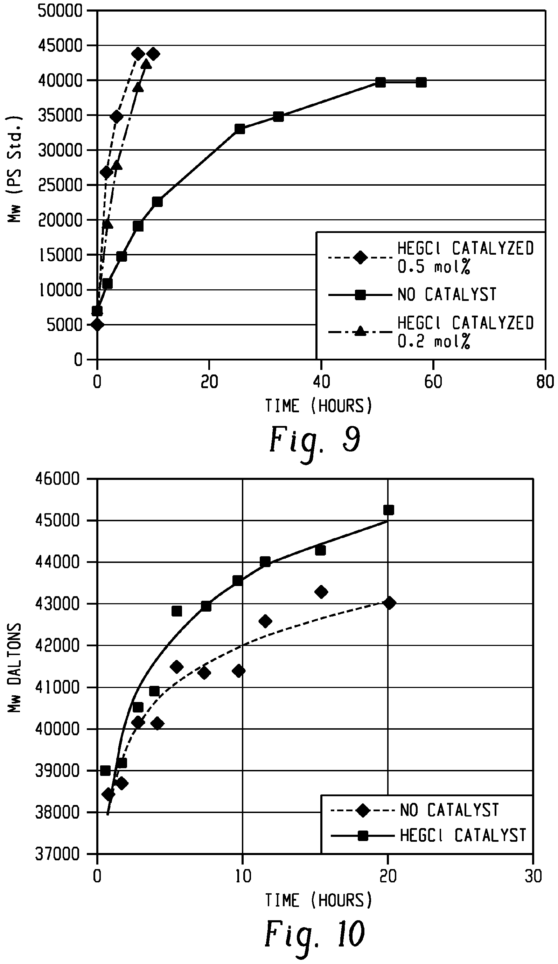

The Mw build over time for Comparative Examples 87 and Comparative Examples 88-89 is shown numerically in Table 13 and graphically in FIG. 9.

**Table 13**

| C.Ex. 87 (Uncatalyzed) | | C.Ex. 88 HEGC1 at 0.5 mol% | | C.Ex. 89 HEGCl at 0.2 mol% | |
|---|---|---|---|---|---|
| Time (hour) | Mw (g/mol) | Time (hour) | Mw (g/mol) | Time (hour) | Mw (g/mol) |
| 0 | 7013 | 0 | 4827 | 0.25 | 7525 |
| 2 | 10652 | 2 | 26785 | 2 | 19807 |
| 4.5 | 14722 | 3.5 | 34882 | 3.5 | 28022 |
| 7.5 | 19455 | 7 | 43826 | 7 | 39190 |
| 10.5 | 22699 | 10 | 43896 | 8.5 | 40789 |
| 25.5 | 32737 | | | 10 | 40044 |
| 32 | 34742 | | | | |
| 50.5 | 39764 | | | | |
| 58 | 39691 | | | | |

### Comparative Examples 90 and 91

The following examples demonstrate that HEGCl is an effective catalyst for the polycondensation of Bisphenol A dianhydride (BPADA) and meta-phenylene diamine, as shown in Equation 3.

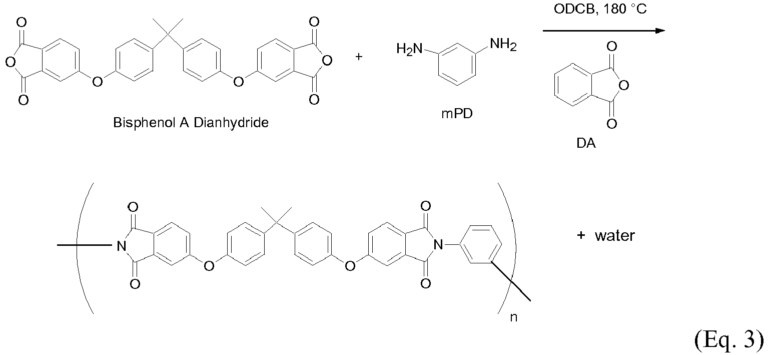

A 500-mL, 3-necked, round-bottomed flask, equipped with a mechanical stirrer, a Dean and Stark receiver, topped with a reflux condenser, and means to maintain an atmosphere of nitrogen was charged with 220 grams of ortho-dichlorobenzene, 80.00 grams of bisphenol A dianhydride (BPADA, 0.1537 moles), 17.197 grams of meta-phenylene diamine (mPD, 0.159 moles), and 1.283 grams of phthalic anhydride (PA, 0.011 moles). The number of moles of phthalic anhydride equaled 3.454 mole% of the total number of moles of anhydride moieties (two times the moles of bisphenol A dianhydride plus the moles of PA). This ratio of monomers and chain terminating agent provide a polyetherimide with a Mw of about 45000 Daltons, with a polydispersity near 2.1, with 0.01 mole% excess anhydride end groups. Phthalic anhydride is the chain-terminating compound.

The reaction mixture was mechanically stirred under an atmosphere of nitrogen and the flask was then heated with an external oil bath maintained at 150 °C. The monomers dissolved and began to polymerize. Water of imidization was collected in the Dean and Stark receiver as it evolved. The oil bath was maintained at 150 °C for 15 minutes and is then heated to 190°C (oil bath) for 3 hours. The temperature of the reaction mass was approximately 180°C. The reaction mixture was then equally divided into two 3-necked, 250-mL round-bottomed flasks, equipped with a mechanical stirrer, a Dean and Stark receiver, topped with a reflux condenser, and means to maintain an atmosphere of nitrogen. One of the flasks was charged with solid dry HEGC1 (0.21 gr, 0.8 mmoles, 0.5 mol% with respect to the moles of mPD employed, Comparative Example 91). The other flask was not charged with HEGC1 (Comparative Example 90). The reaction mixtures were heated with an oil bath set at 190°C and stirred; the time when the reactions achieved 190°C was set as time zero. Samples of each flask were analyzed by GPC to determine the molecular weight of polymer over time. The results are shown in Table 14 graphically in FIG. 10.

**Table 14**

| | C.Ex.90 (without HEGCl) | C.Ex. 91 (with HEGCl) |
|---|---|---|
| Time at 180°C (hour) | Mw (g/mole) | Mw (g/mole) |
| 0.75 | 38409 | 38942 |
| 1.75 | 38719 | 39164 |
| 2.83 | 40182 | 40521 |
| 3.92 | 40128 | 40934 |
| 5.42 | 41499 | 42795 |
| 7.42 | 41330 | 42906 |
| 9.59 | 41397 | 43548 |
| 11.6 | 42606 | 44000 |
| 15.35 | 43288 | 44243 |
| 20.1 | 43008 | 45251 |

It can be seen that the example with HEGC1 achieved the desired Mw within 20 hours of heating, while the reaction without HEGC1 did not achieve the desired Mw. Advantageously, the results show that the use of the catalysts useful for our invention in chloro-displacement processes can also be useful in polycondensation processes.

## Claims

1. A method for the manufacture of a bis(phthalimide) composition, the method comprising catalyzing imidization of a substituted phthalic anhydride and an organic diamine with a catalyst, in the presence of a solvent, the catalyst being selected from quaternary ammonium salts, quaternary phosphonium salts, guanidinium salts, pyridinium salts, imidazolium salts, and combinations thereof;
wherein
the substituted phthalic anhydride has a formula 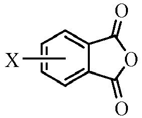 and
the organic diamine has a formula
H₂N-R-NH₂;
to provide the bis(phthalimide) composition comprising a residue of the catalyst, and a bis(phthalimide) of the formula 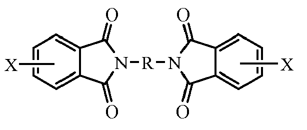 wherein, in the foregoing formulae,
X is selected from fluoro, chloro, bromo, iodo, nitro, and combinations thereof, and
R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 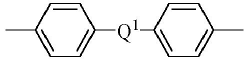 wherein Q¹ is a member selected from the group of a single bond, -O-, -S-, -C(O)-, - SO₂-, -SO-, -C_{y}H_{2y} wherein y is an integer from 1 to 5, and combinations thereof;
wherein conversion to the bis(phthalimide) is 99% complete, based on the moles of the substituted phthalic anhydride, in less than 6 hours, and
wherein the bis(phthalimide) comprises less than or equal to 0.5 mole percent residual substituted phthalic anhydride and less than or equal to 2 mole percent monoamine, based on the total moles of the substituted phthalic anhydride, the organic diamine, and a monofunctional reactant, if used.

2. The method of claim 1, wherein 0.1 to 1 mole % of the catalyst is present during the imidization reaction, based on the moles of the organic diamine.

3. The method of any one of Claims 1 to 2, wherein, if the catalyst reacts in the presence of the solvent at about 5 hours after the substituted phthalic anhydride and the diamine being reacting, the bis(phthalimide) composition comprises less than 0.15 mole percent of residual substituted phthalic anhydride and less than 1.0 mole percent monoamine of the formula 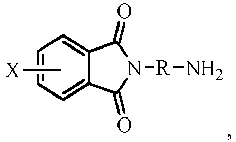 based on the total moles of the substituted phthalic anhydride, the organic diamine, and any monofunctional reactant and monofunctional phthalimide if present, wherein X and R are defined as in Claim 1.

4. A method for the manufacture of a polyetherimide composition, the method comprising
forming a bis(phthalimide) composition according to the method of claim 1, and
catalyzing polymerization of the bis(phthalimide) and an alkali metal salt of a dihydroxy aromatic compound of the formula
MO-Z-OM
in the presence of the catalyst that catalyzes imidization of the substituted phthalic anhydride and the diamine to form the polyetherimide composition comprising
a residue of the catalyst and
a polyetherimide of the formula 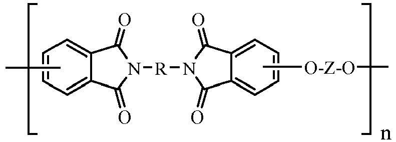 wherein in the foregoing formulae
X is selected from fluoro, chloro, bromo, iodo, nitro, and combinations thereof;
R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 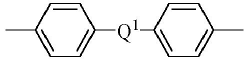 wherein Q¹ is selected from -O-, -S-, -C(O)-, -SO₂-, -SO-, -C_{y}H_{2y}- wherein y is an integer from 1 to 5, and combinations thereof;
M is an alkali metal;
Z is an aromatic C₆₋₂₄ monocyclic or polycyclic moiety optionally substituted with 1 to 6 C₁₋₈ alkyl groups, 1 to 8 halogen atoms, and combinations thereof; and
n is an integer greater than 1.

5. The method of Claim 4, further comprising adding a monofunctional reactant to a combination of the substituted phthalic anhydride and the organic diamine before adding the catalyst, or adding a monofunctional phthalimide to a combination of the substituted phthalic anhydride and the organic diamine before adding the catalyst.

6. The method of claim 4, wherein the catalyst is present in an amount ranging from 0.05 to 1 mole% based on the moles of the organic diamine during the imidization, and a second catalyst is added before the polymerization, wherein the second catalyst is selected from quaternary ammonium salts, quaternary phosphonium salts, guanidinium salts, pyridinium salts, imidazolium salts, and combinations thereof, and wherein the second catalyst is the same or different from the catalyst for the imidization.

7. The method of any one of Claims 4 to 6, wherein the bis(phthalimide) composition and the polyetherimide composition are manufactured in the same vessel.

8. The method of any one of Claims 4 to 7, wherein
X is chloro,
Z is 2,2-(4-phenylene)isopropylidene, and
R is m-phenylene, p-phenylene, diarylsulfone, or a group of the formula 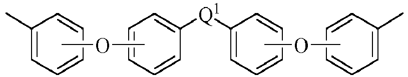 wherein Q¹ is -is a member selected from the group of a single bond, -O-, -S-, -C(O)-, -SO₂-, - SO-, -C_{y}H_{2y} wherein y is an integer from 1 to 5, and combinations thereof.

9. The method of any one of Claims 4 to 8, wherein the catalyst are a guanidinium salt, and the polyetherimide composition comprises less than 1000 ppm of the residue of the catalyst, based on the weight of the polyetherimide.

10. A polyetherimide composition comprising
(i) a polyetherimide of the formula 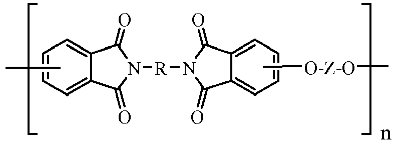 and
(ii) a residue of a catalyst selected from quaternary ammonium salts, quaternary phosphonium salts, guanidinium salts, pyridinium salts, imidazolium salts, and combinations thereof;
the polyetherimide being a catalyzed polymerization reaction product of (1) a bis(phthalimide) composition comprising a catalyzed imidization product of a substituted phthalic anhydride and an organic diamine and (2) an alkali metal salt of a dihydroxy aromatic compound, the imidization product being catalyzed by the catalyst, the bis(phthalimide) composition comprising less than or equal to 0.5 mole percent residual substituted phthalic anhydride and less than or equal to 2 mole percent monoamine, based on the total moles of the substituted phthalic anhydride, the organic diamine, and a monofunctional reactant, if used;
wherein
the substituted phthalic anhydride has a formula 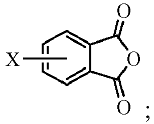 the organic diamine has a formula
H₂N-R-NH₂ ;
the bis(phthalimide) has a formula 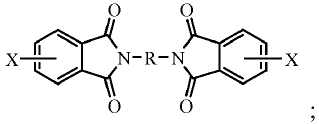 and
the alkali metal salt of the dihydroxy aromatic compound has a formula
MO-Z-OM;
wherein in the foregoing formulae,
X is selected from fluoro, chloro, bromo, iodo, nitro, and combinations thereof;
R is selected from an aromatic hydrocarbon group having 6 to 27 carbon atoms, a halogenated derivative thereof, a straight or branched chain alkylene group having 2 to 10 carbon atoms, a halogenated derivative thereof, a cycloalkylene group having 3 to 20 carbon atoms, a halogenated derivative thereof, -(C₆H₁₀)_{z}- wherein z is an integer from 1 to 4, an aromatic hydrocarbyl moiety having from 1 to 6 aromatic groups, and a divalent group of the formula 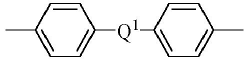 wherein Q¹ is selected from -O-, -S-, -C(O)-, -SO₂-, -SO-, -C_{y}H_{2y}- wherein y is an integer from 1 to 5, and combinations thereof;
M is an alkali metal;
Z is an aromatic C₆₋₂₄ monocyclic or polycyclic moiety optionally substituted with 1 to 6 C₁₋₈ alkyl groups, 1 to 8 halogen atoms, and combinations thereof; and
n is an integer greater than 1.

11. The polyetherimide composition of Claim 10, wherein the polymerization is catalyzed by the same catalyst that catalyzes the imidization.

12. The polyetherimide composition of any one of Claims 10 to 11, wherein the catalyst is a guanidinium salt, and a residue of the guanidinium salt is present in an amount less than 2000 ppm, preferably less than 1000 ppm, based on parts of the polyetherimide composition.

13. The polyetherimide composition of any one of Claims 10 to 12, further comprising an additive selected from catalysts, impact modifiers, fillers, reinforcing agents, antioxidants, thermal stabilizers, light stabilizers, ultraviolet light (UV) absorbers, quenchers, plasticizers, lubricants, mold release agents, antistatic agents, colorants, blowing agents, flame retardants, anti-drip agents, radiation stabilizers, and combinations thereof.

14. An article comprising the polyetherimide composition of any one of Claims 10 to 13.

15. A method of forming an article, comprising shaping, extruding, blow molding, or injection molding the polyetherimide composition of any one of Claims 10 to 13 to form the article.

## Patentansprüche

1. Verfahren zur Herstellung einer Bis(phthalimid)-Zusammensetzung, wobei das Verfahren das Katalysieren der Imidisierung eines substituierten Phthalsäureanhydrids und eines organischen Diamins mit einem Katalysator in Gegenwart eines Lösungsmittels umfasst, wobei der Katalysator aus quartären Ammoniumsalzen, quartären Phosphoniumsalzen, Guanidiniumsalzen, Pyridiniumsalzen, Imidazoliumsalzen und Kombinationen davon ausgewählt ist;
wobei
das substituierte Phthalsäureanhydrid eine Formel 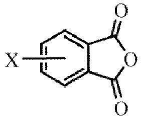 aufweist und
das organische Diamin eine Formel
H₂N-R-NH₂
aufweist;
um die Bis(phthalimid)-Zusammensetzung, die einen Rest des Katalysators umfasst, und ein Bis(phthalimid) der Formel 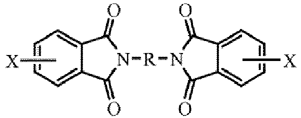 bereitzustellen,
wobei in den vorangehenden Formeln
X aus Fluor, Chlor, Brom, Iod, Nitro und Kombinationen davon ausgewählt ist und
R ausgewählt ist aus einer aromatischen Kohlenwasserstoffgruppe mit 6 bis 27 Kohlenstoffatomen, einem halogenierten Derivat davon, einer gerad- oder verzweigtkettigen Alkylengruppe mit 2 bis 10 Kohlenstoffatomen, einem halogenierten Derivat davon, einer Cycloalkylengruppe mit 3 bis 20 Kohlenstoffatomen, einem halogenierten Derivat davon, -(C₆H₁₀)_{z}-, wobei z eine ganze Zahl von 1 bis 4 ist, einem aromatischen Hydrocarbylrest mit 1 bis 6 aromatischen Gruppen und einer zweiwertigen Gruppe der Formel 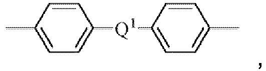 wobei Q¹ ein Bestandteil ist, der aus der Gruppe einer Einfachbindung, -O-, -S-, -C(O)-, -SO₂-, -SO-, -C_{y}H_{2y}-, wobei y eine ganze Zahl von 1 bis 5 ist, und Kombinationen davon ausgewählt ist;
wobei die Umsetzung zum Bis(phthalimid) bezogen auf die Mole des substituierten Phthalsäureanhydrids in weniger als 6 Stunden zu 99 % vollständig ist und
wobei das Bis(phthalimid) weniger als oder gleich 0,5 Molprozent von restlichem substituiertem Phthalsäureanhydrid und weniger als oder gleich 2 Molprozent Monoamin bezogen auf die Gesamtmole des substituierten Phthalsäureanhydrids, des organischen Diamins und eines monofunktionellen Reaktanten, sofern verwendet, umfasst.

2. Verfahren nach Anspruch 1, wobei 0,1 bis 1 Mol-% des Katalysators bezogen auf die Mole des organischen Diamins während der Imidisierungsreaktion vorhanden sind.

3. Verfahren nach irgendeinem der Ansprüche 1 bis 2, wobei, wenn der Katalysator in Gegenwart des Lösungsmittels nach ungefähr 5 Stunden Reagieren des substituierten Phthalsäureanhydrids und des Diamins reagiert, die Bis(phthalimid)-Zusammensetzung weniger als 0,15 Molprozent von restlichem substituiertem Phthalsäureanhydrid und weniger als 1,0 Molprozent eines Monoamins der Formel 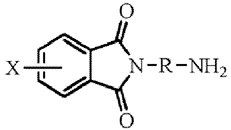 bezogen auf die Gesamtmole des substituierten Phthalsäureanhydrids, des organischen Diamins und irgendeines monofunktionellen Reaktanten und monofunktionellen Phthalimids, sofern vorhanden, umfasst, wobei X und R wie in Anspruch 1 definiert sind.

4. Verfahren zur Herstellung einer Polyetherimid-Zusammensetzung, wobei das Verfahren Folgendes umfasst:
Bilden einer Bis(phthalimid)-Zusammensetzung gemäß dem Verfahren nach Anspruch 1 und
Katalysieren der Polymerisation des Bis(phthalimids) und eines Alkalimetallsalzes einer aromatischen Dihydroxyverbindung der Formel
MO-Z-OM
in Gegenwart des Katalysators, der die Imidisierung des substituierten Phthalsäureanhydrids und des Diamins katalysiert, um die Polyetherimid-Zusammensetzung zu bilden, die Folgendes umfasst:
einen Rest des Katalysators und
ein Polyetherimid der Formel 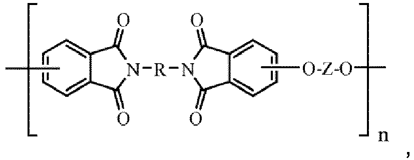 wobei in den vorangehenden Formeln
X aus Fluor, Chlor, Brom, Iod, Nitro und Kombinationen davon ausgewählt ist;
R ausgewählt ist aus einer aromatischen Kohlenwasserstoffgruppe mit 6 bis 27 Kohlenstoffatomen, einem halogenierten Derivat davon, einer gerad- oder verzweigtkettigen Alkylengruppe mit 2 bis 10 Kohlenstoffatomen, einem halogenierten Derivat davon, einer Cycloalkylengruppe mit 3 bis 20 Kohlenstoffatomen, einem halogenierten Derivat davon, -(C₆H₁₀)_{z}-, wobei z eine ganze Zahl von 1 bis 4 ist, einem aromatischen Hydrocarbylrest mit 1 bis 6 aromatischen Gruppen und einer zweiwertigen Gruppe der Formel 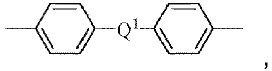 wobei Q¹ aus -O-, -S-, -C(O)-, -SO₂-, -SO-, -C_{y}H_{2y}-, wobei y eine ganze Zahl von 1 bis 5 ist, und Kombinationen davon ausgewählt ist;
M ein Alkalimetall ist;
Z ein aromatischer monocyclischer oder polycyclischer C₆₋₂₄-Rest ist, der gegebenenfalls mit 1 bis 6 C₁₋₈-Alkylgruppen, 1 bis 8 Halogenatomen und Kombinationen davon substituiert ist; und
n eine ganze Zahl größer als 1 ist.

5. Verfahren nach Anspruch 4, ferner umfassend das Zusetzen eines monofunktionellen Reaktanten zu einer Kombination des substituierten Phthalsäureanhydrids und des organischen Diamins vor dem Zusetzen des Katalysators oder das Zusetzen eines monofunktionellen Phthalimids zu einer Kombination des substituierten Phthalsäureanhydrids und des organischen Diamins vor dem Zusetzen des Katalysators.

6. Verfahren nach Anspruch 4, wobei der Katalysator während der Imidisierung in einer Menge vorhanden ist, die von 0,05 bis 1 Mol-% bezogen auf die Mole des organischen Diamins reicht, und ein zweiter Katalysator vor der Polymerisation zugesetzt wird, wobei der zweite Katalysator aus quartären Ammoniumsalzen, quartären Phosphoniumsalzen, Guanidiniumsalzen, Pyridiniumsalzen, Imidazoliumsalzen und Kombinationen davon ausgewählt ist und wobei der zweite Katalysator derselbe wie oder anders als der Katalysator für die Imidisierung ist.

7. Verfahren nach irgendeinem der Ansprüche 4 bis 6, wobei die Bis(phthalimid)-Zusammensetzung und die Polyetherimid-Zusammensetzung in demselben Behälter hergestellt werden.

8. Verfahren nach irgendeinem der Ansprüche 4 bis 7, wobei
X Chlor ist,
Z 2,2-(4-Phenylen)isopropyliden ist und
R m-Phenylen, p-Phenylen, Diarylsulfon oder eine Gruppe der Formel 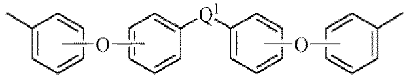 ist, wobei Q¹ ein Bestandteil ist, der aus der Gruppe einer Einfachbindung, -O-, -S-, -C(O)-, -SO₂-, -SO-, -C_{y}H_{2y}-, wobei y eine ganze Zahl von 1 bis 5 ist, und Kombinationen davon ausgewählt ist.

9. Verfahren nach irgendeinem der Ansprüche 4 bis 8, wobei der Katalysator ein Guanidiniumsalz ist und die Polyetherimid-Zusammensetzung weniger als 1000 ppm des Rests des Katalysators bezogen auf das Gewicht des Polyetherimids umfasst.

10. Polyetherimid-Zusammensetzung umfassend
(i) ein Polyetherimid der Formel 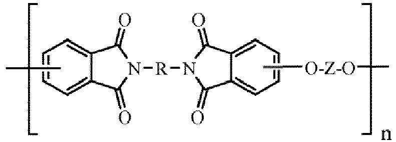 und
(ii) einen Rest eines Katalysators, der aus quartären Ammoniumsalzen, quartären Phosphoniumsalzen, Guanidiniumsalzen, Pyridiniumsalzen, Imidazoliumsalzen und Kombinationen davon ausgewählt ist;
wobei das Polyetherimid ein katalysiertes Polymerisationsreaktionsprodukt aus (1) einer Bis(phthalimid)-Zusammensetzung, die ein katalysiertes Imidisierungsprodukt aus einem substituieren Phthalsäureanhydrid und einem organischen Diamin umfasst, und (2) einem Alkalimetallsalz einer aromatischen Dihydroxyverbindung ist, wobei das Imidisierungsprodukt durch den Katalysator katalysiert wird, wobei die Bis(phthalimid)-Zusammensetzung weniger als oder gleich 0,5 Molprozent von restlichem substituiertem Phthalsäureanhydrid und weniger als oder gleich 2 Molprozent Monoamin bezogen auf die Gesamtmole des substituierten Phthalsäureanhydrids, des organischen Diamins und eines monofunktionellen Reaktanten, sofern verwendet, umfasst;
wobei
das substituierte Phthalsäureanhydrid eine Formel 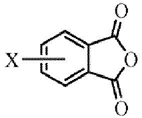 aufweist;
das organische Diamin eine Formel
H₂N-R-NH₂
aufweist;
das Bis(phthalimid) eine Formel 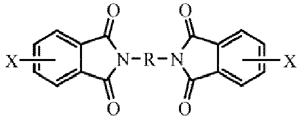 aufweist;
und
das Alkalimetallsalz der aromatischen Dihydroxyverbindung eine Formel
MO-Z-OM
aufweist;
wobei in den vorangehenden Formeln
X aus Fluor, Chlor, Brom, Iod, Nitro und Kombinationen davon ausgewählt ist;
R ausgewählt ist aus einer aromatischen Kohlenwasserstoffgruppe mit 6 bis 27 Kohlenstoffatomen, einem halogenierten Derivat davon, einer gerad- oder verzweigtkettigen Alkylengruppe mit 2 bis 10 Kohlenstoffatomen, einem halogenierten Derivat davon, einer Cycloalkylengruppe mit 3 bis 20 Kohlenstoffatomen, einem halogenierten Derivat davon, -(C₆H₁₀)_{z}-, wobei z eine ganze Zahl von 1 bis 4 ist, einem aromatischen Hydrocarbylrest mit 1 bis 6 aromatischen Gruppen und einer zweiwertigen Gruppe der Formel 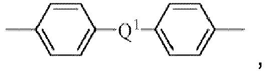 wobei Q¹ aus -O-, -S-, -C(O)-, -SO₂-, -SO-, -C_{y}H_{2y}-, wobei y eine ganze Zahl von 1 bis 5 ist, und Kombinationen davon ausgewählt ist;
M ein Alkalimetall ist;
Z ein aromatischer monocyclischer oder polycyclischer C₆₋₂₄-Rest ist, der gegebenenfalls mit 1 bis 6 C₁₋₈-Alkylgruppen, 1 bis 8 Halogenatomen und Kombinationen davon substituiert ist; und
n eine ganze Zahl größer als 1 ist.

11. Polyetherimid-Zusammensetzung nach Anspruch 10, wobei die Polymerisation durch denselben Katalysator katalysiert wird, der die Imidisierung katalysiert.

12. Polyetherimid-Zusammensetzung nach irgendeinem der Ansprüche 10 bis 11, wobei der Katalysator ein Guanidiniumsalz ist und ein Rest des Guanidiniumsalzes in einer Menge unter 2000 ppm, vorzugsweise unter 1000 ppm, bezogen auf Teile der Polyetherimid-Zusammensetzung vorhanden ist.

13. Polyetherimid-Zusammensetzung nach irgendeinem der Ansprüche 10 bis 12, ferner umfassend ein Additiv, das aus Katalysatoren, Schlagzähmodifikatoren, Füllstoffen, Verstärkungsmitteln, Antioxidantien, Wärmestabilisatoren, Lichtschutzmitteln, Ultraviolettlicht-Absorbern (UV-Absorbern), Löschern, Weichmachern, Schmiermitteln, Formentrennmitteln, Antistatika, Farbstoffen, Treibmitteln, Flammhemmern, Antitropfmitteln, Strahlungsstabilisatoren und Kombinationen davon ausgewählt ist.

14. Artikel umfassend die Polyetherimid-Zusammensetzung nach irgendeinem der Ansprüche 10 bis 13.

15. Verfahren zur Bildung eines Artikels, umfassend das Formen, Extrudieren, Blasformen oder Spritzgießen der Polyetherimid-Zusammensetzung nach irgendeinem der Ansprüche 10 bis 13 zur Bildung des Artikels.

## Revendications

1. Procédé pour la préparation d'une composition de bis(phtalimide), le procédé comprenant l'imidation par catalyse d'un anhydride phtalique substitué et d'une diamine organique avec un catalyseur, en présence d'un solvant, le catalyseur étant choisi parmi des sels d'ammonium quaternaires, des sels de phosphonium quaternaires, des sels de guanidium, des sels de pyridinium, des sels d'imidazolium, ainsi que leurs combinaisons ;
dans lequel
l'anhydride phtalique substitué répond à la formule : 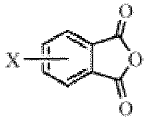 et
la diamine organique répond à la formule
H₂N-R-NH₂ ;
afin d'obtenir la composition de bis(phtalimide) comprenant un résidu du catalyseur, et un bis(phtalimide) répondant à la formule 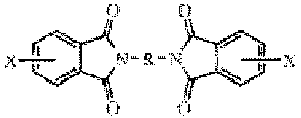 dans laquelle, dans les formules qui précèdent,
X est choisi parmi un groupe fluoro, un groupe chloro, un groupe bromo, un groupe iodo, un groupe nitro, ainsi que leurs combinaisons ;
R est choisi parmi un groupe d'hydrocarbure aromatique contenant de 6 à 27 atomes de carbone, un de ses dérivés halogénés, un groupe alkylène à chaîne droite ou à chaîne ramifiée contenant de 2 à 10 atomes de carbone, un de ses dérivés halogénés, un groupe cycloalkylène contenant de 3 à 20 atomes de carbone, un de ses dérivés halogénés, un groupe -(C₆H₁₀)_{z}- dans lequel z représente un entier de 1 à 4, une fraction hydrocarbyle aromatique contenant de 1 à 6 groupes aromatiques, et un groupe divalent répondant à la formule : 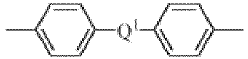 dans laquelle Q¹ représente un membre choisi parmi le groupe comprenant une liaison simple, un atome d'oxygène, un atome de soufre, un groupe -C(O)-, un groupe -SO₂-, un groupe -SO-, un groupe -C_{y}H_{2y}-, dans lequel y représente un entier de 1 à 5, ainsi que leurs combinaisons ;
dans lequel la conversion en bis(phtalimide) est complète à 99 % en se basant sur les moles de l'anhydride phtalique substitué, en moins de 6 heures ; et
dans lequel le bis(phtalimide) comprend une quantité inférieure ou égale à 0,5 mole % d'anhydride phtalique substitué résiduel et une quantité inférieure ou égale de 2 moles % de monoamine, en se basant sur le nombre total de moles de l'anhydride phtalique substitué, de la diamine organique et d'un réactif monofonctionnel, lorsqu'on en utilise.

2. Procédé selon la revendication 1, dans lequel une quantité de 0,1 à 1 mole % du catalyseur est présente au cours de la réaction d'imidation, en se basant sur le nombre de moles de la diamine organique.

3. Procédé selon l'une quelconque des revendications 1 à 2, dans lequel, lorsque le catalyseur réagit en présence du solvant approximativement 5 heures après la mise en réaction entre l'anhydride phtalique substitué et la diamine, la composition de bis(phtalimide) comprend une quantité inférieure à 0,15 mole % d'anhydride phtalique substitué résiduel et une quantité inférieure à 1,0 mole % de monoamine répondant à la formule 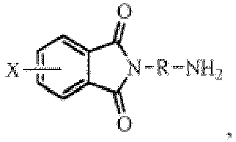 en se basant sur le nombre total de moles de l'anhydride phtalique substitué, de la diamine organique et de n'importe quel réactif monofonctionnel et de n'importe quel phtalimide monofonctionnel lorsqu'ils sont présents, X et R étant tels que définis à la revendication 1.

4. Procédé pour la préparation d'une composition de polyétherimide, le procédé comprenant le fait de :
former une composition de bis(phtalimide) selon le procédé de la revendication 1 ; et
polymériser par catalyse le bis(phtalimide) et un sel de métal alcalin d'un composé aromatique dihydroxylé répondant à la formule
MO-Z-OM
en présence du catalyseur qui catalyse l'imidation de l'anhydride phtalique substitué et de la diamine pour obtenir la composition de polyétherimide comprenant :
un résidu du catalyseur ; et
un polyétherimide répondant à la formule : 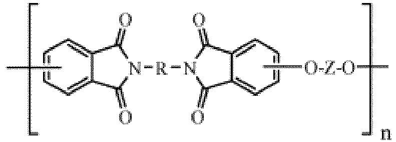 dans lequel, dans les formules qui précèdent :
X est choisi parmi un groupe fluoro, un groupe chloro, un groupe bromo, un groupe iodo, un groupe nitro, ainsi que leurs combinaisons ;
R est choisi parmi un groupe d'hydrocarbure aromatique contenant de 6 à 27 atomes de carbone, un de ses dérivés halogénés, un groupe alkylène à chaîne droite ou à chaîne ramifiée contenant de 2 à 10 atomes de carbone, un de ses dérivés halogénés, un groupe cycloalkylène contenant de 3 à 20 atomes de carbone, un de ses dérivés halogénés, un groupe -(C₆H₁₀)_{z}- dans lequel z représente un entier de 1 à 4, une fraction hydrocarbyle aromatique contenant de 1 à 6 groupes aromatiques, et un groupe divalent répondant à la formule : 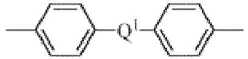 dans laquelle Q¹ est choisi parmi un atome d'oxygène, un atome de soufre, un groupe -C(O)-, un groupe -SO₂-, un groupe -SO-, un groupe -C_{y}H_{2y}-, dans lequel y représente un entier de 1 à 5, ainsi que leurs combinaisons ;
M représente un métal alcalin ;
Z représente une fraction aromatique monocyclique ou polycyclique en C₆-C₂₄, substituée de manière facultative avec de 1 à 6 groupes alkyle en C₁-C₈, de 1 à 8 atomes d'halogène, ainsi que leurs combinaisons ; et
n représente un entier supérieur à 1.

5. Procédé selon la revendication 4, comprenant en outre l'addition d'un réactif monofonctionnel à une combinaison de l'anhydride phtalique substitué et de la diamine organique avant d'ajouter le catalyseur ou l'addition d'un phtalimide monofonctionnel à une combinaison de l'anhydride phtalique substitué et de la diamine organique avant d'ajouter le catalyseur.

6. Procédé selon la revendication 4, dans lequel le catalyseur est présent en une quantité qui se situe dans la plage de 0,05 à 1 mole % en se basant sur le nombre de moles de la diamine organique au cours de l'imidation, et un second catalyseur est ajouté avant la polymérisation, le second catalyseur étant choisi parmi des sels d'ammonium quaternaires, des sels de phosphonium quaternaires, des sels de guanidium, des sels de pyridinium, des sels d'imidazolium, ainsi que leurs combinaisons, et dans lequel le second catalyseur est identique au catalyseur utilisé pour l'imidation ou est différent de celui-ci.

7. Procédé selon l'une quelconque des revendications 4 à 6, dans lequel la composition de bis(phtalimide) et la composition de polyétherimide sont préparées dans le même récipient.

8. Procédé selon l'une quelconque des revendications 4 à 7, dans lequel :
X représente un groupe chloro ;
Z représente un groupe 2,2-(4-phénylène)isopropylidène ; et
R représente un groupe m-phénylène, un groupe p-phénylène, une diarylsulfone ou un groupe répondant à la formule : 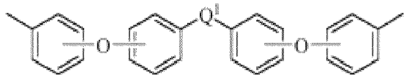 dans laquelle Q¹ représente un membre choisi parmi le groupe comprenant une liaison simple, un atome d'oxygène, un atome de soufre, un groupe -C(O)-, un groupe -SO₂-, un groupe -SO-, un groupe -C_{y}H_{2y}-, dans lequel y représente un entier de 1 à 5, ainsi que leurs combinaisons.

9. Procédé selon l'une quelconque des revendications 4 à 8, dans lequel le catalyseur représente un sel de guanidinium et la composition de polyétherimide comprend une quantité inférieure à 1000 ppm du résidu de catalyseur, basée sur le poids du polyétherimide.

10. Composition de polyétherimide comprenant :
(i) un polyétherimide répondant à la formule : 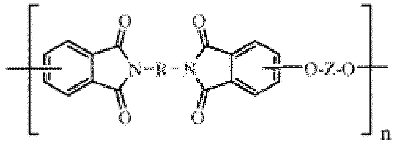 et
(ii) un résidu d'un catalyseur choisi parmi des sels d'ammonium quaternaires, des sels de phosphonium quaternaires, des sels de guanidium, des sels de pyridinium, des sels d'imidazolium, ainsi que leurs combinaisons ;
le polyétherimide représentant un produit d'une réaction de polymérisation par catalyse de (1) une composition de bis(phtalimide) comprenant un produit d'imidation par catalyse d'un anhydride phtalique substitué et d'une diamine organique, et de (2) un sel de métal alcalin d'un composé aromatique dihydroxylé, le produit d'imidation étant catalysé par le catalyseur, la composition de bis(phtalimide) comprenant une quantité inférieure ou égale à 0,5 mole % d'anhydride phtalique substitué résiduel et une quantité inférieure ou égale de 2 moles % de monoamine, en se basant sur le nombre total de moles de l'anhydride phtalique substitué, de la diamine organique et d'un réactif monofonctionnel, lorsqu'on en utilise ;
dans lequel
l'anhydride phtalique substitué répond à la formule : 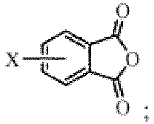 la diamine organique répond à la formule :
H₂N-R-NH₂ ;
le bis(phtalimide) répond à la formule : 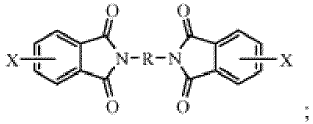 et
le sel de métal alcalin du composé aromatique dihydroxylé répond à la formule :
MO-Z-OM ;
dans lequel, dans les formules qui précèdent :
X est choisi parmi un groupe fluoro, un groupe chloro, un groupe bromo, un groupe iodo, un groupe nitro, ainsi que leurs combinaisons ;
R est choisi parmi un groupe d'hydrocarbure aromatique contenant de 6 à 27 atomes de carbone, un de ses dérivés halogénés, un groupe alkylène à chaîne droite ou à chaîne ramifiée contenant de 2 à 10 atomes de carbone, un de ses dérivés halogénés, un groupe cycloalkylène contenant de 3 à 20 atomes de carbone, un de ses dérivés halogénés, un groupe -(C₆H₁₀)_{z}- dans lequel z représente un entier de 1 à 4, une fraction hydrocarbyle aromatique contenant de 1 à 6 groupes aromatiques, et un groupe divalent répondant à la formule : 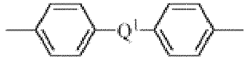 dans laquelle Q¹ est choisi parmi un atome d'oxygène, un atome de soufre, un groupe -C(O)-, un groupe -SO₂-, un groupe -SO-, un groupe -C_{y}H_{2y}-, dans lequel y représente un entier de 1 à 5, ainsi que leurs combinaisons ;
M représente un métal alcalin ;
Z représente une fraction aromatique monocyclique ou polycyclique en C₆-C₂₄, substituée de manière facultative avec de 1 à 6 groupes alkyle en C₁-C₈, de 1 à 8 atomes d'halogène, ainsi que leurs combinaisons ; et
n représente un entier supérieur à 1.

11. Composition de polyétherimide selon la revendication 10, dans laquelle la polymérisation est catalysée par le même catalyseur que celui qui catalyse l'imidation.

12. Composition de polyétherimide selon l'une quelconque des revendications 10 à 11, dans laquelle le catalyseur est un sel de guanidium et un résidu du sel de guanidium est présent en une quantité inférieure à 2.000 ppm, de préférence inférieure à 1.000 ppm, en se basant sur des parties de la composition de polyétherimide.

13. Composition de polyétherimide selon l'une quelconque des revendications 10 à 12, comprenant en outre un additif choisi parmi des catalyseurs, des modificateurs de la résistance aux chocs, des matières de charge, des agents de renforcement, des antioxydants, des stabilisateurs thermiques, des stabilisateurs contre l'effet de la lumière, des agents absorbant la lumière ultraviolette (UV), des désactivateurs, des plastifiants, des lubrifiants, des agents de démoulage, des agents antistatiques, des colorants, des agents de transformation en mousse, des retardateurs des flammes, des agents antigoutte, des agents procurant une stabilité aux rayonnements, ainsi que leurs combinaisons.

14. Article comprenant la composition de polyétherimide selon l'une quelconque des revendications 10 à 13.

15. Procédé de formation d'un article, comprenant le façonnement, l'extrusion, le moulage par soufflage ou bien le moulage par injection de la composition de polyétherimide selon l'une quelconque des revendications 10 à 13 pour former l'article.
